# EUROPEAN PATENT APPLICATION

(11) **EP 4 692 104 A1**
(43) Date of publication of application: **11.02.2026**
(21) Application number: 24784960.7
(22) Date of filing: 04.04.2024
(51) Int. Cl.: C07K 1/13, C07K 16/00

(54) **CONJUGATE OF ANTIBODY AND FUNCTIONAL SUBSTANCE OR SALT OF SAID CONJUGATE, AND ANTIBODY INTERMEDIATE HAVING THIOL GROUP OR SALT OF SAID ANTIBODY INTERMEDIATE**

(30) Priority: 05.04.2023 JP 2023061596
(71) Applicant: AJINOMOTO CO., INC., Chuo-ku Tokyo 104-8315 (JP)
(72) Inventor: MATSUDA, Yutaka, Kawasaki-shi, Kanagawa 210-8681 (JP); FUJII, Tomohiro, Kawasaki-shi, Kanagawa 210-8681 (JP); WATANABE, Tomohiro, Kawasaki-shi, Kanagawa 210-8681 (JP)
(74) Representative: Grünecker Patent- und Rechtsanwälte PartG mbB
(86) International application number: PCT/JP2024/013958
(87) International publication number: WO 2024/210178

(57) **Abstract**

A conjugate of an antibody and a functional substance, or a salt thereof, that has excellent properties is provided. More specifically, a conjugate of an antibody and a functional substance, or a salt thereof, in which
(1) the conjugate includes an immunoglobulin unit including two heavy chains and two light chains,
(2) the immunoglobulin unit includes:
(a) two or more cysteine residue-modifying moieties connected to the immunoglobulin unit by bonding to the sulfur atoms in the side chains of two or more cysteine residues in the two heavy chains and two light chains; and
(ii) two or more lysine residue-modifying moieties regioselectively connected to the immunoglobulin unit by bonding to the nitrogen atoms in the side chains of two or more lysine residues in said two heavy chains, and

(3) the cysteine residue-modifying moiety is represented by a specific formula, and related substances thereof, are provided.

## Description

### TECHNICAL FIELD

The present invention relates to conjugates of antibodies and functional substances, or salts thereof, and antibody intermediates having a thiol group, or salts thereof.

### BACKGROUND ART

Recently, research and development of antibody-drug conjugates (ADCs) have been active. ADCs, as implied by the name, are medicines in which a drug (e.g., an anti-cancer agent) is conjugated with an antibody and has direct cytotoxic activities on cancer cells or the like. A typical ADC is T-DM1 (product name: Kadcyla^{®}), which was jointly developed by Immunogene, Inc. and F. Hoffmann-La Roche Ltd.

ADCs are produced by binding a functional group in the side chain of a specific amino acid residue present in an antibody to a drug. An example of such a functional group utilized in the production of ADCs is the amino group in the side chain of a lysine residue present in the antibody. Several technologies have been reported for modifying lysine groups in antibodies (e.g., lysine residues at positions 246/248, 288/290, or 317) (e.g., Patent documents 1 to 4).

Another example of such a functional group utilized in the production of ADCs is a thiol group. Antibodies have disulfide groups because the heavy-heavy chains and the heavy-light chains are linked by disulfide bonds. For example, an IgG antibody composed of two heavy chains and two light chains has four disulfide groups because the heavy-heavy chains and the heavy-light chains are linked by four disulfide bonds. Such disulfide bonds can be cleaved by a reducing agent. For example, if all four disulfide bonds in an IgG are cleaved, an IgG antibody with eight thiol groups is generated. Even if all four interchain disulfide bonds are cleaved, the heavy and light chains of the antibody do not dissociate. This is because non-covalent bonds between the chains can maintain the higher-order structure of the antibody. This non-covalent bonding also maintains the antibody properties (binding ability to the target). For example, trastuzumab deruxtecan (Patent document 5), known as Enhertu^{®}, is an ADC with a drug-to-antibody ratio (DAR) of 8, which is produced by cleaving (reducing) all four interchain disulfide bonds and then conjugating the drug. Such ADCs are known to act as antigen-specific agents by maintaining their antibody properties.

In an ADC, an antibody and a drug are linked via a linker. Various linkers exist for ADCs. For example, ADCs used as anticancer agents contain a linker comprising a dipeptide consisting of valine-citrulline (Val-Cit: VC structure), which is stable in human plasma and has a structure that can be cleaved by a specific enzyme to release the drug within cancer cells. As shown in (A) below, a linker containing such a dipeptide is stable in human plasma. As shown in (B) below, however, the VC structure is recognized by cathepsin B in the lysosomes of human cancer cells, and the amide bond on the carboxy-terminal side of citrulline is cleaved. Therefore, an ADC having a linker containing such a dipeptide can release the drug within human cancer cells to exhibit its drug efficacies.
(A) **Stable in human plasma** 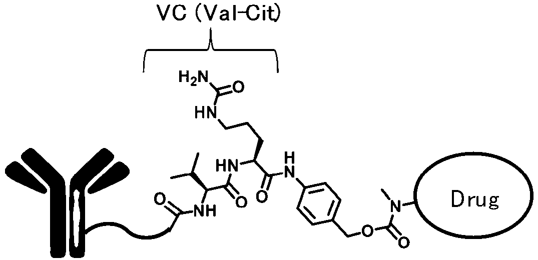
(B) Cleaved in lysosomes in human cancer cells 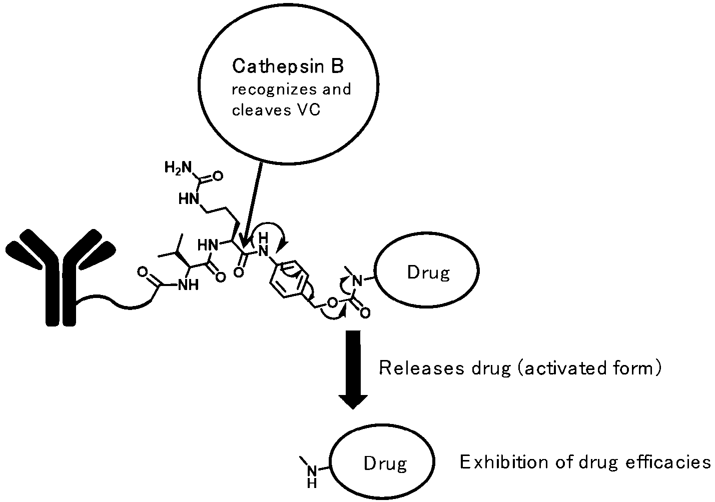 However, ADCs with linkers containing the dipeptides as described above are unstable in mouse plasma (Non-Patent documents 1 and 2). This is because mouse plasma contains Ces1c, a carboxylase that recognizes the VC structure and cleaves the amide bond on the carboxy-terminal side of citrulline, causing the linker containing the dipeptide as described above to be cleaved by Ces1c in the plasma. Therefore, ADCs with linkers containing the dipeptides as described above exhibit significantly different pharmacokinetics between mice and humans. For this reason, therefore, mice disadvantageously make it difficult to evaluate the efficacy in humans.
(C) Undesired reaction in mouse plasma

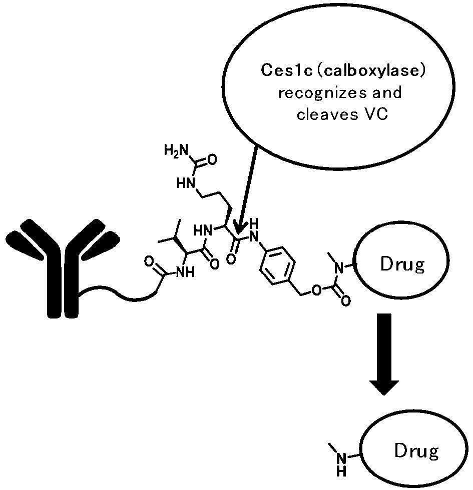

To improve the instability in mouse plasma of ADCs having the structure of "antibody-spacer-VC structure-spacer-drug" as described above, stabilization of ADCs by modifying the linker (i.e., spacer-VC structure-spacer) has been attempted. From this viewpoint, ADCs in which an antibody and a drug or a mimic thereof are linked via the aforementioned linker have been reported. For example, the following ADC has been reported as an ADC containing the linker not in the main chain connecting the antibody and the drug or its mimic, but in the side chain of that main chain (Patent document 6). 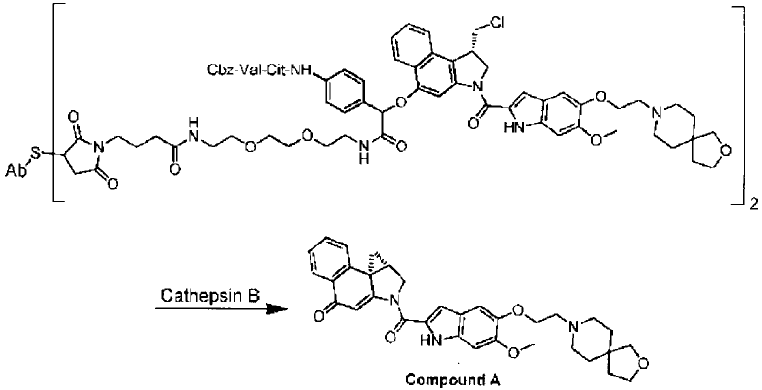
Ab: Antibody
Cbz: Benzyloxycarbonyl
Val: Valine residue
Cit: Citrulline residue

Incidentally, Non-patent document 3 discloses that higher degrees of hydrophobicity of ADCs result in faster plasma clearance, and that the degrees of hydrophobicity of ADCs can be evaluated by Hydrophobic Interaction Chromatography (HIC)-HPLC.

### PRIOR ART DOCUMENTS

### Patent documents

Patent document 1: WO2018/199337
Patent document 2: WO2019/240288
Patent document 3: WO2019/240287
Patent document 4: WO2020/090979
Patent document 5: WO2014/057687
Patent document 6: WO2015/038426

### Non-patent documents

Non-patent document 1: Dorywalska et al., Bioconjugate Chem., 2015, 26 (4), 650-659.
Non-patent document 2: Dorywalska et al., Mol Cancer Ther., 2016, 15 (5), 958-70.
Non-patent document 3: Lyon et al., Nat Biotechnol., 2015, 33 (7), 733-5.

### SUMMARY OF THE INVENTION

### PROBLEMS TO BE ACHIEVED BY THE INVENTION

An object of the present invention is to provide conjugates of antibodies and functional substances, or salts thereof, that have excellent desired properties.

The present inventors have intensively studied to find that a conjugate comprising a linker having a specific structure that contains both a hydrophilic group and a cleavable site, in the side chain of the main chain connecting an antibody and a drug (or drug mimic), and in which the linker is connected to the antibody (immunoglobulin unit), has excellent properties. For example, such a conjugate or a salt thereof can have excellent clearance (long in-vivo residence time) and a low aggregation rate (high monomer ratio). In addition, by using a preferred example of the linker with the specific structure as described above, it is possible to retain the hydrophilic group on the antibody after cleavage. That is, before cleavage, the drug is linked to a hydrophilic group in the form of a conjugate with an antibody, and thus the hydrophobicity of the drug can be attenuated (masking by the hydrophilic group), while after cleavage, the hydrophilic group dissociates from the drug, allowing the drug to exert its original hydrophobicity. Therefore, for example, when the drug contained in the conjugate is a hydrophobic compound from which cell permeability (so-called bystander effect) can be expected, the present invention using the linker as described above has the advantage of making it easy for the drug to exert its cell permeability. In this specification, ADCs having such a linker may be referred to as exo-type ADCs.

The present inventors have also succeeded in developing antibody derivatives and compounds useful for the preparation of such conjugates. The conjugates, antibody intermediates, and compounds of the present invention represented by structures such as formulas (I-1) to (VIII) have the technical feature of sharing the partial structural unit of the structural unit represented by formula (VI), excluding X and Y. The present inventors have succeeded in developing a series of inventions having such technical features, and have completed the present invention. The prior art neither describes nor suggests the chemical structure of the conjugate of the present invention, nor the relationship between such a chemical structure and the excellent properties as described above. The prior art also neither describes nor suggests the antibody derivatives and compounds of the present invention that can be utilized for the preparation of such conjugates.

That is, the present invention is as follows.

In the first embodiment, the present invention provides a conjugate of an antibody and a functional substance, or a salt thereof, wherein
(1) the conjugate comprises an immunoglobulin unit comprising two heavy chains and two light chains, and
(2) the immunoglobulin unit comprises
   (a) two or more cysteine residue-modifying moieties connected to the immunoglobulin unit by bonding to the sulfur atoms in the side chains of two or more cysteine residues in said two heavy chains and two light chains, and
   (b) two or more lysine residue-modifying moieties regioselectively connected to the immunoglobulin unit by bonding to the nitrogen atoms in the side chains of two or more lysine residues in said two heavy chains, and
(3) said cysteine residue-modifying moieties are represented by the following formula (I-1): 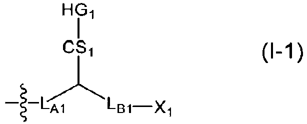 wherein
   the hyphen (-) with a wavy line represents a bond to said sulfur atom,
   HG₁ represents a hydrophilic group or a monovalent group containing a hydrophilic group,
   CS₁ represents a divalent group containing a cleavable site,
   L_{A1} and L_{B1} each independently represent a divalent group, and
   X₁ represents a functional substance.

In a preferred embodiment, the immunoglobulin unit may be a human immunoglobulin unit.

In a preferred embodiment, the human immunoglobulin unit may be a human IgG antibody.

In a preferred embodiment, said two or more cysteine residues may be 2 to 8 cysteine residues,
said two or more cysteine residue-modifying moieties may be 2 to 8 cysteine residue-modifying moieties, and
the 2 to 8 cysteine residue-modifying moieties may be connected to the immunoglobulin unit by bonding to the sulfur atoms in the side chains of 2 to 8 cysteine residues in said two heavy chains and two light chains.

In a preferred embodiment, said two or more cysteine residues may be 8 cysteine residues,
said two or more cysteine residue-modifying moieties may be 8 cysteine residue-modifying moieties, and
the 8 cysteine residue-modifying moieties may be connected to the immunoglobulin unit by bonding to the sulfur atoms in the side chains of 8 cysteine residues in said two heavy chains and two light chains.

In a preferred embodiment, the lysine residue-modifying moieties may be represented by the following formula (I-2): 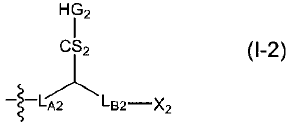 wherein
the hyphen (-) with a wavy line represents a bond to said nitrogen atom,
HG₂ represents a hydrophilic group or a monovalent group containing a hydrophilic group,
CS₂ represents a divalent group containing a cleavable site,
L_{A2} and L_{B2} each independently represent a divalent group, and
X₂ represents a functional substance,
or the following formula (II): 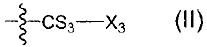 wherein
the hyphen (-) with a wavy line represents a bond to said nitrogen atom,
CS₃ represents a divalent group optionally containing a cleavable site, and
X₃ represents a functional substance.

In a preferred embodiment, the two or more lysine residues may be any of the lysine residues present at the following positions according to Eu numbering:
(A) a lysine residue present at position 246/248 in the two heavy chains;
(B) a lysine residue present at position 288/290 in the two heavy chains;
(C) a lysine residue present at position 317 in the two heavy chains; and
(D) a combination of (A) and (B), (A) and (C), or (B) and (C), or (A), (B), and (C).

In a preferred embodiment, the two or more lysine residue-modifying moieties may be regioselectively connected to the immunoglobulin unit by bonding to the nitrogen atoms in the side chains of two lysine residues present at the same position in said two heavy chains.

In a preferred embodiment, the two or more lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). Here, the first lysine residue-modifying moiety may be represented by the following formula (I-2-1): 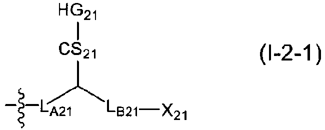 wherein
the hyphen (-) with a wavy line represents a bond to said nitrogen atom,
HG₂₁ represents a hydrophilic group or a monovalent group containing a hydrophilic group,
CS₂₁ represents a divalent group containing a cleavable site,
L_{A21} and L_{B21} each independently represent a divalent group, and
X₂₁ represents a functional substance,
or the following formula (II-1): 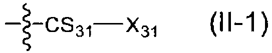 wherein
the hyphen (-) with a wavy line represents a bond to said nitrogen atom,
CS₃₁ represents a divalent group optionally containing a cleavable site, and
X₃₁ represents a functional substance,
   and
the second lysine residue-modifying moiety may be represented by the following formula (I-2-2): 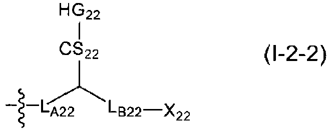 wherein
the hyphen (-) with a wavy line represents a bond to said nitrogen atom,
HG₂₂ represents a hydrophilic group or a monovalent group containing a hydrophilic group,
CS₂₂ represents a divalent group containing a cleavable site,
L_{A22} and L_{B22} each independently represent a divalent group, and
X₂₂ represents a functional substance,
or the following formula (II-2): 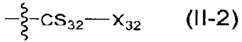 wherein
the hyphen (-) with a wavy line represents a bond to said nitrogen atom,
CS₃₂ represents a divalent group optionally containing a cleavable site, and
X₃₂ represents a functional substance.

In a preferred embodiment, the hydrophilic group may be one or more groups selected from the group consisting of a carboxylic acid group, a sulfonic acid group, a sulfamide group, a hydroxyl group, a polyethylene glycol group, a polysarcosine group, and a sugar moiety.

In a preferred embodiment, the cleavable site may be a site cleavable by an enzyme.

In a preferred embodiment, the site cleavable by an enzyme may be a peptidic site.

In a preferred embodiment, the functional substance may be a pharmaceutical agent, a labeling substance, or a stabilizing agent.

In a preferred embodiment, the conjugation ratio of the functional substance to the antibody (functional substance/antibody) is 10 or more.

In a preferred embodiment, said regioselective conjugate may show an aggregation rate of 2.6% or less as determined by size exclusion chromatography.

In a preferred embodiment, the cysteine residue-modifying moiety may be represented by the following formula (I-1'): 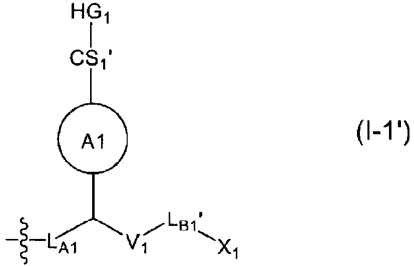 wherein
the hyphen (-) with a wavy line represents a bond to said sulfur atom,
HG₁ represents a hydrophilic group or a monovalent group containing a hydrophilic group,
CS₁' represents a divalent group containing a cleavable site,
Ring A1 represents an optionally substituted divalent aromatic ring group (wherein the divalent aromatic ring group forms a pi electron conjugated system with said cleavable site),
V₁ represents an oxygen atom, a sulfur atom, or an amino group (NH),
L_{A1} and L_{B1}' each independently represent a divalent group, and
X₁ represents a functional substance,
   and
the lysine residue-modifying moiety may be represented by the following formula (I-2'): 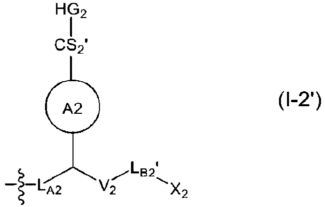 wherein
the hyphen (-) with a wavy line represents a bond to said nitrogen atom,
HG₂ represents a hydrophilic group or a monovalent group containing a hydrophilic group,
CS₂' represents a divalent group containing a cleavable site,
Ring A2 represents an optionally substituted divalent aromatic ring group (wherein the divalent aromatic ring group forms a pi electron conjugated system with said cleavable site),
V₂ represents an oxygen atom, a sulfur atom, or an amino group (NH),
L_{A2} and L_{B2}' each independently represent a divalent group, and
X₂ represents a functional substance,
or the following formula (II): 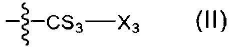 wherein
the hyphen (-) with a wavy line represents a bond to said nitrogen atom,
CS₃ represents a divalent group optionally containing a cleavable site, and
X₃ represents a functional substance.

In a preferred embodiment, the optionally substituted divalent aromatic ring group may be an optionally substituted phenylene group.

A preferred embodiment may be (A) or (B) below:
(A) the lysine residue-modifying moieties are regioselectively connected to the immunoglobulin unit by bonding to the nitrogen atoms in the side chains of two lysine residues present at the same position in said two heavy chains, or
(B) the lysine residue-modifying moieties comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i),
wherein
the first lysine residue-modifying moiety is represented by the following formula (I-2-1'): 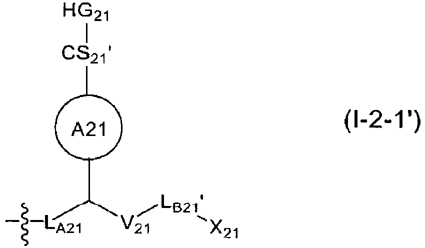 wherein
the hyphen (-) with a wavy line represents a bond to said nitrogen atom,
HG₂₁ represents a hydrophilic group or a monovalent group containing a hydrophilic group,
CS₂₁' represents a divalent group containing a cleavable site,
Ring A21 represents an optionally substituted divalent aromatic ring group (wherein the divalent aromatic ring group forms a pi electron conjugated system with said cleavable site),
V₂₁ represents an oxygen atom, a sulfur atom, or an amino group (NH),
L_{A21} and L_{B21}' each independently represent a divalent group, and
X₂₁ represents a functional substance,
or the following formula (II-1): 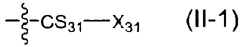 wherein
the hyphen (-) with a wavy line represents a bond to said nitrogen atom,
CS₃₁ represents a divalent group optionally containing a cleavable site, and
X₃₁ represents a functional substance,
   and
the second lysine residue-modifying moiety is represented by the following formula (I-2-2'): 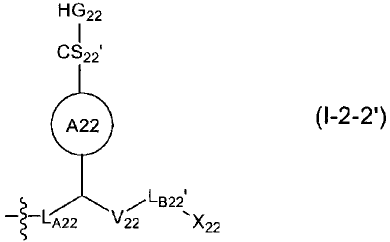 wherein
the hyphen (-) with a wavy line represents a bond to said nitrogen atom,
HG₂₂ represents a hydrophilic group or a monovalent group containing a hydrophilic group,
CS₂₂' represents a divalent group containing a cleavable site,
Ring A22 represents an optionally substituted divalent aromatic ring group (wherein the divalent aromatic ring group forms a pi electron conjugated system with said cleavable site),
V₂₂ represents an oxygen atom, a sulfur atom, or an amino group (NH),
L_{A22} and L_{B22}' each independently represent a divalent group, and
X₂₂ represents a functional substance,
or the following formula (II-2): 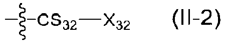 wherein
the hyphen (-) with a wavy line represents a bond to said nitrogen atom,
CS₃₂ represents a divalent group optionally containing a cleavable site, and
X₃₂ represents a functional substance.

In a preferred embodiment, the cysteine residue-modifying moiety may be represented by the following formula (I-1"): 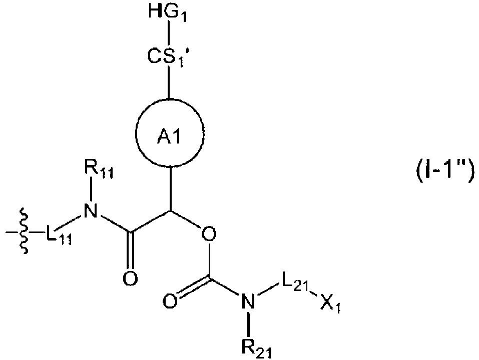 wherein
the hyphen (-) with a wavy line represents a bond to said sulfur atom,
HG₁ represents a hydrophilic group or a monovalent group containing a hydrophilic group,
CS₁' represents a divalent group containing a cleavable site,
Ring A1 represents an optionally substituted divalent aromatic ring group (wherein the divalent aromatic ring group forms a pi electron conjugated system with said cleavable site),
R₁₁ and R₂₁ each independently represent a hydrogen atom or a monovalent group,
L₁₁ and L₂₁ each independently represent a divalent group, and
X₁ represents a functional substance,
   and
the lysine residue-modifying moiety may be represented by the following formula (I-2"): 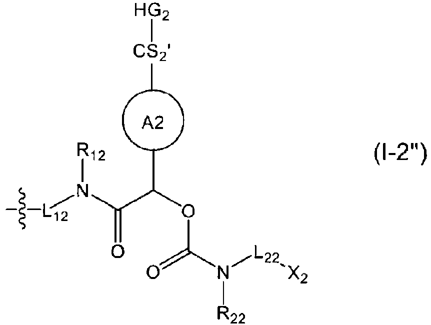 wherein
the hyphen (-) with a wavy line represents a bond to said nitrogen atom,
HG₂ represents a hydrophilic group or a monovalent group containing a hydrophilic group,
CS₂' represents a divalent group containing a cleavable site,
Ring A2 represents an optionally substituted divalent aromatic ring group (wherein the divalent aromatic ring group forms a pi electron conjugated system with said cleavable site),
R₁₂ and R₂₂ each independently represent a hydrogen atom or a monovalent group,
L₁₂ and L₂₂ each independently represent a divalent group, and
X₂ represents a functional substance,
or the following formula (II): 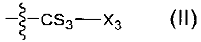 wherein
the hyphen (-) with a wavy line represents a bond to said nitrogen atom,
CS₃ represents a divalent group optionally containing a cleavable site, and
X₃ represents a functional substance.

A preferred embodiment may be (A) or (B) below:
(A) the lysine residue-modifying moieties are regioselectively connected to the immunoglobulin unit by bonding to the nitrogen atoms in the side chains of two lysine residues present at the same position in said two heavy chains, or
(B) the lysine residue-modifying moieties comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i),
wherein
the first lysine residue-modifying moiety may be represented by the following formula (I-2-1"): 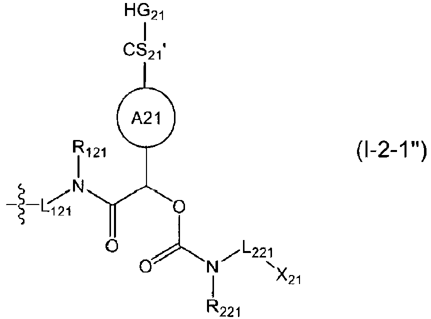 wherein
the hyphen (-) with a wavy line represents a bond to said nitrogen atom,
HG₂₁ represents a hydrophilic group or a monovalent group containing a hydrophilic group,
CS₂₁' represents a divalent group containing a cleavable site,
Ring A21 represents an optionally substituted divalent aromatic ring group (wherein the divalent aromatic ring group forms a pi electron conjugated system with said cleavable site),
R₁₂₁ and R₂₂₁ each independently represent a hydrogen atom or a monovalent group,
L₁₂₁ and L₂₂₁ each independently represent a divalent group, and
X₂₁ represents a functional substance,
or the following formula (II-1): 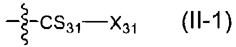 wherein
the hyphen (-) with a wavy line represents a bond to said nitrogen atom,
CS₃₁ represents a divalent group optionally containing a cleavable site,
X₃₁ represents a functional substance,
   and
the second lysine residue-modifying moiety may be represented by the following formula (I-2-2"): 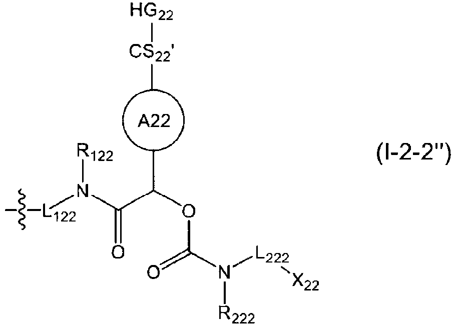 wherein
the hyphen (-) with a wavy line represents a bond to said nitrogen atom,
HG₂₂ represents a hydrophilic group or a monovalent group containing a hydrophilic group,
CS₂₂' represents a divalent group containing a cleavable site,
Ring A22 represents an optionally substituted divalent aromatic ring group (wherein the divalent aromatic ring group forms a pi electron conjugated system with said cleavable site),
R₁₂₂ and R₂₂₂ each independently represent a hydrogen atom or a monovalent group,
L₁₂₂ and L₂₂₂ each independently represent a divalent group, and
X₂₂ represents a functional substance.
or the following formula (II-2): 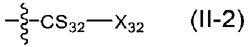 wherein
the hyphen (-) with a wavy line represents a bond to said nitrogen atom,
CS₃₂ represents a divalent group optionally containing a cleavable site, and
X₃₂ represents a functional substance.

In a second embodiment, the present invention provides an antibody intermediate having thiol groups, or a salt thereof, wherein
(1) the antibody intermediate comprises an immunoglobulin unit comprising two heavy chains and two light chains, and
(2) said immunoglobulin unit comprises
   (a') two or more thiol groups present in the side chains of two or more cysteine residues in said two heavy chains and two light chains, and
   (b') two or more lysine residue-modifying moieties regioselectively connected to the immunoglobulin unit by bonding to the nitrogen atoms in the side chains of two or more lysine residues in said two heavy chains.

In a preferred embodiment, the immunoglobulin unit may be a human immunoglobulin unit.

In a preferred embodiment, the human immunoglobulin unit may be a human IgG antibody.

In a preferred embodiment, said two or more cysteine residues may be 2 to 8 cysteine residues, and
said two or more thiol groups may be 2 to 8 thiol groups.

In a preferred embodiment, said two or more cysteine residues may be 8 cysteine residues, and
said two or more thiol groups may be 8 thiol groups.

In a preferred embodiment, the lysine residue-modifying moiety may be represented by the following formula (III): 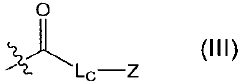 wherein
the hyphen (-) with a wavy line represents a bond to said nitrogen atom,
L_{C} represents a divalent group, and
Z represents a bioorthogonal functional group or a functional substance, or a monovalent group containing a bioorthogonal functional group or a functional substance.

In a preferred embodiment, the two or more lysine residues may be any of the lysine residues present at the following positions according to Eu numbering:
(A) a lysine residue present at position 246/248 in the two heavy chains;
(B) a lysine residue present at position 288/290 in the two heavy chains;
(C) a lysine residue present at position 317 in the two heavy chains; and
(D) a combination of (A) and (B), (A) and (C), or (B) and (C), or (A), (B), and (C).

In a preferred embodiment, the two or more lysine residue-modifying moieties may be regioselectively connected to the immunoglobulin unit by bonding to the nitrogen atoms in the side chains of two lysine residues present at the same position in said two heavy chains.

In a preferred embodiment, the two or more lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). Here, the first lysine residue-modifying moiety may be represented by the following formula (III-1): 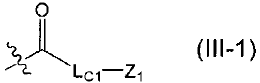 wherein
the hyphen (-) with a wavy line represents a bond to said nitrogen atom,
L_{C1} represents a divalent group, and
Z₁ represents a bioorthogonal functional group or a functional substance, or a monovalent group containing a bioorthogonal functional group or a functional substance,
   and
the second lysine residue-modifying moiety may be represented by the following formula (III-2): 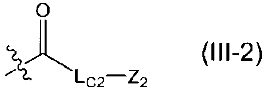 wherein
   the hyphen (-) with a wavy line represents a bond to said nitrogen atom,
   L_{C1} represents a divalent group, and
   Z₂ represents a bioorthogonal functional group or a functional substance, or a monovalent group containing a bioorthogonal functional group or a functional substance.

In a preferred embodiment, the bioorthogonal functional group may be selected from the group consisting of an azide group, an aldehyde group, a thiol group, an alkyne group, an alkene group, a halogen group, a tetrazine group, a nitrone group, a hydroxylamine group, a nitrile group, a hydrazine group, a ketone group, a boronic acid group, a cyanobenzothiazole group, an allyl group, a phosphine group, a maleimide group, a disulfide group, a thioester group, an α-halocarbonyl group, an isonitrile group, a sydnone group, and a selenium group.

In a preferred embodiment, the bioorthogonal functional group may be a thiol group.

In a preferred embodiment, L_{C} may be an alkylene group.

### EFFECTS OF THE INVENTION

The conjugate or a salt thereof of the present invention can have excellent properties such as long in vivo residence time and high monomer ratio (low aggregation rate).

The antibody derivatives and compounds, or salts thereof, and reagents of the present invention are useful, for example, as synthetic intermediates in the production of the conjugate.

### BRIEF DESCRIPTION OF THE DRAWINGS

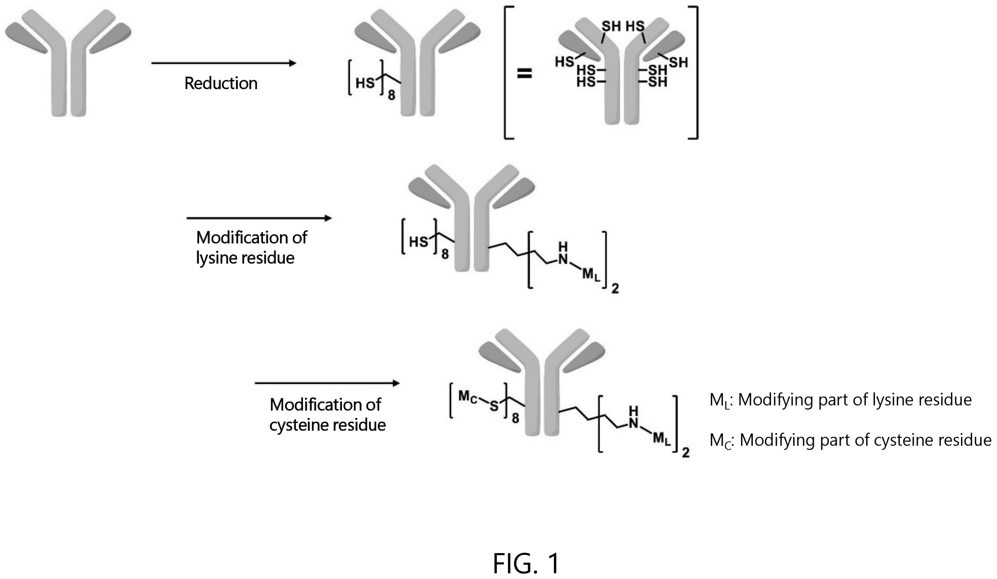

FIG. 1 shows a synthesis example of the conjugate or a salt thereof of the present invention.

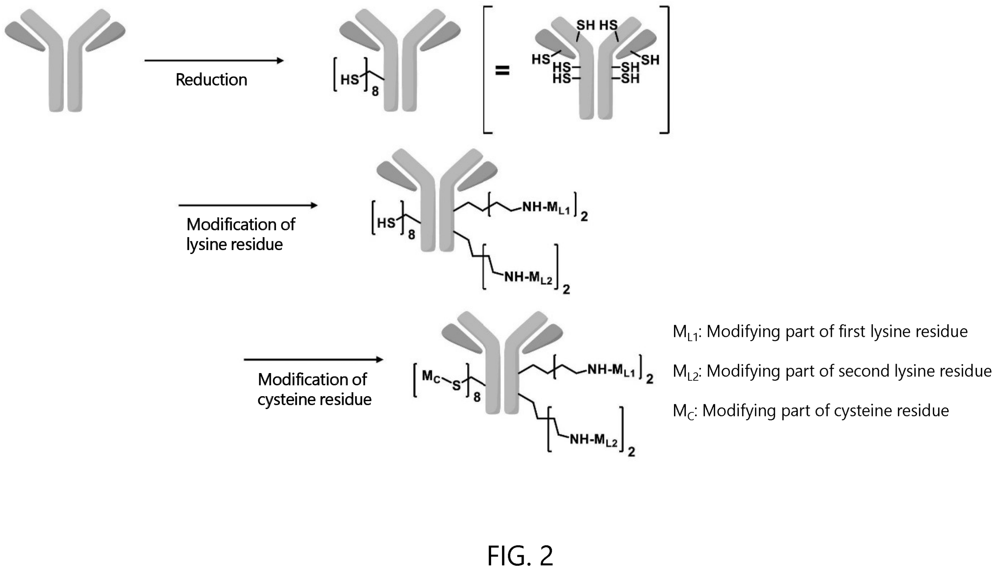

FIG. 2 shows another synthesis example of the conjugate or a salt thereof of the present invention.

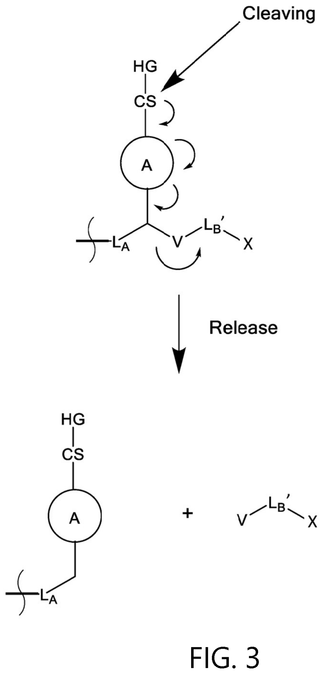

FIG. 3 shows an example of the release of a drug (an active form of the drug in which steric hindrance by the antibody has been eliminated) by the cleavage of a cleavable site and the involvement of a pi electron resonance system. In one embodiment, the conjugate, antibody derivative, and compound of the present invention can be designed to enable such release (refer to Background Art as appropriate). The antibody can retain the hydrophilic group even after cleavage. That is, before cleavage, the drug is linked to a hydrophilic group in the form of a conjugate with an antibody, and thus the hydrophobicity of the drug can be attenuated (masking by the hydrophilic group), while after cleavage, the hydrophilic group dissociates from the drug, allowing the drug to exert its original hydrophobicity.
FIG. 4 shows the interrelationships of substances such as the conjugates of the invention having the portion represented by formula (I-1).

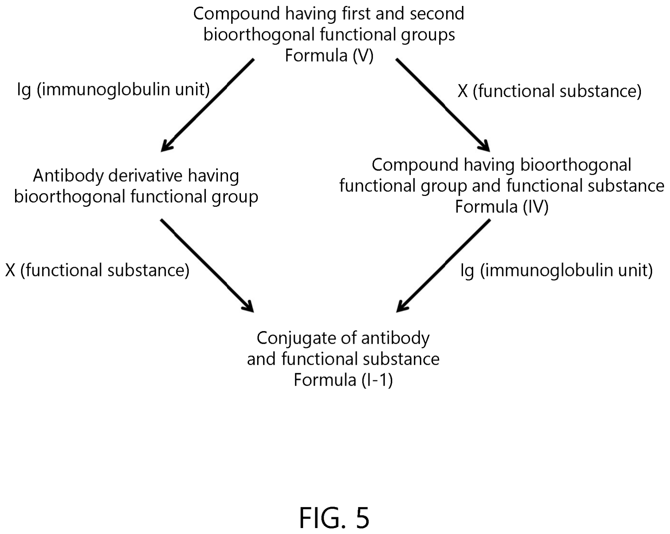

FIG. 5 shows a synthesis outline of substances such as the conjugates of the invention having the portion represented by formula (I-1).
FIG. 6 shows an example of a synthesis outline of substances such as the compound represented by formula (V).
FIG. 7 shows an example of a preferred synthesis outline of substances such as the compound represented by formula (V).
DIPEA: N,N-diisopropylethylamine; DMF: N,N-dimethylformamide

### MODE FOR CARRYING OUT THE INVENTION

### 1. Definitions of General Terms

In the present invention, the term "antibody" has the following meaning. The term "immunoglobulin unit" corresponds to a divalent monomer unit that is the basic constituent element of such an antibody, and is a unit comprising two heavy chains and two light chains. Therefore, for the immunoglobulin unit, the definitions, examples, and preferred examples of its origin, type (polyclonal or monoclonal, isotype, and full-length antibody or antibody fragment), antigen, and position of cysteine residues are the same as those for the antibody described below.

The origin of the antibody is not particularly limited, and for example, the antibody may be derived from an animal such as a mammal or a bird (e.g., a chicken). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), livestock (e.g., cows, pigs, and goats), and working animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bispecific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or a variable region, and an antibody fragment comprising a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include proteins [including oligopeptides and polypeptides, or they may be proteins modified with a biomolecule such as a sugar (e.g., glycoproteins)], sugar chains, nucleic acids, and small compounds. The antibody may be preferably an antibody that recognizes a protein as an antigen. Examples of the protein include cell membrane receptors, cell membrane proteins other than cell membrane receptors (e.g., extracellular matrix proteins), ligands, and soluble receptors.

More specifically, the protein as an antigen of the antibody may be a disease target protein. Examples of the disease target protein include the following.

### (1) Oncology

PD-L1, GD2, PDGFRα (platelet-derived growth factor receptor), CD22, HER2, phosphatidylserine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1(CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137(4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72(CA72-4), and CD70

### (2) Autoimmune and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Cranial nerve diseases

CGRP, CD20, β amyloid, β amyloid protofibril, Calcitonin Gene-Related Peptide Receptor, LINGO (Ig Domain Containing 1), α-Synuclein, extracellular tau, CD52, insulin receptor, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary and Rare diseases

amyloid AL, SEMA4D (CD100), insulin receptor 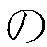 , ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD105 (Endoglin), IGF-1R, and β amyloid.

### (7) Bone and Orthopedic field

Sclerostin, Myostatin, Dickkopf-1, GDF8, RNAKL, HAS, and Siglec-15.

### (8) Blood Diseases

vWF, Factor IXa, Factor X, IFNγ, C5, BMP-6, Ferroportin, and TFPI.

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

An immunoglobulin unit composed of two heavy chains and two light chains (e.g., a divalent antibody such as IgG) has four disulfide bonds because the heavy-heavy chains and the heavy-light chains are linked by four disulfide bonds. When a reducing agent is allowed to act sufficiently on such an immunoglobulin unit, eight thiol groups are generated from the four disulfide bonds. As the reducing agent, any reducing agent capable of cleaving a disulfide bond to generate a thiol group can be used, and examples thereof include tricarboxylethylphosphine (TCEP), cysteine, dithiothreitol, reduced glutathione, and β-mercaptoethanol. Incidentally, even when all four disulfide bonds are reduced, the heavy and light chains of the antibody do not dissociate. This is because the higher-order structure of the antibody can be maintained by non-covalent bonds between the chains. These non-covalent bonds also maintain the antibody properties (target and binding affinity). For example, trastuzumab deruxtecan, known as Enhertu^{®}, is an ADC with a DAR = 8, in which drugs are conjugated to the thiol groups generated by reducing (cleaving) all four interchain disulfide bonds. Such ADCs are known to act as antigen-specific agents by maintaining their antibody properties.

According to the present invention, specific lysine residues (e.g., lysine residues at positions 246/248, 288/290, or 317) in the heavy chain of the immunoglobulin unit constituting the antibody can be regioselectively modified (see, e.g., WO2018/199337, WO2019/240288, WO2019/240287, and WO2020/090979). In the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%.

Furthermore, in the present invention, an immunoglobulin unit comprising two heavy chains and two light chains can be bonded to a modifying group adjacent to Ig (e.g., LA, L1 described later) via the thiol groups in the side chains of a plurality of cysteine residues (a plurality of cysteine residues in the two heavy chains and two light chains contained in the immunoglobulin unit) generated by allowing a reducing agent to act on the immunoglobulin unit. The number corresponding to the plurality is, for example, 2 or more (e.g., 2 to 8), preferably 3 or more (e.g., 3 to 8), more preferably 4 or more (e.g., 4 to 8), still more preferably 5 or more (e.g., 5 to 8), and particularly preferably 6 or more (e.g., 6 to 8), 7 or more (e.g., 7 to 8), or 8.

Furthermore, in the present invention, as long as the lysine residues and/or cysteine residues as described above in the immunoglobulin unit or antibody are modified, specific amino acid residues at other positions may be further regioselectively modified. For example, various methods are known for regioselectively modifying a specific amino acid residue at a certain position in an immunoglobulin unit or antibody. As such a specific amino acid residue, an amino acid residue having a side chain that is easily modified (e.g., amino group, carboxyl group, amide group, hydroxyl group) (e.g., aspartic acid residue, glutamic acid residue, asparagine residue, glutamine residue, threonine residue, serine residue, tyrosine residue) can be used, but preferably, a tyrosine residue, serine residue, and threonine residue having a side chain containing a hydroxyl group can be utilized.

### Halogen atoms

Examples of the halogen atom include a fluorine atom, a chlorine atom, a bromine atom, and an iodine atom.

### Monovalent groups

Examples of the monovalent group include a monovalent hydrocarbon group and a monovalent heterocyclic group.

The monovalent group may be substituted by one or more (e.g., 1 to 10, preferably 1 to 8, more preferably 1 to 6, still more preferably 1 to 5, particularly preferably 1 to 3) substituents as described below.

### Monovalent hydrocarbon groups and related terms

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in the main chain thereof. Note that the chain structure may be linear or branched. Examples of the monovalent chain hydrocarbon group include alkyl, alkenyl, and alkynyl. The alkyl, alkenyl, and alkynyl may be linear or branched.

The alkyl is preferably C1-C12 alkyl, more preferably C1-C6 alkyl, and still more preferably C1-C4 alkyl. When the alkyl has a substituent, the number of carbon atoms does not include the number of carbon atoms of the substituent. Examples of the C1-C12 alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C2-C12 alkenyl, more preferably C2-C6 alkenyl, and still more preferably C2-C4 alkenyl. When the alkenyl has a substituent, the number of carbon atoms does not include the number of carbon atoms of the substituent. Examples of the C2-C12 alkenyl include vinyl, propenyl, and n-butenyl.

The alkynyl is preferably C2-C12 alkynyl, more preferably C2-C6 alkynyl, and still more preferably C2-C4 alkynyl. When the alkyl has a substituent, the number of carbon atoms does not include the number of carbon atoms of the substituent. Examples of the C2-C12 alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

Cycloalkyl is preferably C3-C12 cycloalkyl, more preferably C3-C6 cycloalkyl, and still more preferably C5-C6 cycloalkyl. When the cycloalkyl has a substituent, the number of carbon atoms does not include the number of carbon atoms of the substituent. Examples of the C3-C12 cycloalkyl include cyclopropyl, cyclobutyl, cyclopentyl, and cyclohexyl.

Cycloalkenyl is preferably C3-C12 cycloalkenyl, more preferably C3-C6 cycloalkenyl, and still more preferably C5-C6 cycloalkenyl. When the cycloalkenyl has a substituent, the number of carbon atoms does not include the number of carbon atoms of the substituent. Examples of the C3-C12 cycloalkenyl include cyclopropenyl, cyclobutenyl, cyclopentenyl, and cyclohexenyl.

Cycloalkynyl is preferably C3-C12 cycloalkynyl, more preferably C3-C6 cycloalkynyl, and still more preferably C5-C6 cycloalkynyl. When the cycloalkynyl has a substituent, the number of carbon atoms does not include the number of carbon atoms of the substituent. Examples of the C3-C12 cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C6-C12 aryl, more preferably C6-C10 aryl, and still more preferably C6 aryl. When the monovalent aromatic hydrocarbon group has a substituent, the number of carbon atoms does not include the number of carbon atoms of the substituent. Examples of the C6-C12 aryl include phenyl and naphthyl.

The monovalent aromatic hydrocarbon group is preferably phenyl.

Among these groups, the monovalent hydrocarbon group is preferably alkyl, cycloalkyl, or aryl.

### Monovalent heterocyclic group and related terms

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom, and more preferably comprises one or more selected from the group consisting of an oxygen atom, a sulfur atom, and a nitrogen atom as a heteroatom comprised in the heterocyclic group.

The monovalent aromatic heterocyclic group is preferably a C1-C15 aromatic heterocyclic group, more preferably a C1-C9 aromatic heterocyclic group, and still more preferably a C1-C6 aromatic heterocyclic group. When the monovalent aromatic heterocyclic group has a substituent, the number of carbon atoms does not include the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C2-C15 nonaromatic heterocyclic group, more preferably a C2-C9 nonaromatic heterocyclic group, and still more preferably a C2-C6 nonaromatic heterocyclic group. When the monovalent nonaromatic heterocyclic group has a substituent, the number of carbon atoms does not include the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

### Divalent groups

The divalent group is a group having a main chain structure containing one group or 2 or more (e.g., 2 to 10, preferably 2 to 8, more preferably 2 to 6, still more preferably 2 to 5, and particularly preferably 2 or 3) groups selected from the group consisting of a divalent linear hydrocarbon group, a divalent cyclic hydrocarbon group, a divalent heterocyclic group, -C(=O)-, -C(=S)-, -NR7-, -C(=O)-NR7-, -NR7-C(=O)-, -C(=S)-NR7-, -NR7-C(=S)-, -O-, -S-, -(O-R₈)ₘ-, and -(S-R₈)ₘ₁-. R7 indicates a hydrogen atom or a substituent as described below. R8 indicates a divalent linear hydrocarbon group, a divalent cyclic hydrocarbon group, or a divalent heterocyclic group. m1 is an integer of 1 to 10, preferably an integer of 1 to 8, more preferably an integer of 1 to 6, still more preferably an integer of 1 to 5, and particularly preferably an integer of 1 to 3.

The divalent linear hydrocarbon group is a linear alkylene, a linear alkenylene, or a linear alkynylene.

The linear alkylene is a C1-C6 linear alkylene, and is preferably a C1-C4 linear alkylene. Examples of the linear alkylene include methylene, ethylene, n-propylene, n-butylene, n-pentylene, and n-hexylene. The linear alkenylene is a C2-C6 linear alkenylene, and is preferably a C2-C4 linear alkenylene. Examples of the linear alkenylene include ethylenylene, n-propynylene, n-butenylene, n-pentenylene, and n-hexenylene. The linear alkynylene is a C2-C6 linear alkynylene, and is preferably a C2-C4 linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C6-C14 arylene, more preferably a C6-C10 arylene, and particularly preferably a C6 arylene. Examples of the arylene include phenylene, naphthylene, and anthracenylene.

The divalent nonaromatic cyclic hydrocarbon group is preferably a C3-C12 monocyclic or polycyclic divalent nonaromatic cyclic hydrocarbon group, more preferably a C4-C10 monocyclic or polycyclic divalent nonaromatic cyclic hydrocarbon group, and particularly preferably a C5-C8 monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a heteroatom forming a heterocycle, one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom.

The divalent aromatic heterocyclic group is preferably a C3-C15 divalent aromatic heterocyclic group, more preferably a C3-C9 divalent aromatic heterocyclic group, and particularly preferably a C3-C6 divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C3-C15 nonaromatic heterocyclic group, more preferably a C3-C9 nonaromatic heterocyclic group, and particularly preferably a C3-C6 nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

Preferably, the divalent group is a divalent group having a main chain structure containing one group selected from the group consisting of alkylene, arylene, -C(=O)-, -NR7-, -C(=O)-NR7-, -NR7-C(=O)-, -O-, and -(O-R₈)ₘ-, or
a divalent group having a main chain structure containing two or more selected from the group consisting of alkylene, arylene, -C(=O)-, -NR7-, -C(=O)-NR7-, -NR7-C(=O)-, -O-, and - (O-R₈)ₘ₁-,
R7 is a hydrogen atom or alkyl,
R8 is alkylene or arylene,
m1 may be an integer of 1 to 5 (or 1, 2, 3, 4, or 5). The alkylene, arylene, and alkyl are similar to those described above.

The main chain structure in the divalent group may be substituted by one or more (e.g., 1 to 10, preferably 1 to 8, more preferably 1 to 6, still more preferably 1 to 5, particularly preferably 1 to 3) substituents as described below.

### Substituents

Examples of the substituents include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O- (Rₐ indicates a hydrogen atom, or a monovalent hydrocarbon group); or
(vi) NR_{b}R_{c}-, NR_{b}R_{c}-C(=O)-, NR_{b}R_{c}-C(=O)-O-, or R_{b}-C(=O)-NR_{c}- (R_{b} and R_{c} are the same or different from each other, and each represents a hydrogen atom or a monovalent hydrocarbon group);
(vii) a nitro group, a sulfate group, a sulfonic acid group, a cyano group, and a carboxyl group.

The definitions, examples, and preferred examples of the halogen atom, the monovalent hydrocarbon group, and the monovalent heterocyclic group in the substituent are similar to those described above.

The aralkyl refers to arylalkyl. The definitions, examples, and preferred examples of the aryl and the alkyl in the arylalkyl are as described above. The aralkyl is preferably C3-C15 aralkyl. Examples of such an aralkyl include benzoyl, phenethyl, naphthylmethyl, and naphthylethyl.

Preferably, the substituent may be:
(i) a halogen atom;
(ii) a C1-C12 alkyl, a phenyl, or a naphthyl;
(iii) a C3-CC15 aralkyl;
(iv) a 5- or 6-membered heterocycle;
(v) Rₐ-O-, Rₐ-C(=O)-, Rₐ-O-C(=O)-, or Rₐ-C(=O)-O- (where Rₐ represents a hydrogen atom, or a C1-C12 alkyl);
(vi) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same as or different from each other, and each represents a hydrogen atom or a C1-C12 alkyl); or
(vii) the same groups as listed in item (vii) above.

More preferably, the substituent may be:
(i) a halogen atom;
(ii) a C1-C12 alkyl;
(iii) Rₐ-O-, Rₐ-C(=O)-, Rₐ-O-C(=O)-, or Rₐ-C(=O)-O-, (where Rₐ represents a hydrogen atom or a C1-C12 alkyl);
(iv) NR_{b}R_{c}-, NR_{b}R_{c}-C(=O)-, NR_{b}R_{c}-C(=O)-O-, or Rb-C(=O)-NR_{c}- (where R_{b} and R_{c} are the same as or different from each other, and each represents a hydrogen atom or a C1-C12 alkyl); or
(v) the same groups as listed in item (vii) above.

Even more preferably, the substituent may be:
(i) a halogen atom;
(ii) a C1-C6 alkyl;
(iii) Rₐ-O-, Rₐ-C(=O)-, Rₐ-O-C(=O)-, or Rₐ-C(=O)-O- (where Rₐ represents a hydrogen atom or a C1-C6 alkyl);
(iv) NR_{b}R_{c}-, NR_{b}R_{c}-C(=O)-, NR_{b}R_{c}-C(=O)-O-, or Rb-C(=O)-NR_{c}- (where R_{b} and R_{c} are the same as or different from each other, and each represents a hydrogen atom or a C1-C6 alkyl); or
(v) the same groups as listed in item (vii) above.

Particularly preferably, the substituent may be:
(i) a halogen atom;
(ii) a C1-C4 alkyl;
(iii) Rₐ-O-, Rₐ-C(=O)-, Rₐ-O-C(=O)-, or Rₐ-C(=O)-O- (where Rₐ represents a hydrogen atom or a C1-C4 alkyl);
(iv) NR_{b}R_{c}-, NR_{b}R_{c}-C(=O)-, NR_{b}R_{c}-C(=O)-O-, or Rb-C(=O)-NR_{c}- (where R_{b} and R_{c} are the same as or different from each other, and each represents a hydrogen atom or a C1-C4 alkyl); or
(v) the same groups as listed in item (vii) above.

### Hydrophilic groups

A hydrophilic group is a group that can make the structural unit represented by formulas (1) to (7) or a sub-formula thereof more hydrophilic. By having a hydrophilic group at a certain site in said structural unit, the properties of the conjugate can be further improved. Examples of such a hydrophilic group include a carboxylic acid group, a sulfonic acid group, a sulfamide group, a hydroxyl group, a polyethylene glycol group, a polysarcosine group, and a sugar moiety. One or more (e.g., 1, 2, 3, 4, or 5) hydrophilic groups may be contained in the conjugate.

A polyethylene glycol (PEG) group is a divalent group represented by -(CH2-CH2-O-)ₖ₁-. When the conjugate has a polyethylene glycol group, the conjugate may have a monovalent group in which one bond of the polyethylene glycol group is bonded to a hydrogen atom or a monovalent group (e.g., a monovalent hydrocarbon group). k1 may be, for example, an integer of 3 or more, preferably 4 or more, more preferably 5 or more, and still more preferably 6 or more. k1 may also be an integer of 15 or less, preferably 12 or less, more preferably 10 or less, and still more preferably 9 or less. More specifically, k1 may be an integer from 3 to 15, preferably from 4 to 12, more preferably from 5 to 10, and still more preferably from 4 to 9.

A polysarcosine group is a divalent group represented by -(NCH3-CH2-CO-)ₖ₂-. A polysarcosine group can be used as a substitute for PEG. k2 may be, for example, an integer of 3 or more, preferably 4 or more, more preferably 5 or more, and still more preferably 6 or more. k2 may also be an integer of 15 or less, preferably 12 or less, more preferably 10 or less, and still more preferably 9 or less. More specifically, k2 may be an integer from 3 to 15, preferably from 4 to 12, more preferably from 5 to 10, and still more preferably from 4 to 9.

The sugar moiety is a monosaccharide, an oligosaccharide (e.g., disaccharide, trisaccharide, tetrasaccharide, pentasaccharide), or a polysaccharide. The sugar moiety can include an aldose or a ketose, or a combination thereof. The sugar moiety may be a monosaccharide such as ribose, deoxyribose, xylose, arabinose, glucose, mannose, galactose, or fructose, or an amino sugar (e.g., glucosamine), or an oligosaccharide or polysaccharide containing such a monosaccharide.

In a specific embodiment, the sugar moiety may be a low molecular weight hydrophilic group. A low molecular weight hydrophilic group refers to a hydrophilic group with a molecular weight of 1500 or less. The molecular weight of the low molecular weight hydrophilic group may be preferably 1200 or less, 1000 or less, 800 or less, 700 or less, 600 or less, 500 or less, 400 or less, 300 or less, 200 or less, or 100 or less. Examples of the low molecular weight hydrophilic group include a carboxylic acid group, a sulfonic acid group, and a hydroxyl group; and a polyethylene glycol group, a polysarcosine group, and a sugar moiety (e.g., monosaccharide, oligosaccharide) that satisfy the above molecular weight.

### Monovalent group containing a hydrophilic group

The monovalent group containing a hydrophilic group is a monovalent group containing a hydrophilic group as described above. The monovalent group is as described above.

Preferably, the monovalent group containing a hydrophilic group may be a monovalent group represented by the following formula (A):

N(R_{HG1})(R_{HG2})-L_{HG}- (A)

wherein
L_{HG} represents a bond or a divalent group optionally containing a hydrophilic group,
R_{HG1} and R_{HG2} each independently represent a hydrogen atom, a hydrophilic group, or a monovalent group optionally containing a hydrophilic group,
at least one hydrophilic group is contained in one or more moieties selected from the group consisting of L_{HG}, R_{HG1}, and R_{HG2}. The hyphen (-) placed at the right end in formula (A) indicates a bond.

L_{HG} represents a bond or a divalent group optionally containing a hydrophilic group. When R_{HG1} and R_{HG2} are each independently a hydrogen atom or a monovalent group not containing a hydrophilic group, L_{HG} is a divalent group containing a hydrophilic group. When at least one of R_{HG1} and R_{HG2} is a hydrophilic group or a monovalent group containing a hydrophilic group, L_{HG} may be a divalent group optionally containing a hydrophilic group, but is also preferably a divalent group not containing a hydrophilic group.

R_{HG1} and R_{HG2} each independently represent a hydrogen atom, a hydrophilic group, or a monovalent group optionally containing a hydrophilic group. The hydrophilic group and the monovalent group are as described above.

**In** a specific embodiment, the monovalent group optionally containing a hydrophilic group, represented by R_{HG1} and R_{HG2}, may be a protecting group for an amino group. Also, one of R_{HG1} and R_{HG2} may be a hydrogen atom, and the other may be a protecting group for an amino group. Examples of the protecting group for an amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group).

Alternatively, one of R_{HG1} and R_{HG2} may be a hydrogen atom, and the other may be a monovalent group containing a hydrophilic group. Examples of the monovalent group containing a hydrophilic group include an alkyl group containing a hydrophilic group, a carboxyl group containing a hydrophilic group, an alkylcarbonyl group containing a hydrophilic group, an alkyloxycarbonyl group containing a hydrophilic group, and an oxycarbonyl group containing a hydrophilic group.

**In** the formula (A), at least one hydrophilic group is contained in one or more moieties selected from the group consisting of L_{HG}, R_{HG1}, and R_{HG2}. Examples of the moiety containing at least one hydrophilic group and combinations thereof include the following:
(i) L_{HG} alone;
(ii) R_{HG1} alone;
(iii) R_{HG2} alone;
(iv) a combination of L_{HG} and L_{HG1};
(v) a combination of L_{HG} and L_{HG2};
(vi) a combination of L_{HG1} and L_{HG2}; and
(vii) a combination of L_{HG}, L_{HG1}, and L_{HG2}. Each of these L_{HG}, R_{HG1}, and R_{HG2} moieties may contain one hydrophilic group, or two or more hydrophilic groups.

### Divalent groups optionally containing a hydrophilic group

The divalent group optionally containing a hydrophilic group is a divalent group optionally containing a hydrophilic group as described above. The divalent group is as described above.

Preferably, the divalent group optionally containing a hydrophilic group may be a divalent group represented by the following formula (a):

-(C(R_{HG})₂)ₙ₁-(C=O)ₙ₂-(NR_{HG})ₙ₃-(C(R_{HG})₂)ₙ₄- (a)

wherein
the plurality of R_{HG} each independently represent a hydrogen atom, a hydrophilic group, or a monovalent group optionally containing a hydrophilic group,
n1 is an integer of 0 to 3,
n2 is an integer of 0 or 1,
n3 is an integer of 0 or 1, and
n4 is an integer of 0 to 3. The hyphens (-) placed at both ends in the formula (a) represent bonds.

In the formula (a), the plurality of R_{HG} each independently represent a hydrogen atom, a hydrophilic group, or a monovalent group optionally containing a hydrophilic group. The hydrophilic group and the monovalent group are as described above.
n1 is an integer from 0 to 3, preferably an integer from 0 to 2, and more preferably an integer of 0 or 1.
n2 is an integer of 0 or 1.
n3 is an integer of 0 or 1.
n4 is an integer from 0 to 3, preferably an integer from 0 to 2, and more preferably an integer of 0 or 1.

More preferably, the divalent group optionally containing a hydrophilic group may be a divalent group represented by the following formula (a1), (a2), or (a3):

(a1) -(C(R_{HG})₂)-;

(a2) -(C(R_{HG})₂)-(C=O)-(NR_{HG})-(C(R_{HG})₂)-; or

(a3) -(C=O)-(C(R_{HG})₂)₂-.

In the formulas (a1), (a2), or (a3), the plurality of R_{HG}s each independently represent a hydrogen atom, a hydrophilic group, or a C1-C6 alkyl group containing a hydrophilic group. The hydrophilic group and the C1-C6 alkyl group are as described above.

### Cleavable sites

A cleavable site is a site that can be cleaved in an appropriate environment (e.g., an intracellular or extracellular environment). Examples of cleavable sites include sites cleavable by an enzyme (e.g., U.S. Patent No. 6,214,345; Dubowchik et al., Pharm. Therapeutics 83:67-123

(1999); The FEBS Journal 287:1936-1969 (2020)), sites cleavable under acidic conditions (sites cleavable at local acidic locations present in vivo) (e.g., U.S. Patent Nos. 5,622,929, 5,122,368, 5,824,805; The FEBS Journal 287:1936-1969 (2020)), sites cleavable under reducing conditions (e.g., Protein & Cell 9, 33-46 (2018)), and sites cleavable by blood glutathione (e.g., The FEBS Journal 287:1936-1969 (2020)). The cleavable site may be self-immolative (e.g., WO02/083180, WO04/043493, WO05/112919).

The cleavable site may be a cleavable site containing a peptide. The cleavable site containing a peptide may be a cleavable site composed of a cleavable peptide that is cleaved by an enzyme. The cleavable site containing a peptide may also further contain a peptide not involved in cleavage, in addition to the cleavable peptide cleaved by an enzyme. The cleavable site containing a peptide, even if it further contains a peptide not involved in cleavage, can be cleaved as long as the cleavable site contains a cleavable peptide that is cleaved by an enzyme.

The amino acid residues constituting the peptide in the cleavable site may be any amino acid residues. Examples of such any amino acid residues include α-amino acid residues, β-amino acid residues, and γ-amino acid residues, with α-amino acid residues being preferable. Examples of α-amino acid residues include alanine, asparagine, cysteine, glutamine, glycine, isoleucine, leucine, methionine, phenylalanine, proline, serine, threonine, tryptophan, tyrosine, valine, aspartic acid, glutamic acid, arginine, histidine, lysine, and citrulline residues. Such any amino acid residues may also be L-amino acid residues or D-amino acid residues, with L-amino acid residues being preferable. Preferably, the any amino acid residue may be an L-α-amino acid residue (e.g., the L-form of the specific α-amino acid residue described above) or a glycine residue.

In a specific embodiment, the cleavable site containing a peptide may contain a cleavable peptide composed of amino acid residues selected from the group consisting of a valine residue, a phenylalanine residue, a threonine residue, a leucine residue, a citrulline residue, an alanine residue, a glutamic acid residue, a glutamine residue, a lysine residue, an arginine residue, and a methionine residue, and a combination thereof.

The number of amino acid residues constituting the peptide in the cleavable site may be 2 or more, 3 or more, 4 or more, 5 or more, or 6 or more. The number of amino acid residues constituting the peptide in the cleavable site may also be 20 or less, 15 or less, 12 or less, 10 or less, 8 or less, 6 or less, or 4 or less. More specifically, the number of amino acid residues constituting the peptide may be from 2 to 20, from 2 to 15, from 2 to 12, from 2 to 10, from 2 to 8, from 2 to 6, or from 2 to 4; may be from 3 to 20, from 3 to 15, from 3 to 12, from 3 to 10, from 3 to 8, from 3 to 6, or from 3 to 4; or may be from 4 to 20, from 4 to 15, from 4 to 12, from 4 to 10, from 4 to 8, or from 4 to 6.

In a specific embodiment, the number of amino acid residues constituting the peptide in the cleavable site may be 2. The number of amino acid residues constituting the peptide in the cleavable site may also be 3 or 4. Alternatively, the number of amino acid residues constituting the cleavable peptide may be 2. The number of amino acid residues constituting the cleavable peptide may also be 3 or 4.

In a preferred embodiment, the cleavable site may be a site cleavable by an enzyme. Examples of sites cleavable by an enzyme include sites (typically, peptide sites) cleavable by intracellular proteases (e.g., proteases present in lysosomes or endosomes) or extracellular proteases (e.g., secretory proteases). More preferably, the cleavable site is a site cleavable by an intracellular protease (e.g., a protease present in lysosomes or endosomes). Even more preferably, the cleavable site is a site cleavable by a protease present in lysosomes. Particularly preferably, the cleavable site is a site cleavable by cathepsin B, plasmin, legumain, and caspases.

More specifically, the site cleavable by cathepsin B, plasmin, legumain, and caspases may be a cleavable site comprising VC, VA, AA, GGFG (SEQ ID NO: 5), FK, VK, AK, GC, VLK, NN, or DDVD (SEQ ID NO: 6). The peptide sites of VC, VA, AA, GGFG (SEQ ID NO: 5), FK, VK, AK, and GC can be cleaved by cathepsin B. The VLK peptide site is known to be cleaved by interstitial plasmin. The NN peptide site is known to be cleaved by legumain. The DDVD peptide site is known to be cleaved by caspase. Examples of such cleavable sites include EVC, EEVC (SEQ ID NO: 7), EEEEVC (SEQ ID NO: 8), DVC, EVA, EAA, EGGFG (SEQ ID NO: 9), EFK, EEFK (SEQ ID NO: 10), EEVK (SEQ ID NO: 11), EEAK (SEQ ID NO: 12), EGC, EEVLK (SEQ ID NO: 13), ENN, EDDVD (SEQ ID NO: 14), β-Ala-VC, EGC, and EEGC (SEQ ID NO: 15).

In another preferred embodiment, the cleavable site may be a site cleavable under acidic or reducing conditions. Examples of sites cleavable under acidic conditions include alkyloxyarylalkyl residues, tertiary alkyloxycarbamate residues, acetal residues, silane residues, imine residues, vinyl ether residues, β-thiopropionate residues, trityl residues, hydrazone residues, aconityl residues, orthoester residues, carbamoyl residues, and 2-(diphenylphosphino)benzoate residues. Examples of sites cleavable under reducing conditions include disulfide residues, alkoxyalkyl residues, and azo residues.

In a specific embodiment, the divalent group containing a cleavable site, when adjacent to an optionally substituted divalent aromatic ring group (e.g., Ring A, Ring A1), may satisfy the following conditions (i) to (iii):
(i) the cleavable site is -CO-W- (where the hyphen (-) represents a bond, W is an oxygen atom, a sulfur atom, or an amino group (NH), and is bonded to the above-described divalent aromatic ring group);
(ii) the bond between CO and W is the site that undergoes cleavage; and
(iii) the above-described divalent aromatic ring group constitutes a pi electron conjugated system with W.

When a divalent group containing a site cleavable by cathepsin B satisfies the above conditions (i) to (iii), the cleavage of the bond between CO and W, in conjunction with the pi electron conjugated system and V in the divalent aromatic ring group in Ring A (wherein the divalent aromatic ring group constitutes the pi electron conjugated system with the cleavable site), can efficiently cleave the bond between the tertiary carbon atom connected to Ring A and V (FIG. 3). W is preferably an oxygen atom or an amino group (NH), and more preferably an amino group (NH). Therefore, the cleavable site is preferably an amide bond site.

In a specific embodiment, the divalent group containing a cleavable site, represented by CS, may be -L_{CSa}-CSₐ-W_{csa}- [wherein, the hyphen (-) represents a bond, L_{CSa} represents a bond or a divalent group, CSₐ represents a cleavable site, and W_{cs} represents an oxygen atom, a sulfur atom, or an amino group (NH).] The definition, examples, and preferred examples of the divalent group represented by L_{CSa} are the same as for the divalent group described above. The definition, examples, and preferred examples of the cleavable site represented by CSₐ are the same as for the cleavable site described above. W_{csa} represents an oxygen atom, a sulfur atom, or an amino group (NH), preferably represents an oxygen atom or an amino group (NH), and more preferably represents an amino group (NH).

In another specific embodiment, the divalent group containing a cleavable site, represented by CS, may be -L_{CSb}-CS_{b}-W_{csb}- [wherein, the hyphen (-) represents a bond, L_{CSb} represents a bond or a divalent group, CS_{b} represents a cleavable site, and W_{csb} represents a bond or a divalent group.] The definition, examples, and preferred examples of the divalent group represented by L_{CSb} and W_{csb} are the same as for the divalent group described above. The definition, examples, and preferred examples of the cleavable site represented by CS_{b} are the same as for the cleavable site described above. Preferably, in this embodiment, a disulfide bond is preferable as CS_{b}, and an alkylene (e.g., methylene) is preferable as the divalent group of W_{csa}.

### Bioorthogonal functional groups

Bioorthogonal functional groups refer to groups that do not react with biological components (e.g., amino acids, proteins, nucleic acids, lipids, carbohydrates, and phosphates), or react slowly with biological components but selectively with non-biological components. Bioorthogonal functional groups are well known in the art (see, e.g., Sharpless K. B. et al., Angew. Chem. Int. Ed. 40, 2004 (2015); Bertozzi C. R. et al., Science 291, 2357 (2001); Bertozzi C. R. et al., Nature Chemical Biology 1, 13 (2005)).

In the present invention, bioorthogonal functional groups for proteins are used as bioorthogonal functional groups. This is because antibodies to be derivatized with the reagent of the present invention are proteins. The bioorthogonal functional groups for proteins are groups that do not react with the side chains of the 20 naturally-occurring amino acid residues constituting proteins, or react slowly with the side chains but react with a target functional group. The 20 naturally-occurring amino acids constituting proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (K). Of these 20 naturally-occurring amino acids, glycine that has no side chain (i.e., the side chain is a hydrogen atom), and alanine, isoleucine, leucine, phenylalanine, and valine with a side chain that is a hydrocarbon group (i.e., the side chain does not contain any heteroatom selected from the group consisting of a sulfur atom, a nitrogen atom, and an oxygen atom) are inactive to common reactions. Thus, the bioorthogonal functional groups for proteins are groups that do not react not only with the side chains of these amino acids wherein the side chains are inactive to common reactions, but also with the side chains of asparagine, glutamine, methionine, proline, serine, threonine, tryptophan, tyrosine, aspartic acid, glutamic acid, arginine, histidine, and lysine, or react slowly with them but react with a target functional group.

Examples of such bioorthogonal functional groups include azide groups, aldehyde groups, thiol groups, alkene groups (in other words, the groups may have a vinylene (ethenylene) moiety, which is a minimum unit having a carbon-carbon double bond; the same applies hereinafter), alkyne groups (in other words, the groups may have an ethynylene moiety, which is a minimum unit having a carbon-carbon triple bond; the same applies hereinafter), halogen groups, tetrazine groups, nitrone groups, hydroxylamine groups, nitrile groups, hydrazine groups, ketone groups, boronic acid groups, cyanobenzothiazole groups, allyl groups, phosphine groups, maleimide groups, disulfide groups, thioester groups, α-halocarbonyl groups (e.g., carbonyl groups having a fluorine atom, a chlorine atom, a bromine atom, or an iodine atom at the alpha position. The same applies hereinafter), isonitrile groups, sydnone groups, and selenium groups.

More specifically, the bioorthogonal functional group may correspond to any one chemical structure selected from the group below. 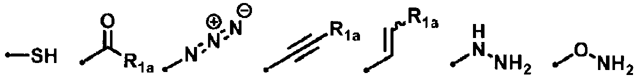 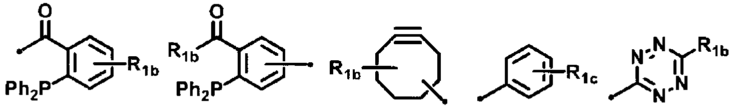 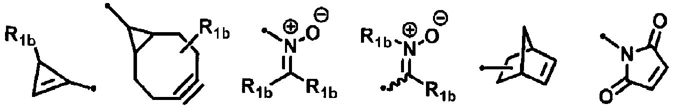 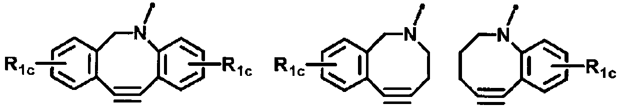 wherein
R₁ₐ, a single or a plurality of R_{1b}, and a single or a plurality of R_{1c} are the same or different, and are the above-mentioned substituents or electron-withdrawing groups, and
- is a bond.

Examples of the electron-withdrawing group include a halogen atom, an alkyl substituted with a halogen atom (e.g., trifluoromethyl), a boronic acid group, mesyl, tosyl, triflate, nitro, cyano, a phenyl group, and a keto group (e.g., acyl), with a halogen atom, a boronic acid group, mesyl, tosyl, and triflate being preferred.

In a specific embodiment, the bioorthogonal functional group may be protected. An optionally protected bioorthogonal functional group refers to an unprotected bioorthogonal functional group or a protected bioorthogonal functional group. The unprotected bioorthogonal functional group corresponds to the bioorthogonal functional group as described above. The protected bioorthogonal functional group is a group that generates a bioorthogonal functional group by cleavage of the protecting group. The cleavage of the protecting group can be performed by a specific treatment under conditions that cannot cause protein denaturation or degradation (e.g., cleavage of an amide bond) (mild conditions). Examples of such a specific treatment include (a) treatment with one or more substances selected from the group consisting of an acidic substance, a basic substance, a reducing agent, an oxidizing agent, and an enzyme, (b) treatment with physicochemical stimulation selected from the group consisting of light, or (c) incubation using a cleavable linker comprising a self-degradable cleavable site. Such protecting groups and the conditions for cleaving them are common technical knowledge in the art (e.g., G. Leriche, L. Chisholm, A. Wagner, Bioorganic & Medicinal Chemistry, 20,571 (2012); Feng P. et al., Jounal of American Chemical Society, 132,1500 (2010).; Bessodes M. et al., Journal of Controlled Release, 99,423 (2004).; DeSimone, J. M., Journal of American Chemical Society. 132,17928

(2010); Thompson, D. H., Journal of Controlled Release, 91,187 (2003); Schoenmarks, R. G., Journal of Controlled Release, 95,291 (2004)).

Examples of the protected bioorthogonal functional group include disulfide groups, ester groups, acetal groups, ketal groups, imine groups, and vicinaldiol groups.

More specifically, the protected bioorthogonal functional group may correspond to any one chemical structure selected from the group consisting of the following. 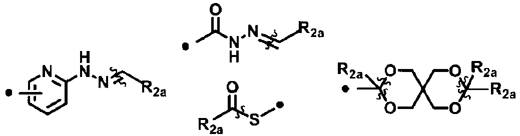 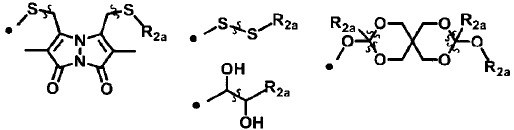 wherein
the wavy line orthogonal to the bond represents a cleavage site, and
the single or plurality of R₂ₐ is/are the same or different, and is/are selected from the group consisting of a hydrogen atom or the above-mentioned substituents, and
   - is a bond.

Preferably, the optionally protected bioorthogonal functional group is an unprotected bioorthogonal functional group.

### Functional substance

The functional substance is not particularly limited as long as it is a substance that imparts an arbitrary function to the antibody, and examples thereof include drugs, labeling substances, and stabilizers, with drugs or labeling substances being preferable. The functional substance may be a single functional substance or a substance in which two or more functional substances are linked with each other.

The drug may be a drug for any disease. Examples of such a disease include cancer (e.g., lung cancer, stomach cancer, colon cancer, pancreatic cancer, renal cancer, liver cancer, thyroid cancer, prostate cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), neurological diseases (e.g., cerebral infarction, Alzheimer's disease, Parkinson disease, and amyotrophic lateral sclerosis), infectious diseases (e.g., bacterial infections and viral infections), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukemia and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes, and substances comprising them. Examples of chemotherapeutic agents include DNA damaging agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorus atom (e.g., ³²P), radioisotopes of a sulfur atom (e.g., 35^{S}), radioisotopes of yttrium (e.g., ⁹⁰Y), radioisotopes of technetium (e.g., ^{99m}Tc), radioisotopes of indium (e.g., ¹¹¹In), radioisotopes of an iodine atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracyclines, dmDNA31, tubulysin, and exatecan.

The labelling substance is a substance that makes detection of a target (e.g., a tissue, a cell, or a substance) possible. Examples of the labelling substance include enzymes (e.g., peroxidase, alkaline phosphatase, luciferase, and β-galactosidase), affinity substances (e.g., streptavidin, biotin, digoxigenin, and aptamers), fluorescent substances (e.g., fluorescein, fluorescein isothiocyanate, rhodamine, green-fluorescent proteins, and red-fluorescent proteins), luminescent substances (e.g., luciferin, aequorin, acridinium esters, tris(2,2'-bipyridyl) ruthenium, and luminol), and radioisotopes (e.g., those described above) or substances comprising them.

The stabilizer is a substance that makes stabilization of an antibody possible. Examples of the stabilizer include diols, glycerol, nonionic surfactants, anionic surfactants, natural surfactants, saccharides, and polyols.

The functional substance may also be a peptide, a protein, a nucleic acid, a low molecular weight organic compound, a sugar chain, a lipid, a high molecular weight polymer, a metal (e.g., gold), or a chelator. Examples of the peptide include cell membrane permeable peptides, blood-brain barrier permeable peptides, and peptide medicaments. Examples of the protein include enzymes, cytokines, fragment antibodies, lectins, interferons, serum albumin, and antibodies. Examples of the nucleic acid include DNAs, RNAs, and artificial nucleic acids. Examples of the nucleic acid also include RNA interference inducible nucleic acids (e.g., siRNAs), aptamers, and antisense nucleic acids. Examples of the low molecular weight organic compound include proteolysis targeting chimeras, dyes, and photodegradable compounds.

### Salts

In the present invention, examples of the term "salt" include salts with inorganic acids, salts with organic acids, salts with inorganic bases, salts with organic bases, and salts with amino acids. Examples of salts with inorganic acids include salts with hydrochloride, hydrobromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrochloride) or a salt with an organic acid (e.g., trifluoroacetic acid).

### 2. Conjugates or salts thereof

2-1. A conjugate of an antibody and a functional substance, or a salt thereof, comprising an immunoglobulin unit comprising a cysteine residue-modifying moiety represented by formula (I-1)
The present invention provides a conjugate of an antibody and a functional substance, or a salt thereof, wherein
(1) the conjugate comprises an immunoglobulin unit comprising two heavy chains and two light chains, and
(2) said immunoglobulin unit comprises
   (a) two or more cysteine residue-modifying moieties connected to the immunoglobulin unit by bonding to the sulfur atoms in the side chains of two or more cysteine residues in said two heavy chains and two light chains, and
   (b) two or more lysine residue-modifying moieties regioselectively connected to the immunoglobulin unit by bonding to the nitrogen atoms in the side chains of two or more lysine residues in said two heavy chains, and
(3) said cysteine residue-modifying moieties are represented by the following formula (I-1): 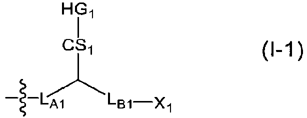 wherein
   the hyphen (-) with a wavy line represents a bond to said sulfur atom,
   HG₁ represents a hydrophilic group or a monovalent group containing a hydrophilic group,
   CS₁ represents a divalent group containing a cleavable site,
   L_{A1} and L_{B1} each independently represent a divalent group, and
   X₁ represents a functional substance.

In formula (I-1) and other formulas presented in relation to the present invention, a hyphen (-) indicates that two units present on both sides thereof are covalently bonded to each other. When a unit is not present on one side of a hyphen (-), that hyphen (-) represents a bond.

In relation to (1) above, the antibody comprises an immunoglobulin (Ig) unit as described above. Examples of such an antibody include IgG antibodies, IgD antibodies, and IgE antibodies, which contain one immunoglobulin unit comprising two heavy chains and two light chains and having disulfide bonds between the heavy chains and between the heavy and light chains; IgA antibodies containing two such immunoglobulin units; and IgM antibodies containing four such immunoglobulin units, with IgG antibodies (e.g., IgG1, IgG2, IgG3, IgG4) being preferred. The antibody is preferably a human IgG monoclonal antibody, and more preferably a human IgG full-length monoclonal antibody.

In relation to (2) (a) above, the two or more cysteine residue-modifying moieties may be connected to the immunoglobulin unit by bonding to the sulfur atoms in the side chains of two or more cysteine residues in the two heavy chains and two light chains. This connection can be achieved by a bond between the thiol group in the side chain of the cysteine residue and an atom or group capable of bonding thereto (e.g., a maleimide residue, a carbonyl group, or a thiol group). The number of the cysteine residue-modifying moieties is 2 or more, preferably 2 to 8, more preferably 4 to 8, still more preferably 6 to 8, and particularly preferably 8. An Ig unit usually comprises eight cysteine residues that form four disulfide bonds. Therefore, by reacting an antibody containing an Ig unit with a reducing agent, eight thiol groups can be generated from the four disulfide bonds in the Ig unit, and cysteine residue-modifying moieties can be appropriately reacted with the generated thiol groups. The number of cysteine residue-modifying moieties introduced into the Ig unit can be appropriately adjusted by controlling the type of Ig unit (e.g., an amino acid residue-modified antibody), the type and amount of the reducing agent, and the reaction time.

In relation to (2) (b) above, the two or more lysine residue-modifying moieties are regioselectively connected to the immunoglobulin unit by bonding to the nitrogen atoms in the side chains of two or more lysine residues in the two heavy chains. This connection can be achieved by a bond between the amino group in the side chain of the lysine residue and an atom or group capable of bonding thereto (e.g., a carbonyl group). The number of the lysine residue-modifying moieties is 2 or more, preferably 2 to 6, and more preferably 2 or 4.

The two or more lysine residues may be any of the lysine residues present at the following positions according to Eu numbering:
(A) a lysine residue present at position 246/248 in the two heavy chains;
(B) a lysine residue present at position 288/290 in the two heavy chains;
(C) a lysine residue present at position 317 in the two heavy chains; and
(D) a combination of (A) and (B), (A) and (C), or (B) and (C), or (A), (B), and (C).

Among the above, the following are preferred as lysine residues:
(A') a lysine residue present at position 246/248 in the two heavy chains;
(B') a lysine residue present at position 288/290 in the two heavy chains; and
(C') a combination of (A) and (B).

In relation to (3) above, in formula (I-1), HG₁ represents a hydrophilic group or a monovalent group containing a hydrophilic group. The hydrophilic group and the monovalent group are as described above. Preferably, HG₁ may represent a monovalent group containing a hydrophilic group.

CS₁ represents a divalent group containing a cleavable site. The cleavable site and the divalent group are as described above.

The divalent group represented by L_{A1} is a divalent group having a bond to the sulfur atom in the side chain of a cysteine residue. The divalent group represented by L_{B1} is a divalent group that links the carbon atom adjacent to CS₁ and X₁. The divalent group is as described above.

The functional substance represented by X₁ is as described above.

In a specific embodiment, L_{A1} may be a divalent group having a side chain containing a hydrophilic group or a functional substance. The side chain containing a hydrophilic group or a functional substance may be a side chain containing a hydrophilic group and a functional substance. The side chain containing a hydrophilic group and a functional substance may be a side chain containing (i) a hydrophilic group, (ii) a functional substance, and (iii) a cleavable site between the hydrophilic group and the functional substance. The number of such side chains contained in the divalent group is not particularly limited, and may be, for example, 1 to 4, 1 to 3, 1 or 2, or 1.

In a specific embodiment, the side chain comprising (i) a hydrophilic group, (ii) a functional substance, and (iii) a cleavable site between the hydrophilic group and the functional substance may be represented by the following formula (α): 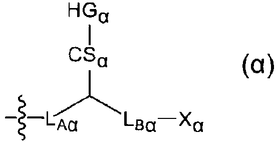 wherein
the hyphen (-) with a wavy line represents a bond to the main chain in L_{A},
HG_{α} represents a hydrophilic group or a monovalent group containing a hydrophilic group,
CS_{α} represents a divalent group containing a cleavable site,
L_{Aα} and L_{Bα} each independently represent a divalent group, and
X_{α} represents a functional substance.

HG_{α} represents a hydrophilic group or a monovalent group containing a hydrophilic group. The hydrophilic group and the monovalent group are as described above. The hydrophilic group or the monovalent group containing a hydrophilic group represented by HG_{α} may be the same as or different from the hydrophilic group or the monovalent group containing a hydrophilic group represented by HG₁.

CS_{α} represents a divalent group containing a cleavable site. The cleavable site and the divalent group are as described above. The divalent group containing a cleavable site represented by CS_{α} may be the same as or different from the divalent group containing a cleavable site represented by CS₁.

The divalent group represented by L_{Aα} is a divalent group having a bond to the main chain in L_{A1}. The divalent group represented by L_{Aα} may be the same as or different from the divalent group represented by L_{A1}. The divalent group represented by L_{Bα} is a divalent group capable of linking D_{α} and V_{α}. The divalent group represented by L_{Bα} may be the same as or different from the divalent group represented by L_{B1}.

X_{α} represents a functional substance. The functional substance is as described above. The functional substance represented by X_{α} may be the same as or different from the functional substance represented by X₁.

In a specific embodiment, the two or more (e.g., two) lysine residue-modifying moieties may be represented by the following formula (I-2): 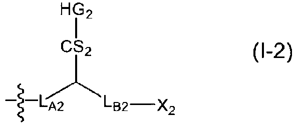 wherein
the hyphen (-) with a wavy line represents a bond to said nitrogen atom,
HG₂ represents a hydrophilic group or a monovalent group containing a hydrophilic group,
CS₂ represents a divalent group containing a cleavable site,
L_{A2} and L_{B2} each independently represent a divalent group, and
X₂ represents a functional substance,
or the following formula (II): 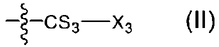 wherein
the hyphen (-) with a wavy line represents a bond to said nitrogen atom,
CS₃ represents a divalent group optionally containing a cleavable site, and
X₃ represents a functional substance. When the number of the lysine residue-modifying moieties is two, the two lysine residue-modifying moieties may be the same as or different from each other. When the number of the lysine residue-modifying moieties is four, the four lysine residue-modifying moieties may be the same as or different from each other.

HG₂ represents a hydrophilic group or a monovalent group containing a hydrophilic group. The hydrophilic group and the monovalent group are as described above. The hydrophilic group or the monovalent group containing a hydrophilic group represented by HG₂ may be the same as or different from the hydrophilic group or the monovalent group containing a hydrophilic group represented by HG₁.

CS₂ represents a divalent group containing a cleavable site. The cleavable site and the divalent group are as described above. The divalent group containing a cleavable site represented by CS₂ may be the same as or different from the divalent group containing a cleavable site represented by CS₁.

The divalent group represented by L_{A2} is a divalent group having a bond to the nitrogen atom in the side chain of a lysine residue. The divalent group represented by L_{B2} is a divalent group that links the carbon atom adjacent to CS₂ and X₂ The divalent group is as described above. The divalent group represented by L_{A2} may be the same as or different from the divalent group represented by L_{A1}. The divalent group represented by L_{B2} may be the same as or different from the divalent group represented by L_{B1}.

X₂ represents a functional substance. The functional substance is as described above. The functional substance represented by X₂ may be the same as or different from the functional substance represented by X₁.

CS₃ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃ may be the same as or different from the divalent group with a cleavable site represented by CS₁.

X₃ represents a functional substance. The functional substance is as described above. The functional substance represented by X₃ may be the same as or different from the functional substance represented by X₁.

In a specific embodiment, the two or more (e.g., four) lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). When the number of the lysine residue-modifying moieties is four, two first lysine residue-modifying moieties are the same, and two second lysine residue-modifying moieties are the same. The first lysine residue-modifying moieties and the second lysine residue-modifying moieties are the same as or different from each other.

The first lysine residue-modifying moiety can be represented by the following formula (I-2-1): 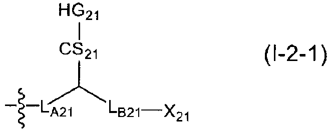 wherein
the hyphen (-) with a wavy line represents a bond to said nitrogen atom,
HG₂₁ represents a hydrophilic group or a monovalent group containing a hydrophilic group,
CS₂₁ represents a divalent group containing a cleavable site,
L_{A21} and L_{B21} each independently represent a divalent group, and
X₂₁ represents a functional substance,
or the following formula (II-1): 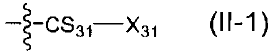 wherein
the hyphen (-) with a wavy line represents a bond to said nitrogen atom,
CS₃₁ represents a divalent group optionally containing a cleavable site, and
X₃₁ represents a functional substance.

HG₂₁ represents a hydrophilic group or a monovalent group containing a hydrophilic group. The hydrophilic group and the monovalent group are as described above. The hydrophilic group or the monovalent group containing a hydrophilic group represented by HG₂₁ may be the same as or different from the hydrophilic group or the monovalent group containing a hydrophilic group represented by HG₁.

CS₂₁ represents a divalent group containing a cleavable site. The cleavable site and the divalent group are as described above. The divalent group containing a cleavable site represented by CS₂₁ may be the same as or different from the divalent group containing a cleavable site represented by CS₁.

The divalent group represented by L_{A21} is a divalent group having a bond to the nitrogen atom in the side chain of a lysine residue. The divalent group represented by L_{B21} is a divalent group that links the carbon atom adjacent to CS₂₁ and X₂₁. The divalent group is as described above. The divalent group represented by L_{A21} may be the same as or different from the divalent group represented by L_{A1}. The divalent group represented by L_{B21} may be the same as or different from the divalent group represented by L_{B1}.

X₂₁ represents a functional substance. The functional substance is as described above. The functional substance represented by X₂₁ may be the same as or different from the functional substance represented by X₁.

CS₃₁ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃₁ may be the same as or different from the divalent group with a cleavable site represented by CS₁.

X₃₁ represents a functional substance. The functional substance is as described above. The functional substance represented by X₃ may be the same as or different from the functional substance represented by X₁.

The second lysine residue-modifying moiety can be represented by the following formula (I-2-2): 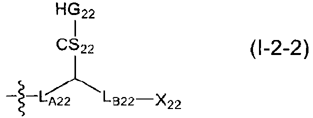 wherein
the hyphen (-) with a wavy line represents a bond to said nitrogen atom,
HG₂₂ represents a hydrophilic group or a monovalent group containing a hydrophilic group,
CS₂₂ represents a divalent group containing a cleavable site,
L_{A22} and L_{B22} each independently represent a divalent group, and
X₂₂ represents a functional substance,
or the following formula (II-2): 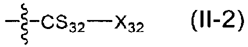 wherein
the hyphen (-) with a wavy line represents a bond to said nitrogen atom,
CS₃₂ represents a divalent group optionally containing a cleavable site, and
X₃₂ represents a functional substance.

HG₂₂ represents a hydrophilic group or a monovalent group containing a hydrophilic group. The hydrophilic group and the monovalent group are as described above. The hydrophilic group or the monovalent group containing a hydrophilic group represented by HG₂₂ may be the same as or different from the hydrophilic group or the monovalent group containing a hydrophilic group represented by HG₁ and/or HG₂₁.

CS₂₂ represents a divalent group containing a cleavable site. The cleavable site and the divalent group are as described above. The divalent group containing a cleavable site represented by CS₂₂ may be the same as or different from the divalent group containing a cleavable site represented by CS₁ and/or CS₂₁.

The divalent group represented by L_{A22} is a divalent group having a bond to the nitrogen atom in the side chain of a lysine residue. The divalent group represented by L_{B22} is a divalent group that links the carbon atom adjacent to CS₂₂ and X₂₂. The divalent group is as described above. The divalent group represented by L_{A22} may be the same as or different from a divalent group represented by L_{A1} and/or L_{A21}. The divalent group represented by L_{B22} may be the same as or different from the divalent group represented by L_{B1} and/or L_{B21}.

X₂₂ represents a functional substance. The functional substance is as described above. The functional substance represented by X₂₂ may be the same as or different from the functional substance represented by X₁ and/or X₂₁.

CS₃₂ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃₂ may be the same as or different from a divalent group with a cleavable site represented by CS₁ and/or a divalent group optionally containing a cleavable site represented by CS₃₁.

X₃₂ represents a functional substance. The functional substance is as described above. The functional substance represented by X₃₂ may be the same as or different from the functional substance represented by X₁ and/or X₃₁.

2-2. A conjugate of an antibody and a functional substance, or a salt thereof, comprising an immunoglobulin unit comprising a cysteine residue-modifying moiety represented by formula (I-1')
The present invention also provides a conjugate of an antibody and a functional substance, or a salt thereof, wherein
(1) the conjugate comprises an immunoglobulin unit comprising two heavy chains and two light chains, and
(2) said immunoglobulin unit comprises:
   (a) two or more cysteine residue-modifying moieties connected to the immunoglobulin unit by bonding to the sulfur atoms in the side chains of two or more cysteine residues in said two heavy chains and two light chains, and
   (b) two or more lysine residue-modifying moieties regioselectively connected to the immunoglobulin unit by bonding to the nitrogen atoms in the side chains of two or more lysine residues in said two heavy chains, and
(3) said cysteine residue-modifying moiety is represented by the following formula (I-1'): 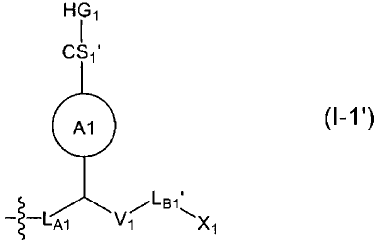 wherein
   the hyphen (-) with a wavy line represents a bond to said sulfur atom,
   HG₁ represents a hydrophilic group or a monovalent group containing a hydrophilic group,
   CS₁' represents a divalent group containing a cleavable site,
   Ring A1 represents an optionally substituted divalent aromatic ring group (wherein the divalent aromatic ring group forms a pi electron conjugated system with said cleavable site),
   V₁ represents an oxygen atom, a sulfur atom, or an amino group (NH),
   L_{A1} and L_{B1}' each independently represent a divalent group, and
   X₁ represents a functional substance.

The characteristics of (1), (2) (a), and (2) (b) above in the conjugate of an antibody and a functional substance, or a salt thereof, comprising an immunoglobulin unit comprising a cysteine residue-modifying moiety represented by formula (I-1'), are the same as those in the conjugate of an antibody and a functional substance, or a salt thereof, comprising an immunoglobulin unit comprising a cysteine residue-modifying moiety represented by formula (I-1).

HG₁, L_{A1}, and X₁ in formula (I-1') each are the same as those represented by formula (I-1).

CS₁' represents a divalent group containing a cleavable site. The cleavable site and the divalent group are as described above.

Ring A1 represents an optionally substituted divalent aromatic ring group (wherein the divalent aromatic ring group forms a pi electron conjugated system with said cleavable site). The divalent aromatic ring group is the arylene or divalent aromatic heterocyclic group described above. The position of the divalent aromatic ring group to which the two adjacent atoms (a carbon atom and an adjacent atom in CS') are bonded is not particularly limited as long as cleavage occurs between V and its adjacent carbon atom due to the conjugation of pi electrons when the cleavable site is cleaved (see FIG. 3). Such a position is common general technical knowledge in the art, and can be easily determined by a person skilled in the art according to factors such as the types of cleavable site and divalent aromatic ring group.

Preferably, Ring A1 may be an optionally substituted divalent monocyclic aromatic ring group. The divalent aromatic ring group is a phenylene group or a divalent monocyclic aromatic heterocyclic group.

More preferably, Ring A1 may be a divalent 6-membered cyclic aromatic ring group. Examples of the 6-membered cyclic aromatic ring group include the various groups described above. In this case, the position of the divalent 6-membered cyclic aromatic ring group to which the two adjacent atoms are bonded is the ortho position or the para position, preferably the para position.

Still more preferably, Ring A1 may be an optionally substituted phenylene group. In this case, the position of the phenylene group to which the two adjacent atoms are bonded is the ortho position or the para position, preferably the para position.

The substituent in the optionally substituted divalent aromatic ring group is as described above. Such a substituent may be an electron-withdrawing group as described above.

V₁ represents an oxygen atom, a sulfur atom, or an amino group (NH). V₁ is preferably an oxygen atom or a sulfur atom, and more preferably an oxygen atom.

L_{B1}' represents a divalent group capable of linking V₁ and X₁. The divalent group is as described above.

In a specific embodiment, L_{A1} may be a divalent group having a side chain containing a hydrophilic group or a functional substance. The side chain containing a hydrophilic group or a functional substance may be a side chain containing a hydrophilic group and a functional substance. The side chain containing a hydrophilic group and a functional substance may be a side chain containing (i) a hydrophilic group, (ii) a functional substance, and (iii) a cleavable site between the hydrophilic group and the functional substance. The number of such side chains contained in the divalent group is not particularly limited, and may be, for example, 1 to 4, 1 to 3, 1 or 2, or 1.

In a specific embodiment, the side chain comprising (i) a hydrophilic group, (ii) a functional substance, and (iii) a cleavable site between the hydrophilic group and the functional substance may be represented by the following formula (α'): 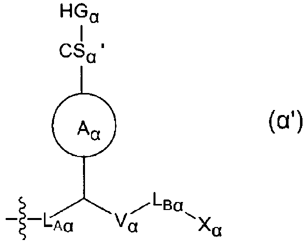 wherein
the hyphen (-) with a wavy line represents a bond to the main chain in L_{A1},
HG_{α} represents a hydrophilic group or a monovalent group containing a hydrophilic group,
CS_{α}' represents a divalent group containing a cleavable site,
Ring Aα represents an optionally substituted divalent aromatic ring group (wherein the divalent aromatic ring group forms a pi electron conjugated system with said cleavable site),
V_{α} represents an oxygen atom, a sulfur atom, or an amino group (NH),
L_{Aα} and L_{Bα} each independently represent a divalent group, and
X_{α} represents a functional substance.

In formula (α'), HG_{α}, L_{Aα}, L_{Bα}, and X_{α} are each the same as those represented by formula (α).

CS_{α}' represents a divalent group containing a cleavable site. The cleavable site and the divalent group are as described above. The divalent group containing a cleavable site represented by CS_{α}' may be the same as or different from the divalent group containing a cleavable site represented by CS'.

Ring A_{α} represents an optionally substituted divalent aromatic ring group (wherein the divalent aromatic ring group forms a pi electron conjugated system with the cleavable site). The optionally substituted divalent aromatic ring group is as described above. The optionally substituted divalent aromatic ring group represented by Ring A_{α} may be the same as or different from the optionally substituted divalent aromatic ring group represented by Ring A.

V_{α} represents an oxygen atom, a sulfur atom, or an amino group (NH). The type of V_{α} may be the same as or different from the type of V. Therefore, when V is an oxygen atom, V_{α} may be a sulfur atom or an amino group (NH). V_{α} is preferably an oxygen atom or a sulfur atom, and more preferably an oxygen atom.

In a specific embodiment, the two or more (e.g., two) lysine residue-modifying moieties may be represented by the following formula (I-2'): 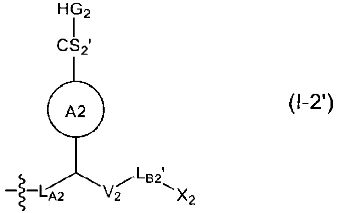 wherein
the hyphen (-) with a wavy line represents a bond to said nitrogen atom,
HG₂ represents a hydrophilic group or a monovalent group containing a hydrophilic group,
CS₂' represents a divalent group containing a cleavable site,
Ring A2 represents an optionally substituted divalent aromatic ring group (wherein the divalent aromatic ring group forms a pi electron conjugated system with said cleavable site),
V₂ represents an oxygen atom, a sulfur atom, or an amino group (NH),
L_{A2} and L_{B2}' each independently represent a divalent group, and
X₂ represents a functional substance,
or the following formula (II): 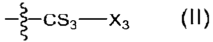 wherein
the hyphen (-) with a wavy line represents a bond to said nitrogen atom,
CS₃ represents a divalent group optionally containing a cleavable site, and
X₃ represents a functional substance. When the number of the lysine residue-modifying moieties is two, the two lysine residue-modifying moieties may be the same as or different from each other. When the number of the lysine residue-modifying moieties is four, the four lysine residue-modifying moieties may be the same as or different from each other.

HG₂, L_{A2}, and X₂ in formula (I-2') each are the same as those represented by formula (I-2).

CS₂' represents a divalent group containing a cleavable site. The cleavable site and the divalent group are as described above. The divalent group containing a cleavable site represented by CS₂' may be the same as or different from the divalent group containing a cleavable site represented by CS₁'.

Ring A2 represents an optionally substituted divalent aromatic ring group (wherein the divalent aromatic ring group forms a pi electron conjugated system with said cleavable site). The optionally substituted divalent aromatic ring group is as described above. The optionally substituted divalent aromatic ring group represented by Ring A2 may be the same as or different from the optionally substituted divalent aromatic ring group represented by Ring A1.

V₂ represents an oxygen atom, a sulfur atom, or an amino group (NH). V₂ is preferably an oxygen atom or a sulfur atom, and more preferably an oxygen atom.

L_{B2}' represents a divalent group capable of linking V₂ and X₂. The divalent group is as described above. The divalent group with a cleavable site represented by L_{B2}' may be the same as or different from the divalent group with a cleavable site represented by L_{B1}' .

CS₃ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃ may be the same as or different from the divalent group with a cleavable site represented by CS₁'.

X₃ represents a functional substance. The functional substance is as described above. The functional substance represented by X₃ may be the same as or different from the functional substance represented by X₁.

In a specific embodiment, the two or more (e.g., four) lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). When the number of the lysine residue-modifying moieties is four, two first lysine residue-modifying moieties are the same, and two second lysine residue-modifying moieties are the same. The first lysine residue-modifying moieties and the second lysine residue-modifying moieties are the same as or different from each other.

The first lysine residue-modifying moiety can be represented by the following formula (I-2-1'): 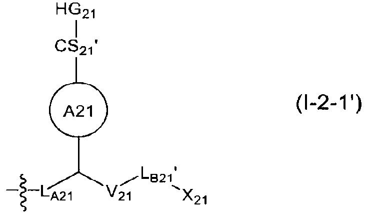 wherein
the hyphen (-) with a wavy line represents a bond to said nitrogen atom,
HG₂₁ represents a hydrophilic group or a monovalent group containing a hydrophilic group,
CS₂₁' represents a divalent group containing a cleavable site,
Ring A21 represents an optionally substituted divalent aromatic ring group (wherein the divalent aromatic ring group forms a pi electron conjugated system with said cleavable site),
V₂₁ represents an oxygen atom, a sulfur atom, or an amino group (NH),
L_{A21} and L_{B21}' each independently represent a divalent group,
X₂₁ represents a functional substance,
or the following formula (II-1): 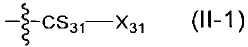 wherein
the hyphen (-) with a wavy line represents a bond to said nitrogen atom,
CS₃₁ represents a divalent group optionally containing a cleavable site, and
X₃₁ represents a functional substance.

HG₂₁ represents a hydrophilic group or a monovalent group containing a hydrophilic group. The hydrophilic group and the monovalent group are as described above. The hydrophilic group or the monovalent group containing a hydrophilic group represented by HG₂₁ may be the same as or different from the hydrophilic group or the monovalent group containing a hydrophilic group represented by HG₁.

CS₂₁' represents a divalent group containing a cleavable site. The cleavable site and the divalent group are as described above. The divalent group containing a cleavable site represented by CS₂₁' may be the same as or different from the divalent group containing a cleavable site represented by CS₁.

Ring A21 represents an optionally substituted divalent aromatic ring group (wherein the divalent aromatic ring group forms a pi electron conjugated system with said cleavable site). The optionally substituted divalent aromatic ring group is as described above. The optionally substituted divalent aromatic ring group represented by Ring A21 may be the same as or different from the optionally substituted divalent aromatic ring group represented by Ring A1.

The divalent group represented by L_{A21} is a divalent group having a bond to the nitrogen atom in the side chain of a lysine residue. The divalent group represented by L_{B21}' is a divalent group that links V₂₁ and X₂₁. The divalent group is as described above. The divalent group represented by L_{A21} may be the same as or different from the divalent group represented by L_{A1}. The divalent group represented by L_{B21}' may be the same as or different from the divalent group represented by L_{B1}'.

V₂₁ represents an oxygen atom, a sulfur atom, or an amino group (NH). V₂₁ is preferably an oxygen atom or a sulfur atom, and more preferably an oxygen atom.

X₂₁ represents a functional substance. The functional substance is as described above. The functional substance represented by X₂₁ may be the same as or different from the functional substance represented by X₁.

CS₃₁ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃₁ may be the same as or different from the divalent group with a cleavable site represented by CS₁.

X₃₁ represents a functional substance. The functional substance is as described above. The functional substance represented by X₃ may be the same as or different from the functional substance represented by X₁.

The second lysine residue-modifying moiety can be represented by the following formula (I-2-2'): 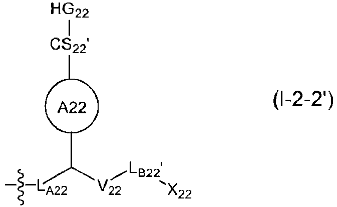 wherein
the hyphen (-) with a wavy line represents a bond to said nitrogen atom,
HG₂₂ represents a hydrophilic group or a monovalent group containing a hydrophilic group,
CS₂₂' represents a divalent group containing a cleavable site,
Ring A22 represents an optionally substituted divalent aromatic ring group,
V₂₂ represents an oxygen atom, a sulfur atom, or an amino group (NH),
L_{A22} and L_{B22}' each independently represent a divalent group, and
X₂₂ represents a functional substance,
or the following formula (II-2): 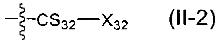 wherein
the hyphen (-) with a wavy line represents a bond to said nitrogen atom,
CS₃₂ represents a divalent group optionally containing a cleavable site, and
X₃₂ represents a functional substance.

HG₂₂ represents a hydrophilic group or a monovalent group containing a hydrophilic group. The hydrophilic group and the monovalent group are as described above. The hydrophilic group or the monovalent group containing a hydrophilic group represented by HG₂₂ may be the same as or different from the hydrophilic group or the monovalent group containing a hydrophilic group represented by HG₁ and/or HG₂₁.

CS₂₂' represents a divalent group containing a cleavable site. The cleavable site and the divalent group are as described above. The divalent group containing a cleavable site represented by CS₂₂' may be the same as or different from the divalent group containing a cleavable site represented by CS₁ and/or CS₂₁'.

Ring A22 represents an optionally substituted divalent aromatic ring group (wherein the divalent aromatic ring group forms a pi electron conjugated system with said cleavable site). The optionally substituted divalent aromatic ring group is as described above. The optionally substituted divalent aromatic ring group represented by Ring A22 may be the same as or different from the optionally substituted divalent aromatic ring group represented by Ring A1 and/or Ring A21.

The divalent group represented by L_{A22} is a divalent group having a bond to the nitrogen atom in the side chain of a lysine residue. The divalent group represented by L_{B22}' is a divalent group that links V₂₂ and X₂₂. The divalent group is as described above. The divalent group represented by L_{A22} may be the same as or different from the divalent group represented by L_{A1} and/or L_{A21}. The divalent group represented by L_{B22}' may be the same as or different from the divalent group represented by L_{B1} and/or L_{B21}.

V₂₂ represents an oxygen atom, a sulfur atom, or an amino group (NH). V₂₂ is preferably an oxygen atom or a sulfur atom, and more preferably an oxygen atom.

X₂₂ represents a functional substance. The functional substance is as described above. The functional substance represented by X₂₂ may be the same as or different from the functional substance represented by X₁ and/or X₂₁.

CS₃₂ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃₂ may be the same as or different from a divalent group with a cleavable site represented by CS₁ and/or a divalent group optionally containing a cleavable site represented by CS₃₁.

X₃₂ represents a functional substance. The functional substance is as described above. The functional substance represented by X₃₂ may be the same as or different from the functional substance represented by X₁ and/or X₃₁.

2-3. A conjugate of an antibody and a functional substance, or a salt thereof, comprising an immunoglobulin unit comprising a cysteine residue-modifying moiety represented by formula (I-1'')
The present invention also provides a conjugate of an antibody and a functional substance, or a salt thereof, wherein
(1) the conjugate comprises an immunoglobulin unit comprising two heavy chains and two light chains, and
(2) said immunoglobulin unit comprises:
   (a) two or more cysteine residue-modifying moieties connected to the immunoglobulin unit by bonding to the sulfur atoms in the side chains of two or more cysteine residues in said two heavy chains and two light chains, and
   (b) two or more lysine residue-modifying moieties regioselectively connected to the immunoglobulin unit by bonding to the nitrogen atoms in the side chains of two or more lysine residues in said two heavy chains, and
(3) said cysteine residue-modifying moiety is represented by the following formula (I-1 "): 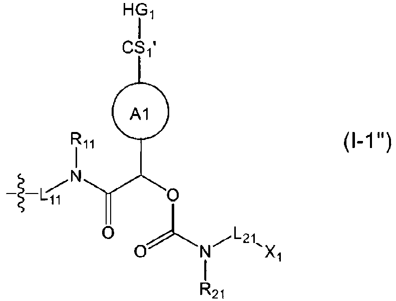 wherein
   the hyphen (-) with a wavy line represents a bond to said sulfur atom,
   HG₁ represents a hydrophilic group or a monovalent group containing a hydrophilic group,
   CS₁' represents a divalent group containing a cleavable site,
   Ring A1 represents an optionally substituted divalent aromatic ring group (wherein the divalent aromatic ring group forms a pi electron conjugated system with said cleavable site),
   R₁₁ and R₂₁ each independently represent a hydrogen atom or a monovalent group,
   L₁₁ and L₂₁ each independently represent a divalent group, and
   X₁ represents a functional substance.

The characteristics of (1), (2) (a), and (2) (b) above in the conjugate of an antibody and a functional substance, or a salt thereof, comprising an immunoglobulin unit comprising a cysteine residue-modifying moiety represented by formula (I-1''), are the same as those in the conjugate of an antibody and a functional substance, or a salt thereof, comprising an immunoglobulin unit comprising a cysteine residue-modifying moiety represented by formula (I-1).

HG₁ and X₁ in formula (I-1") each are the same as those represented by formula (I-1). CS₁' and Ring A1 in formula (1-1") are each the same as those represented by formula (I-1').

R₁₁ and R₂₁ each independently represent a hydrogen atom or a monovalent group. The monovalent group is as described above.

L₁₁ and L₂₁ each independently represent a divalent group. The divalent group is as described above.

In a specific embodiment, L_{A1} may be a divalent group having a side chain containing a hydrophilic group or a functional substance. The side chain containing a hydrophilic group or a functional substance may be a side chain containing a hydrophilic group and a functional substance. The side chain containing a hydrophilic group and a functional substance may be a side chain containing (i) a hydrophilic group, (ii) a functional substance, and (iii) a cleavable site between the hydrophilic group and the functional substance. The number of such side chains contained in the divalent group is not particularly limited, and may be, for example, 1 to 4, 1 to 3, 1 or 2, or 1.

In a specific embodiment, the two or more (e.g., two) lysine residue-modifying moieties may be represented by the following formula (I-2''): 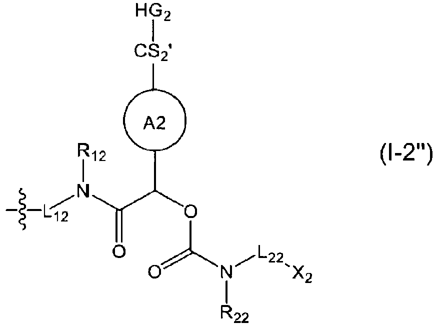 wherein
the hyphen (-) with a wavy line represents a bond to said nitrogen atom,
HG₂ represents a hydrophilic group or a monovalent group containing a hydrophilic group,
CS₂' represents a divalent group containing a cleavable site,
Ring A2 represents an optionally substituted divalent aromatic ring group (wherein the divalent aromatic ring group forms a pi electron conjugated system with said cleavable site),
R₁₂ and R₂₂ each independently represent a hydrogen atom or a monovalent group,
L₁₂ and L₂₂ each independently represent a divalent group, and
X₂ represents a functional substance,
or the following formula (II): 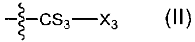 wherein
the hyphen (-) with a wavy line represents a bond to said nitrogen atom,
CS₃ represents a divalent group optionally containing a cleavable site, and
X₃ represents a functional substance. When the number of the lysine residue-modifying moieties is two, the two lysine residue-modifying moieties may be the same as or different from each other. When the number of the lysine residue-modifying moieties is four, the four lysine residue-modifying moieties may be the same as or different from each other.

HG₂ and X₂ in formula (I-2'') each are the same as those represented by formula (I-2). In formula (I-2"), CS₂' and Ring A2 are each the same as those represented by formula (I-2').

R₁₂ and R₂₂ each independently represent a hydrogen atom or a monovalent group. The monovalent group is as described above.

L₁₂ and L₂₂ each independently represent a divalent group. The divalent group is as described above.

CS₃ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS3 may be the same as or different from the divalent group with a cleavable site represented by CS₁'.

X₃ represents a functional substance. The functional substance is as described above. The functional substance represented by X₃ may be the same as or different from the functional substance represented by X₁.

In a specific embodiment, the two or more (e.g., four) lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). When the number of the lysine residue-modifying moieties is four, two first lysine residue-modifying moieties are the same, and two second lysine residue-modifying moieties are the same. The first lysine residue-modifying moieties and the second lysine residue-modifying moieties are the same as or different from each other.

The first lysine residue-modifying moiety can be represented by the following formula (I-2-1"): 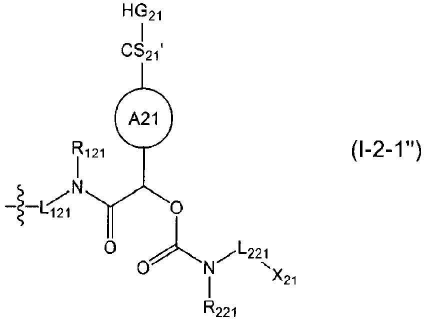 wherein
the hyphen (-) with a wavy line represents a bond to said nitrogen atom,
HG₂₁ represents a hydrophilic group or a monovalent group containing a hydrophilic group,
CS₂₁' represents a divalent group containing a cleavable site,
Ring A21 represents an optionally substituted divalent aromatic ring group (wherein the divalent aromatic ring group forms a pi electron conjugated system with said cleavable site),
R₁₂₁ and R₂₂₁ each independently represent a hydrogen atom or a monovalent group,
L₁₂₁ and L₂₂₁ each independently represent a divalent group, and
X₂₁ represents a functional substance,
or the following formula (II-1): 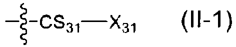 wherein
the hyphen (-) with a wavy line represents a bond to said nitrogen atom,
CS₃₁ represents a divalent group optionally containing a cleavable site, and
X₃₁ represents a functional substance.

HG₂₁ represents a hydrophilic group or a monovalent group containing a hydrophilic group. The hydrophilic group and the monovalent group are as described above. The hydrophilic group or the monovalent group containing a hydrophilic group represented by HG₂₁ may be the same as or different from the hydrophilic group or the monovalent group containing a hydrophilic group represented by HG₁.

CS₂₁' represents a divalent group containing a cleavable site. The cleavable site and the divalent group are as described above. The divalent group containing a cleavable site represented by CS₂₁' may be the same as or different from the divalent group containing a cleavable site represented by CS₁.

Ring A21 represents an optionally substituted divalent aromatic ring group (wherein the divalent aromatic ring group forms a pi electron conjugated system with said cleavable site). The optionally substituted divalent aromatic ring group is as described above. The optionally substituted divalent aromatic ring group represented by Ring A21 may be the same as or different from the optionally substituted divalent aromatic ring group represented by Ring A1.

R₁₂₁ and R₂₂₁ each independently represent a hydrogen atom or a monovalent group. The monovalent group is as described above.

L₁₂₁ and L₂₂₁ each independently represent a divalent group. The divalent group is as described above.

X₂₁ represents a functional substance. The functional substance is as described above. The functional substance represented by X₂₁ may be the same as or different from the functional substance represented by X₁.

CS₃₁ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃₁ may be the same as or different from the divalent group with a cleavable site represented by CS₁'.

X₃₁ represents a functional substance. The functional substance is as described above. The functional substance represented by X₃ may be the same as or different from the functional substance represented by X₁.

The second lysine residue-modifying moiety can be represented by the following formula (I-2-2"): 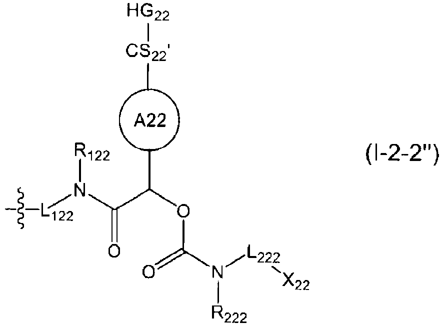 wherein
the hyphen (-) with a wavy line represents a bond to said nitrogen atom,
HG₂₂ represents a hydrophilic group or a monovalent group containing a hydrophilic group,
CS₂₂' represents a divalent group containing a cleavable site,
Ring A22 represents an optionally substituted divalent aromatic ring group (wherein the divalent aromatic ring group forms a pi electron conjugated system with said cleavable site),
R₁₂₂ and R₂₂₂ each independently represent a hydrogen atom or a monovalent group,
L₁₂₂ and L₂₂₂ each independently represent a divalent group, and
X₂₂ represents a functional substance,
or the following formula (II-2): 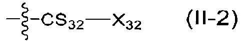 wherein
the hyphen (-) with a wavy line represents a bond to said nitrogen atom,
CS₃₂ represents a divalent group optionally containing a cleavable site, and
X₃₂ represents a functional substance.

HG₂₂ represents a hydrophilic group or a monovalent group containing a hydrophilic group. The hydrophilic group and the monovalent group are as described above. The hydrophilic group or the monovalent group containing a hydrophilic group represented by HG₂₂ may be the same as or different from the hydrophilic group or the monovalent group containing a hydrophilic group represented by HG₁ and/or HG₂₁.

CS₂₂' represents a divalent group containing a cleavable site. The cleavable site and the divalent group are as described above. The divalent group containing a cleavable site represented by CS₂₂' may be the same as or different from the divalent group containing a cleavable site represented by CS₁' and/or CS₂₁'.

Ring A22 represents an optionally substituted divalent aromatic ring group (wherein the divalent aromatic ring group forms a pi electron conjugated system with said cleavable site). The optionally substituted divalent aromatic ring group is as described above. The optionally substituted divalent aromatic ring group represented by Ring A22 may be the same as or different from the optionally substituted divalent aromatic ring group represented by Ring A1 and/or Ring A21.

R₁₂₂ and R₂₂₂ each independently represent a hydrogen atom or a monovalent group. The monovalent group is as described above.

L₁₂₂ and L₂₂₂ each independently represent a divalent group. The divalent group is as described above.

X₂₂ represents a functional substance. The functional substance is as described above. The functional substance represented by X₂₂ may be the same as or different from the functional substance represented by X₁ and/or X₂₁.

CS₃₂ represents a divalent group optionally containing a cleavable site. The divalent group optionally containing a cleavable site is a divalent group without a cleavable site or a divalent group with a cleavable site. The cleavable site and the divalent group are as described above. The divalent group optionally containing a cleavable site represented by CS₃₂ may be the same as or different from a divalent group with a cleavable site represented by CS₁ and/or a divalent group optionally containing a cleavable site represented by CS₃₁.

X₃₂ represents a functional substance. The functional substance is as described above. The functional substance represented by X₃₂ may be the same as or different from the functional substance represented by X₁ and/or X₃₁.

### 2-4. Additional characteristics of conjugates or salts thereof of the present invention

The conjugate or a salt thereof of the present invention has the desired property of being difficult to aggregate, and thus can be characterized by its aggregation rate. More specifically, the aggregation rate of the conjugate or a salt thereof of the present invention may be 5% or less. This is because the present invention makes it easy to avoid antibody aggregation. The aggregation rate is preferably 4.8% or less, more preferably 4.6% or less, still more preferably 4.4% or less, and particularly preferably 4.2% or less, 4.0% or less, 3.8% or less, 3.6% or less, 3.4% or less, 3.2% or less, 3.0% or less, 2.8% or less, or 2.6% or less. The aggregation rate of the antibody can be measured by size exclusion chromatography (SEC)-HPLC (see Examples and Chemistry Select, 2020, 5, 8435-8439).

In a preferred embodiment, the conjugate or a salt thereof of the present invention may have an aggregation rate of 2.6% or less. The aggregation rate may also be 2.4% or less, 2.2% or less, 2.0% or less, 1.8% or less, 1.6% or less, 1.4% or less, 1.2% or less, 1.0% or less, or 0.8% or less.

In a specific embodiment, the conjugation ratio of the functional substance to the antibody (functional substance/antibody) may be 10 or more. Such a conjugation ratio may be, for example, 10 to 16, preferably 10 to 14, and more preferably 10 or 12.

The conjugate or salt thereof of the present invention is useful, for example, as pharmaceuticals or reagents (e.g., diagnostic reagents and reagents for research).

The conjugate or salt thereof of the present invention may be provided in the form of a pharmaceutical composition. Such a pharmaceutical composition may comprise a pharmaceutically acceptable carrier in addition to the conjugate or salt thereof of the present invention. Examples of the pharmaceutically acceptable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogen carbonate, calcium phosphate, and calcium citrate; lubricants such as magnesium stearate, Aerosil, talc, sodium lauryl sulfate; fragrances such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspending agents such as methylcellulose, polyvinylpyrrolidone, and aluminum stearate; dispersing agents such as surfactants; diluents such as water, a physiological saline solution, and orange juice; and base waxes such as cacao butter, polyethylene glycol, and refined kerosene. The conjugate or salt thereof of the present invention may also have any modification (e.g., PEGylation) for achieving stability.

Examples of preparations suitable for oral administration include solutions in which an effective amount of a ligand is dissolved in diluents such as water, a physiological saline solution; capsules, sachets, and tablets comprising an effective amount of a ligand as a solid or granules; suspensions in which an effective amount of an active ingredient is suspended in an appropriate dispersion medium; and emulsions in which an effective amount of an active ingredient is dissolved and then emulsified in an appropriate dispersion medium.

The pharmaceutical composition is suitable for nonoral administration (e.g., intravenous injection, subcutaneous injection, intramuscular injection, local injection, and intraperitoneal administration). Examples of the pharmaceutical composition suitable for such nonoral administration include aqueous and nonaqueous, isotonic, sterile injectable solutions, which may comprise an antioxidant, a buffer, an antimicrobial agent, a tonicity agent, or the like. Examples thereof also include aqueous and nonaqueous, sterile suspensions, which may comprise a suspending agent, a solubilizing agent, a thickener, a stabilizer, an antiseptic, or the like.

The dose of the pharmaceutical composition, which varies depending on the type and activity of the active ingredient, the severity of the diseases, the species of the animal to be dosed, the drug receptivity, body weight, and age of the subject to be dosed, or the like, can be set as appropriate.

The conjugate or a salt thereof of the present invention can be produced by subjecting an antibody comprising an immunoglobulin (Ig) unit comprising two heavy chains and two light chains to (1) treatment with a reducing agent (a step of reducing the disulfide bonds in the antibody to cleave the disulfide bonds and generate thiol groups), (2) regioselective modification of amino groups in the antibody (a step of adding lysine residue-modifying moieties to the antibody), and (3) modification of thiol groups in the antibody (a step of adding cysteine residue-modifying moieties to the antibody).

As the reducing agent, any reducing agent capable of cleaving a disulfide bond to generate a thiol group can be used, and examples thereof include tricarboxylethylphosphine (TCEP), cysteine, dithiothreitol, reduced glutathione, and β-mercaptoethanol. An Ig unit usually comprises eight cysteine residues that form four disulfide bonds. Therefore, by reacting an antibody containing an Ig unit with a reducing agent, eight thiol groups can be generated from the four disulfide bonds in the Ig unit. The number of thiol groups generated in the antibody can be appropriately adjusted by controlling the type of Ig unit (e.g., an amino acid residue-modified antibody), the type and amount of the reducing agent, and the reaction time. The treatment with a reducing agent can be appropriately carried out under mild conditions that will not cause protein denaturation/degradation (e.g., cleavage of an amide bond). For example, such a reaction can be carried out at room temperature (e.g., about 15 to 30°C) in a suitable reaction system, such as in a buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and still more preferably 30 minutes to 8 hours. For details of such a reaction, see, e.g., G. J. L. Bernardes et al., Chem. Rev., 115, 2174 (2015); G. J. L. Bernardes et al., Chem. Asian. J., 4,630 (2009); B. G. Davies et al., Nat. Commun., 5,4740 (2014); A. Wagner et al., Bioconjugate. Chem., 25, 825 (2014).

Regioselective modification of amino groups in an antibody can be carried out by a method of regioselectively modifying specific lysine residues (e.g., lysine residues at positions 246/248, 288/290, or 317) in the heavy chain of the immunoglobulin unit constituting the antibody (see, e.g., WO2018/199337, WO2019/240288, WO2019/240287, and WO2020/090979). This modification can be appropriately carried out under the above-described conditions that will not cause protein denaturation/degradation (e.g., cleavage of an amide bond) (mild conditions). After regioselectively modifying specific lysine residues to introduce specific bioorthogonal functional groups into the antibody, the antibody after the introduction may be further reacted with a compound comprising another bioorthogonal functional group capable of reacting with the specific bioorthogonal functional group, or a salt thereof.

Modification of thiol groups in the antibody can be carried out by adding a compound comprising a cysteine residue-modifying moiety, or a salt thereof, (which comprises a bioorthogonal functional group capable of reacting with a thiol group) to the antibody via a reaction between the bioorthogonal functional group and the thiol group. As the bioorthogonal functional group, a group capable of efficiently reacting with a thiol group (e.g., a maleimide residue, a halocarbonyl group, a thiol group) is preferred. This modification can be appropriately carried out under the above-described conditions that will not cause protein denaturation/degradation (e.g., cleavage of an amide bond) (mild conditions). As the compound comprising a cysteine residue-modifying moiety, or a salt thereof, for example, a compound comprising a bioorthogonal functional group and a functional substance, or a salt thereof, which will be described later, can be used.

The order of the steps (1) to (3) described above can be appropriately interchanged, however, the step (3) requires modification of the thiol groups generated by the step (1), and therefore, needs to be performed after the step (1). For example, the steps may be carried out in the order of (1), (2), and (3), or (1), (3), and (2), or in the order of (2), (1), and (3). When generating a first lysine residue-modifying moiety and a second lysine residue-modifying moiety as regioselective modification of amino groups in the antibody, the modification of the first lysine residue-modifying moiety and the second lysine residue-modifying moiety can be carried out separately or sequentially. For example, one or both of the modifications of the first lysine residue-modifying moiety and the second lysine residue-modifying moiety may be carried out after the steps (1) and (3), or may be carried out before the steps (1) and (3).

The conjugate or a salt thereof of the present invention can also be produced by first generating an antibody derivative having a bioorthogonal functional group, or a salt thereof (e.g., an antibody derivative or a salt thereof in which the functional substance that is X₁ in the conjugate represented by formula (I-1) is replaced with a bioorthogonal functional group), and then reacting the generated antibody derivative or a salt thereof with a functional substance. Such a reaction can proceed by the reaction between the bioorthogonal functional group in the antibody derivative and the functional substance.

When the functional substance has a functional group that readily reacts with a bioorthogonal functional group, the functional group of the functional substance can be appropriately reacted with the bioorthogonal functional group in the antibody derivative. The functional group that readily reacts with a bioorthogonal functional group may vary depending on the specific type of the bioorthogonal functional group. A person skilled in the art can appropriately select a suitable functional group as one that readily reacts with a bioorthogonal functional group (e.g., Boutureira et al., Chem. Rev., 2015, 115, 2174-2195). Examples of functional groups that readily react with bioorthogonal functional groups include, but are not limited thereto, an alkyne group when the bioorthogonal functional group is an azide group; a maleimide group and a disulfide group when the bioorthogonal functional group is a thiol group; a hydrazine group when the bioorthogonal functional group is an aldehyde or ketone group; an azide group when the bioorthogonal functional group is a norbornene group; and an alkyne group when the bioorthogonal functional group is a tetrazine group. Of course, in the above combinations of bioorthogonal functional groups and functional groups that readily react with them, the combinations can be interchanged. Thus, if the first example of the combinations described above is interchanged, a combination of an alkyne group as the bioorthogonal functional group and an azide group as the functional group that readily reacts with the bioorthogonal functional group can be used.

If the functional substance does not have a functional group that readily reacts with the bioorthogonal functional group in the antibody derivative, the drug may be derivatized to have such a functional group. Derivatization is a matter of common general technical knowledge in the art (e.g., WO2004/010957, US Patent Application Publication No. 2006/0074008, US Patent Application Publication No. 2005/0238649). For example, derivatization may be carried out using any cross-linking agent. Alternatively, derivatization may be carried out using a specific linker having a desired functional group. In the present invention, derivatized functional substances are also merely one type of functional substance and are therefore simply referred to as "functional substances".

Alternatively, the conjugate or a salt thereof of the present invention can be produced from an antibody intermediate having thiol groups, or a salt thereof, which will be described later. More specifically, the conjugate or a salt thereof of the present invention can be produced by reacting an antibody intermediate having thiol groups, or a salt thereof, with a compound comprising a cysteine residue-modifying moiety or a salt thereof (which comprises a bioorthogonal functional group capable of reacting with a thiol group). As the bioorthogonal functional group, a group capable of efficiently reacting with a thiol group (e.g., a maleimide residue, a halocarbonyl group, a thiol group) is preferred. This modification can be appropriately carried out under the above-described conditions that will not cause protein denaturation/degradation (e.g., cleavage of an amide bond) (mild conditions). As the compound comprising a cysteine residue-modifying moiety, or a salt thereof, for example, a compound comprising a bioorthogonal functional group and a functional substance, or a salt thereof, which will be described later, can be used.

The production of the conjugate or a salt thereof can be confirmed, depending on the specific starting materials and the molecular weight of the product, for example, by reversed-phase HPLC under reducing conditions or by mass spectrometry. The conjugate or a salt thereof can be appropriately purified by any purification method such as chromatography (e.g., gel filtration chromatography, ion-exchange chromatography, reversed-phase column chromatography, high-performance liquid chromatography, and affinity chromatography).

3. Antibody intermediates having a thiol group or salts thereof
The present invention provides an antibody intermediate having thiol groups, or a salt thereof, wherein
(1) the antibody intermediate comprises an immunoglobulin unit comprising two heavy chains and two light chains, and
(2) said immunoglobulin unit comprises:
   (a') two or more thiol groups present in the side chains of two or more cysteine residues in said two heavy chains and two light chains, and
   (b') two or more lysine residue-modifying moieties regioselectively connected to the immunoglobulin unit by bonding to the nitrogen atoms in the side chains of two or more lysine residues in said two heavy chains.

In relation to (1) above, the antibody comprises an immunoglobulin (Ig) unit as described above. Examples of such an antibody include IgG antibodies, IgD antibodies, and IgE antibodies, which contain one immunoglobulin unit comprising two heavy chains and two light chains and having disulfide bonds between the heavy chains and between the heavy and light chains; IgA antibodies containing two such immunoglobulin units; and IgM antibodies containing four such immunoglobulin units, with IgG antibodies (e.g., IgG1, IgG2, IgG3, IgG4) being preferred. The antibody is preferably a human IgG monoclonal antibody, and more preferably a human IgG full-length monoclonal antibody.

In relation to (2) (a') above, the numbers of the cysteine residues and thiol groups are 2 or more, preferably 2 to 8, more preferably 4 to 8, still more preferably 6 to 8, and particularly preferably 8. An Ig unit usually comprises eight cysteine residues that form four disulfide bonds. Therefore, by reacting an antibody containing an Ig unit with a reducing agent, eight thiol groups can be generated from the four disulfide bonds in the Ig unit. The number of cysteine residues and thiol groups introduced into the Ig unit can be appropriately adjusted by controlling the type of Ig unit (e.g., an amino acid residue-modified antibody), the type and amount of the reducing agent, and the reaction time.

In relation to (2) (b') above, the two or more lysine residue-modifying moieties are regioselectively connected to the immunoglobulin unit by bonding to the nitrogen atoms in the side chains of two or more lysine residues in the two heavy chains. This connection can be achieved by a bond between the amino group in the side chain of the lysine residue and an atom or group capable of bonding thereto (e.g., a carbonyl group). The number of the lysine residue-modifying moieties is 2 or more, preferably 2 to 6, and more preferably 2 or 4.

In a specific embodiment, the number of the lysine residue-modifying moieties may be four.

The two or more lysine residues may be any of the lysine residues present at the following positions according to Eu numbering:
(A) a lysine residue present at position 246/248 in the two heavy chains;
(B) a lysine residue present at position 288/290 in the two heavy chains;
(C) a lysine residue present at position 317 in the two heavy chains; and
(D) a combination of (A) and (B), (A) and (C), or (B) and (C), or (A), (B), and (C).

Among the above, the following are preferred as lysine residues.
(A') a lysine residue present at position 246/248 in the two heavy chains;
(B') a lysine residue present at position 288/290 in the two heavy chains; and
(C') a combination of (A) and (B).

In a specific embodiment, the number of the lysine residue-modifying moieties may be four.

In a specific embodiment, the lysine residue-modifying moiety may comprise a bioorthogonal functional group. Examples of the bioorthogonal functional group include an azide group, an aldehyde group, a thiol group, an alkyne group, an alkene group, a halogen group, a tetrazine group, a nitrone group, a hydroxylamine group, a nitrile group, a hydrazine group, a ketone group, a boronic acid group, a cyanobenzothiazole group, an allyl group, a phosphine group, a maleimide group, a disulfide group, a thioester group, an α-halocarbonyl group, an isonitrile group, a sydnone group, and a selenium group.

In a specific embodiment, the bioorthogonal functional group may be a thiol group.

In a specific embodiment, the bioorthogonal functional group may be a bioorthogonal functional group other than a thiol group.

In a specific embodiment, the two or more (e.g., two) lysine residue-modifying moieties may be represented by the following formula (III): 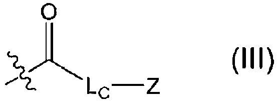 wherein
the hyphen (-) with a wavy line represents a bond to said nitrogen atom,
L_{C} represents a divalent group, and
Z represents a bioorthogonal functional group or a functional substance, or a monovalent group containing a bioorthogonal functional group or a functional substance. When the number of the lysine residue-modifying moieties is two, the two lysine residue-modifying moieties may be the same as or different from each other. When the number of the lysine residue-modifying moieties is four, the four lysine residue-modifying moieties may be the same as or different from each other.

In formula (III), L_{C} represents a divalent group. The divalent group is as described above. Preferably, L_{C} is an alkylene group (e.g., C1-C6).

Z represents a bioorthogonal functional group or a functional substance, or a monovalent group containing a bioorthogonal functional group or a functional substance. The bioorthogonal functional group, functional substance, and monovalent group are as described above.

In one embodiment, Z represents a bioorthogonal functional group, or a monovalent group containing a bioorthogonal functional group.

In another embodiment, Z represents a functional substance, or a monovalent group containing a functional substance.

In a specific embodiment, the two or more (e.g., four) lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). When the number of the lysine residue-modifying moieties is four, two first lysine residue-modifying moieties are the same, and two second lysine residue-modifying moieties are the same. The first lysine residue-modifying moieties and the second lysine residue-modifying moieties are the same as or different from each other.

The first lysine residue-modifying moiety can be represented by the following formula (III-1): 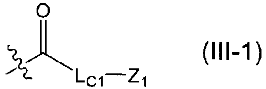 wherein
the hyphen (-) with a wavy line represents a bond to said nitrogen atom,
L_{C1} represents a divalent group, and
Z₁ represents a bioorthogonal functional group or a functional substance, or a monovalent group containing a bioorthogonal functional group or a functional substance.

L_{C1} represents a divalent group. The divalent group is as described above. Preferably, L_{C1} is an alkylene group (e.g., C 1-C6).

Z₁ represents a bioorthogonal functional group or a functional substance, or a monovalent group containing a bioorthogonal functional group or a functional substance. The bioorthogonal functional group, functional substance, and monovalent group are as described above.

The second lysine residue-modifying moiety can be represented by the following formula (III-2): 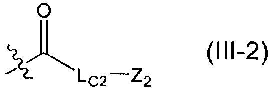 wherein
the hyphen (-) with a wavy line represents a bond to said nitrogen atom,
L_{C1} represents a divalent group, and
Z₂ represents a bioorthogonal functional group or a functional substance, or a monovalent group containing a bioorthogonal functional group or a functional substance.

L_{C2} represents a divalent group. The divalent group is as described above. Preferably, L_{C2} is an alkylene group (e.g., C1-C6). The divalent group represented by L_{C2} may be the same as or different from the divalent group represented by L_{C1}.

Z₂ represents a bioorthogonal functional group or a functional substance, or a monovalent group containing a bioorthogonal functional group or a functional substance. The bioorthogonal functional group, functional substance, and monovalent group are as described above. The bioorthogonal functional group or the functional substance, or the monovalent group containing a bioorthogonal functional group or a functional substance represented by Z₂ may be the same as or different from those represented by Z₁.

In a specific embodiment, the two or more (e.g., two) lysine residue-modifying moieties may be represented by formula (I-2) or (II) above. The details of the lysine residue-modifying moiety represented by formula (I-2) or formula (II) above are as described above. Preferably, the lysine residue-modifying moiety may be represented by formula (I-2) above.

In a specific embodiment, the two or more (e.g., two) lysine residue-modifying moieties may be represented by formula (I-2') or (II) above. The details of the lysine residue-modifying moiety represented by formula (I-2') or formula (II) above are as described above. Preferably, the lysine residue-modifying moiety may be represented by formula (I-2') above.

In a specific embodiment, the two or more (e.g., two) lysine residue-modifying moieties may be represented by formula (I-2") or (II) above. The details of the lysine residue-modifying moiety represented by formula (I-2") or formula (II) above are as described above. Preferably, the lysine residue-modifying moiety may be represented by formula (I-2") above.

In a specific embodiment, the two or more (e.g., four) lysine residue-modifying moieties may comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i). When the number of the lysine residue-modifying moieties is four, two first lysine residue-modifying moieties are the same, and two second lysine residue-modifying moieties are the same. The first lysine residue-modifying moieties and the second lysine residue-modifying moieties are the same as or different from each other.

In the specific embodiment described above, the first lysine residue-modifying moiety can be represented by formula (I-2-1) or (II-1) above, and the second lysine residue-modifying moiety can be represented by formula (I-2-2) or (II-2) above. Preferably, the first lysine residue-modifying moiety may be represented by formula (I-2-1), and the second lysine residue-modifying moiety may be represented by formula (I-2-2).

In the specific embodiment described above, the first lysine residue-modifying moiety can be represented by formula (I-2-1) or (II-1) above, and the second lysine residue-modifying moiety can be represented by formula (I-2-2) or (II-2) above. The details of the first and second lysine residue-modifying moieties represented by formula (I-2-1), (I-2-2), or (II-2) above are as described above. Preferably, the first lysine residue-modifying moiety may be represented by formula (I-2-1), and the second lysine residue-modifying moiety may be represented by formula (I-2-2).

In the specific embodiment described above, the first lysine residue-modifying moiety can be represented by formula (I-2-1') or (II-1) above, and the second lysine residue-modifying moiety can be represented by formula (I-2-2') or (II-2) above. The details of the first and second lysine residue-modifying moieties represented by formula (I-2-1'), (I-2-2'), or (II-2) above are as described above. Preferably, the first lysine residue-modifying moiety may be represented by formula (I-2-1'), and the second lysine residue-modifying moiety may be represented by formula (I-2-2').

In the specific embodiment described above, the first lysine residue-modifying moiety can be represented by formula (I-2-17") or (II-1) above, and the second lysine residue-modifying moiety can be represented by formula (I-2-2") or (II-2) above. The details of the first and second lysine residue-modifying moieties represented by formula (I-2-1''), (I-2-2''), or (II-2) above are as described above. Preferably, the first lysine residue-modifying moiety may be represented by formula (I-2-1''), and the second lysine residue-modifying moiety may be represented by formula (I-2-2'').

The antibody intermediate or a salt thereof of the present invention is useful, for example, in the convenient production of the conjugate or a salt thereof of the present invention.

The antibody intermediate or a salt thereof of the present invention can be produced by subjecting an antibody comprising an immunoglobulin (Ig) unit comprising two heavy chains and two light chains to (1) treatment with a reducing agent (a step of reducing the disulfide bonds in the antibody to cleave the disulfide bonds and generate thiol groups), and (2) regioselective modification of amino groups in the antibody (a step of adding lysine residue-modifying moieties to the antibody). The details of the steps (1) and (2) in the production of the antibody intermediate or a salt thereof of the present invention are the same as those of the steps (1) and (2) in the production of the conjugate or a salt thereof of the present invention.

The order of the steps (1) and (2) described above can be appropriately interchanged. For example, the steps may be carried out in the order of (1) and (2), or (2) and (1). When generating a first lysine residue-modifying moiety and a second lysine residue-modifying moiety as regioselective modification of amino groups in the antibody, the modification of the first lysine residue-modifying moiety and the second lysine residue-modifying moiety can be carried out separately or sequentially. For example, one or both of the modifications of the first lysine residue-modifying moiety and the second lysine residue-modifying moiety may be carried out after the step (1), or may be carried out before the step (1).

Confirmation of the generation of the antibody intermediate or a salt thereof can be performed in the same manner as described for the conjugate of the present invention. The antibody intermediate or a salt thereof can be appropriately purified by any purification method as described for the conjugate of the present invention.

4. Compounds having a bioorthogonal functional group and a functional substance or salts thereof
The present invention also provides a compound having a bioorthogonal functional group and a functional substance, or a salt thereof, represented by the following formula (IV): 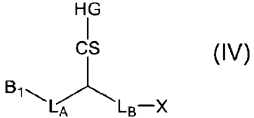 wherein
HG represents a hydrophilic group or a monovalent group containing a hydrophilic group,
CS represents a divalent group containing a cleavable site,
L_{A} and L_{B} each independently represent a divalent group,
B₁ represents a bioorthogonal functional group, and
X represents a functional substance.

HG, CS, L_{A}, L_{B}, and X in formula (IV) are as described above for HG₁, CS₁, L_{A1}, L_{B1}, and X₁ in formula (I-1), respectively. Therefore, the definitions, examples, and preferred examples of these elements and other elements related thereto are the same as those described above in formula (I-1).

B₁ represents a bioorthogonal functional group. The bioorthogonal functional group is as described above. Preferably, the bioorthogonal functional group is a group capable of efficiently reacting with a thiol group (e.g., a maleimide residue, a halocarbonyl group, or a thiol group), or a group capable of forming an amide bond together with an amino group (i.e., a group that serves as a donor for the carbonyl group moiety in an amide bond).

The present invention also provides a compound having a bioorthogonal functional group and a functional substance, or a salt thereof, represented by the following formula (IV'): 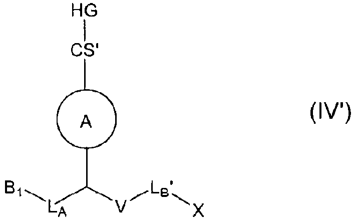 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
V represents an oxygen atom, a sulfur atom, or an amino group (NH),
L_{A} and L_{B}' each independently represent a divalent group,
B₁ represents a bioorthogonal functional group, and
X represents a functional substance.

HG, CS', Ring A, V, L_{A}, L_{B}', and X in formula (IV') are as described above for HG₁, CS₁', Ring A1, V₁, L_{A1}, L_{B1}', and X₁ in formula (I-1'). In formula (IV'), B₁ is as described above for formula (IV).

The present invention also provides a compound having a bioorthogonal functional group and a functional substance, or a salt thereof, represented by the following formula (IV''): 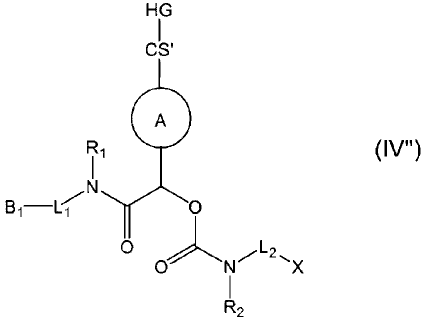 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
R₁ and R₂ each independently represent a hydrogen atom or a monovalent group,
L₁ and L₂ each independently represent a divalent group,
B₁ represents a bioorthogonal functional group, and
X represents a functional substance.

HG, CS', Ring A, R₁, R₂, L₁, L₂, and X in formula (IV") are as described above for HG₁, CS₁', Ring A1, R₁₁, R₂₁, L₁₁, L₂₁, and X₁ in formula (I-1"), respectively. In formula (IV"), B₁ is as described above for formula (IV).

The compounds represented by formulas (IV), (IV'), and (IV"), or salts thereof, are useful, for example, as compounds for the preparation of the conjugate or antibody intermediate of the present invention. The compounds represented by formulas (IV), (IV'), and (IV"), or salts thereof are also useful for derivatizing any substance such as biomolecules (e.g., proteins such as antibodies, saccharides, nucleic acids, and lipids).

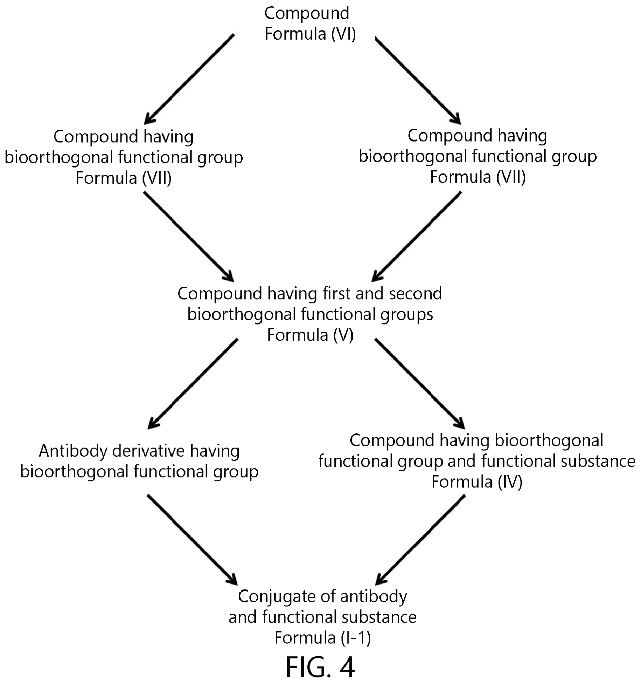

The compound having a bioorthogonal functional group and a functional substance, or a salt thereof, can be produced, for example, by reacting a compound having a first bioorthogonal functional group and a second bioorthogonal functional group, or a salt thereof with a functional substance (FIG. 4). The details of the functional substance are as described above.

The reaction of the compound having a first bioorthogonal functional group and a second bioorthogonal functional group, or a salt thereof, with a functional substance can be carried out in a suitable reaction system, for example, in an organic solvent system or an aqueous solution (e.g., buffer) system, at an appropriate temperature (e.g., about 15 to 200°C). The reaction system may contain a suitable catalyst. The reaction time is, for example, 1 minute to 20 hours, preferably 10 minutes to 15 hours, more preferably 20 minutes to 10 hours, and still more preferably 30 minutes to 8 hours. Of course, such a reaction can also be carried out under the mild conditions described above.

The production of the compound having a bioorthogonal functional group and a functional substance, or a salt thereof, can be confirmed, depending on the specific starting materials and the molecular weight of the product, for example, by NMR, HPLC, or mass spectrometry. Such a compound or a salt thereof can be appropriately purified by any purification method such as chromatography (e.g., gel filtration chromatography, ion-exchange chromatography, reversed-phase column chromatography, high-performance liquid chromatography, and affinity chromatography).

5. Compounds having a first bioorthogonal functional group and a second bioorthogonal functional group or salts thereof

The present invention also provides a compound having a first bioorthogonal functional group and a second bioorthogonal functional group, or a salt thereof, represented by the following formula (V): 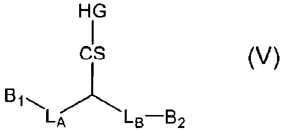 wherein
HG represents a hydrophilic group or a monovalent group containing a hydrophilic group,
CS represents a divalent group containing a cleavable site,
L_{A} and L_{B} each independently represent a divalent group,
B₁ represents a first bioorthogonal functional group, and
B₂ represents a second bioorthogonal functional group.

In formula (V), HG, CS, L_{A}, and L_{B} are as described above for formula (IV). The first bioorthogonal functional group represented by B₁ and the second bioorthogonal functional group represented by B₂ are as described above for the bioorthogonal functional group. Therefore, the definitions, examples, and preferred examples of these elements and other elements related thereto are the same as those described above.

Preferably, the second bioorthogonal functional group may be a bioorthogonal functional group that does not react with the first bioorthogonal functional group or has low reactivity towards the first bioorthogonal functional group. In this case, intermolecular reactions of the compound represented by formula (V) or a salt thereof can be reduced. Therefore, the first and second bioorthogonal functional groups can be used in combinations that do not react with each other or have low reactivity with each other. Such combinations of bioorthogonal functional groups are well known in the art. For example, for preferred bioorthogonal functional groups such as a maleimide group, a thiol group, a furan group, a halocarbonyl group, an alkene group, an alkyne group, an azide group, and a tetrazine group, examples of such combinations are as follows.

**Table A. Examples of combinations of first and second bioorthogonal functional groups that do not react with each other or are less reactive with each other**

| First bioorthogonal functional group | Second bioorthogonal functional group |
|---|---|
| Maleimide group | Halocarbonyl, alkene, or alkyne group |
| Thiol group | Furan, alkene, alkyne, azide, or tetrazine group |
| Furan group | Thiol group, or halocarbonyl group |
| Halocarbonyl group | Maleimide, furan, alkene, alkyne, azide, or tetrazine group |
| Alkene group | Maleimide, thiol, halocarbonyl, or alkyne group |
| Alkyne group | Maleimide, thiol, halocarbonyl, or alkyne group |
| Azide group | Thiol group or halocarbonyl group |
| Tetrazine group | Thiol group or halocarbonyl group |

The present invention also provides a compound having a first bioorthogonal functional group and a second bioorthogonal functional group, or a salt thereof, represented by the following formula (V'): 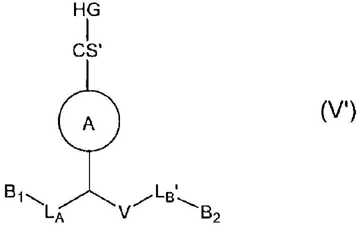 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
V represents an oxygen atom, a sulfur atom, or an amino group (NH),
L_{A} and L_{B}' each independently represent a divalent group,
B₁ represents a first bioorthogonal functional group, and
B₂ represents a second bioorthogonal functional group.

In formula (V'), HG, CS', Ring A, V, L_{A}, and L_{B}' are as described above for formula (IV'). B₁ and B₂ in formula (V') are as described above for formula (V).

The present invention also provides a compound having a first bioorthogonal functional group and a second bioorthogonal functional group, or a salt thereof, represented by the following formula (V"): 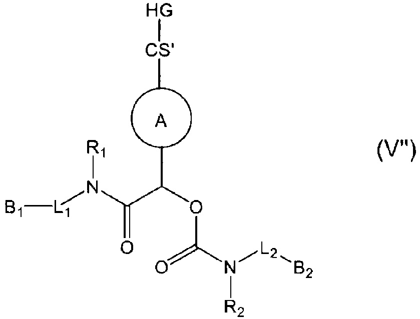 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
R₁ and R₂ each independently represent a hydrogen atom or a monovalent group,
L₁ and L₂ each independently represent a divalent group,
B₁ represents a first bioorthogonal functional group, and
B₂ represents a second bioorthogonal functional group.

In formula (V''), HG, CS', Ring A, R₁, R₂, L₁, and L₂ are as described above for formula (IV"). B₁ and B₂ in formula (V'') are as described above for formula (V).

The compounds represented by formulas (V), (V'), and (V"), or salts thereof, are useful, for example, as intermediates for the preparation of the antibody intermediate, and the compounds represented by formulas (IV), (IV'), and (IV'') of the present invention. The compounds represented by formulas (V), (V'), and (V"), or salts thereof are also useful for derivatizing any substance such as biomolecules (e.g., proteins such as antibodies, saccharides, nucleic acids, and lipids).

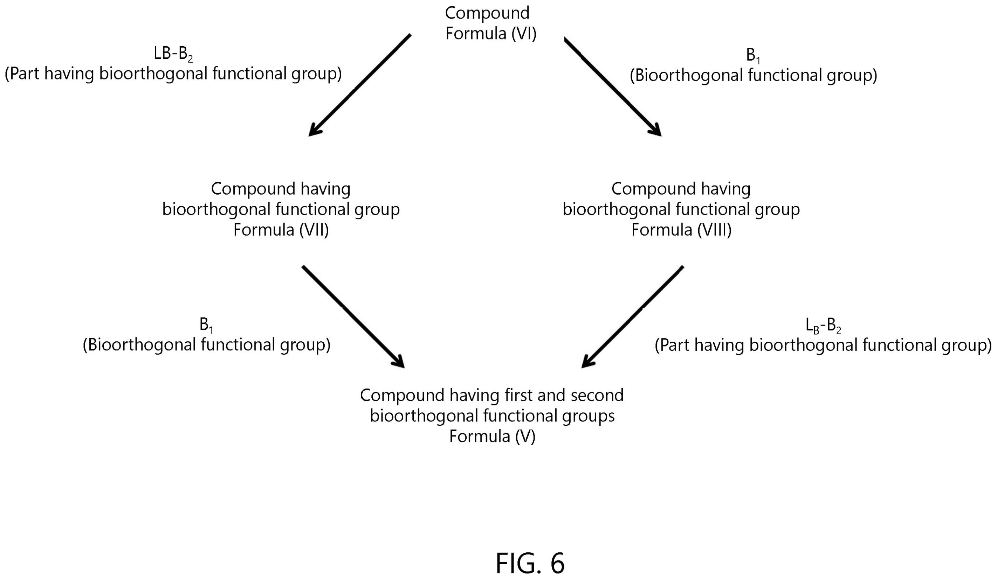

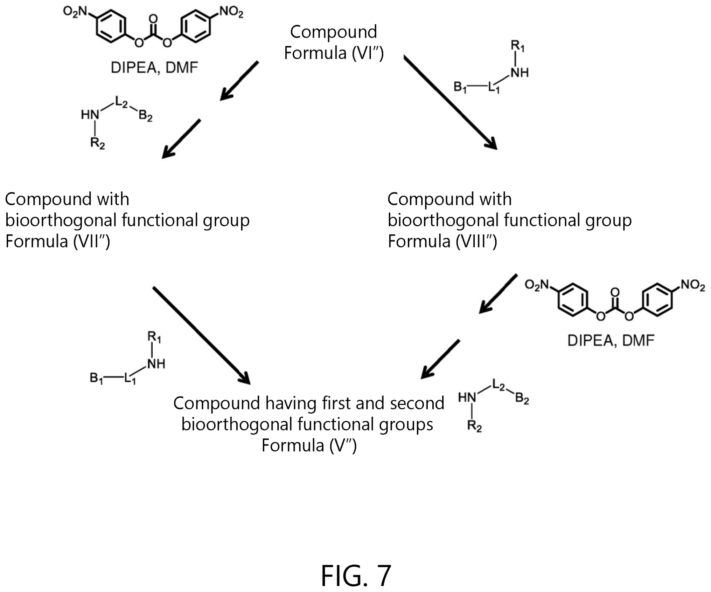

In one embodiment, a compound having a first bioorthogonal functional group and a second bioorthogonal functional group, or a salt thereof, can be produced by reacting a compound having a bioorthogonal functional group represented by formula (VII), or a salt thereof, with a suitable compound having B1 (e.g., a compound represented by B₁-L₁-NH-R₁) (FIGS. 6 and 7). The definitions, examples, and preferred examples of B₁, L₁, and R₁ are as described above.

In another embodiment, the compound having a first bioorthogonal functional group and a second bioorthogonal functional group, or a salt thereof, can be produced by reacting a compound having a bioorthogonal functional group represented by formula (VIII), or a salt thereof, with a suitable compound having L₁-B₂ (e.g., by reaction with bis(4-nitrophenyl) carbonate and N,N-diisopropylethylamine (DIPEA), followed by reaction with a compound of B₂-L₂-NH-R₂) (FIGS. 6 and 7). The definitions, examples, and preferred examples of B₂, L_{B}, and R₂ are as described above.

The reaction can be carried out in a suitable reaction system, for example, in an organic solvent system or an aqueous solution (e.g., buffer) system, at an appropriate temperature (e.g., about 15 to 200°C). The reaction system may contain a suitable catalyst. The reaction time is, for example, 1 minute to 20 hours, preferably 10 minutes to 15 hours, more preferably 20 minutes to 10 hours, and still more preferably 30 minutes to 8 hours. Of course, such a reaction can also be carried out under the mild conditions described above.

The production of the compound having a first bioorthogonal functional group and a second bioorthogonal functional group, or a salt thereof, can be confirmed, depending on the specific starting materials and the molecular weight of the product, for example, by NMR, HPLC, or mass spectrometry. Such a compound or a salt thereof can be appropriately purified by any purification method such as chromatography (e.g., gel filtration chromatography, ion-exchange chromatography, reversed-phase column chromatography, high-performance liquid chromatography, and affinity chromatography).

### 6. A series of compounds or salts thereof

### (1) Compounds or salts thereof

The present invention also provides a compound, or a salt thereof, represented by the following formula (VI): 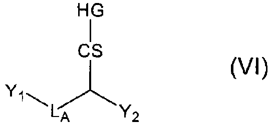 wherein
HG represents a hydrophilic group or a monovalent group containing a hydrophilic group,
CS represents a divalent group containing a cleavable site,
L_{A} each independently represents a divalent group, and
Y₁ and Y₂ each independently represents a monovalent group.

In formula (VI), HG, CS, and L_{A} are as described above for formula (IV). Therefore, the definitions, examples, and preferred examples of these elements and other elements related thereto are the same as those described above in formula (IV).

Y₁ and Y₂ each independently represents a monovalent group. The monovalent group is as described above.

The present invention also provides a compound or a salt thereof represented by the following formula (VI'): 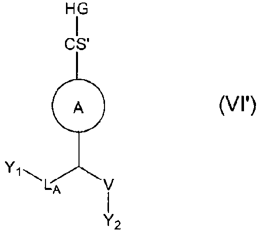 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
V represents an oxygen atom, a sulfur atom, or an amino group (NH),
L_{A} each independently represents a divalent group, and
Y₁ and Y₂ each independently represents a monovalent group.

In formula (VI'), HG, CS', Ring A, V, and L_{A} are as described above for formula (IV'). In formula (VI'), Y₁ and Y₂ are the same as those represented by formula (IV).

The present invention also provides a compound or a salt thereof represented by the following formula (VI"): 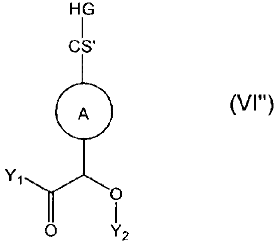 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site), and
Y₁ and Y₂ each independently represents a monovalent group.

In formula (VI"), HG, CS', and Ring A are as described above for formula (VI"). In formula (VI"), Y₁ and Y₂ are the same as those represented by formula (VI).

The compounds represented by formulas (VI), (VI'), and (VI''), or salts thereof, are useful, for example, as synthetic intermediates for the conjugates and antibody intermediates of the present invention, and other compounds of the present invention.

As a synthesis example of the compounds represented by formulas (VI), (VI'), and (VI"), or salts thereof, a synthesis example of the compound represented by formula (VI'), or a salt thereof, will be described. The compound represented by formula (VI'), or a salt thereof, can be produced, for example, by reacting a compound represented by the following formula (VI-1): 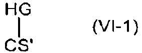 wherein
HG represents a hydrophilic group or a monovalent group containing a hydrophilic group, and
CS' represents a reactive divalent group containing a cleavable site,
or a salt thereof, with a compound represented by the following formula (VI-2): 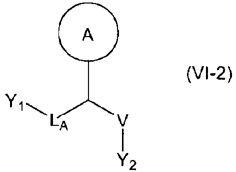 wherein
Ring A represents an optionally substituted divalent aromatic ring group capable of reacting with CS', and
Y₁ and Y₂ each independently represents a monovalent group,
or a salt thereof. The definitions, examples, and preferred examples of HG in formula (VI-1), the cleavable site and divalent group in CS', and Ring A, L_{A}, V, Y₁, and Y₂ in formula (VI-2) are as described above. Such a reaction can be carried out under the same conditions as the reaction conditions described above for the production of a compound having a first bioorthogonal functional group and a second bioorthogonal functional group, or a salt thereof.

Preferably, the compound represented by formula (VI-2) may be represented by the following formula (VI-2'): 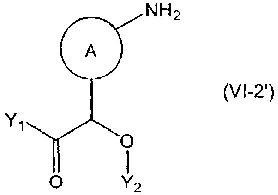 wherein
Ring A represents an optionally substituted divalent aromatic ring group, and
Y₁ and Y₂ each independently represents a monovalent group.

(2) Compound having a bioorthogonal functional group represented by formula (VII), or a salt thereof
The present invention also provides a compound having a bioorthogonal functional group, represented by the following formula (VII): 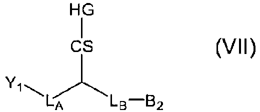 wherein
HG represents a hydrophilic group or a monovalent group containing a hydrophilic group,
CS represents a divalent group containing a cleavable site,
L_{A} and L_{B} each independently represent a divalent group,
Y₁ represents a monovalent group, and
B₂ represents a bioorthogonal functional group,
or a salt thereof.

In formula (VII), HG, CS, L_{A}, L_{B}, and B₂ are as described above for formula (IV). In formula (VII), Y₁ is the same as those described above for formula (VI).

The present invention also provides a compound having a bioorthogonal functional group, represented by the following formula (VII'): 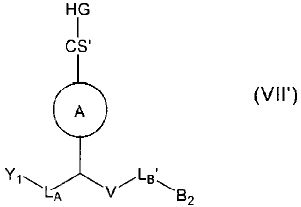 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
V represents an oxygen atom, a sulfur atom, or an amino group (NH),
L_{A} and L_{B}' each independently represent a divalent group,
Y₁ represents a monovalent group, and
B₂ represents a bioorthogonal functional group,
or a salt thereof.

In formula (VII'), HG, CS', Ring A, V, L_{A}, L_{B}', and B₂ are as described above for formula (IV'). In formula (VII'), Y₁ is the same as that described above for formula (VII).

The present invention also provides a compound having a bioorthogonal functional group, represented by the following formula (VII''): 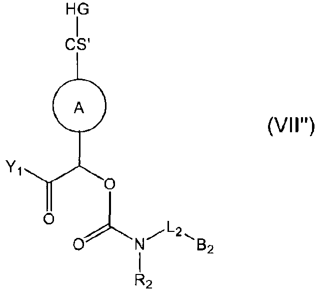 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
R₂ represents a hydrogen atom or a monovalent group,
L₂ represents a divalent group,
Y₁ represents a monovalent group, and
B₂ represents a bioorthogonal functional group,
or a salt thereof.

In formula (VII"), HG, CS', Ring A, R₂, L₂, and B₂ are as described above for formula (IV"). In formula (VII"), Y₁ is the same as described above for formula (VII).

The compounds represented by formulas (VII), (VII'), and (VII"), or salts thereof, are useful, for example, as synthetic intermediates for the conjugates and antibody intermediates of the present invention, and certain compounds of the present invention. Such a compound or a salt thereof is also useful, for example, for derivatizing functional substances.

The compounds represented by formulas (VII), (VII'), and (VII"), or salts thereof, can be produced by reacting a compound represented by formulas (VI), (VI'), and (VI"), or a salt thereof, with a suitable compound having L_{B}-B₂ (e.g., by reaction with bis(4-nitrophenyl) carbonate and N,N-diisopropylethylamine (DIPEA), followed by reaction with a compound of B₂-L₂-NH-R₂) (FIGS. 6 and 7). The definitions, examples, and preferred examples of B₂, L₂, and R₂ are as described above. Such a reaction can be carried out under the same conditions as the reaction conditions described above for the production of a compound having a first bioorthogonal functional group and a second bioorthogonal functional group, or a salt thereof.

(3) Compounds having a bioorthogonal functional group represented by formula (VIII), or salts thereof
The present invention also provides a compound having a bioorthogonal functional group, represented by the following formula (VIII): 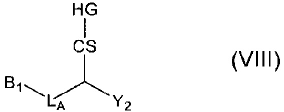 wherein
HG represents a hydrophilic group or a monovalent group containing a hydrophilic group,
CS represents a divalent group containing a cleavable site,
L_{A} each independently represents a divalent group,
B₁ represents a bioorthogonal functional group, and
Y₂ represents a monovalent group,
or a salt thereof.

In formula (VIII), HG, CS, L_{A}, and B₁ are as described above for formula (IV). In formula (VIII), Y₂ is as described above for formula (VI).

The present invention also provides a compound having a bioorthogonal functional group, represented by the following formula (VIII'): 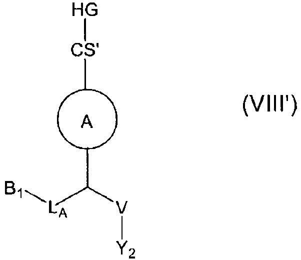 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
V represents an oxygen atom, a sulfur atom, or an amino group (NH),
L_{A} represents a divalent group,
B₁ represents a bioorthogonal functional group, and
Y₂ represents a monovalent group,
or a salt thereof.

In formula (VIII'), HG, CS', Ring A, V, L_{A}, and B₁ are as described above for formula (IV'). In formula (VIII'), Y₂ is the same as described above for formula (VI).

The present invention also provides a compound having a bioorthogonal functional group, represented by the following formula (VIII"): 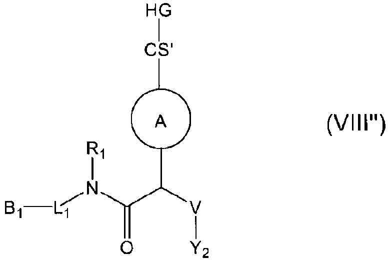 wherein
HG represents a hydrophilic group or a monovalent group containing a hydrophilic group,
CS' represents a divalent group containing a cleavable site,
Ring A represents an optionally substituted divalent aromatic ring group (wherein the divalent aromatic ring group forms a pi electron conjugated system with said cleavable site),
V represents an oxygen atom, a sulfur atom, or an amino group (NH),
R₁ represents a hydrogen atom or a monovalent group,
L₁ represents a divalent group,
B₁ represents a bioorthogonal functional group, and
Y₂ represents a monovalent group,
or a salt thereof.

In formula (VIII"), HG, CS', Ring A, R₁, L₁, and B₁ are as described above for formula (IV"). In formula (VIII''), Y₂ is the same as those represented by formula (VI).

The compounds represented by formulas (VIII), (VIII'), and (VIII"), or salts thereof, are useful, for example, as synthetic intermediates for the conjugates and antibody derivatives of the present invention, and certain compounds of the present invention. Such compounds or salts thereof are also useful for derivatizing any substance such as biomolecules (e.g., proteins such as antibodies, saccharides, nucleic acids, and lipids).

The compounds represented by formulas (VIII), (VIII'), and (VIII") or salts thereof can be produced, for example, by reacting the compounds represented by formulas (VI), (VI'), and (VI"), or salts thereof with suitable compounds having B₁ (e.g., a compound represented by B₁-L₁-NH-R₁) (FIGS. 6 and 7). The definitions, examples, and preferred examples of B₁, L₁, and R₁ are as described above. Such a reaction can be carried out under the same conditions as the reaction conditions described above for the production of a compound having a first bioorthogonal functional group and a second bioorthogonal functional group, or a salt thereof.

The production of the compounds represented by formulas (VI), (VI'), and (VI"), (VII), (VII'), and (VII''), and (VIII), (VIII'), and (VIII''), or salts thereof, can be confirmed, depending on the specific starting materials and the molecular weight of the product, for example, by NMR, HPLC, or mass spectrometry. Such a compound or a salt thereof can be appropriately purified by any purification method such as chromatography (e.g., gel filtration chromatography, ion-exchange chromatography, reversed-phase column chromatography, high-performance liquid chromatography, and affinity chromatography).

### EXAMPLES

Next, the present invention will be described in more detail with reference to Examples, but the present invention is not limited to the following Examples.

### Example 1: Synthesis of Payload-Linker and Payload mimic-Linker

The following peptides in Example 1, Ac-Glu(OtBu)-Val-Cit-OH, Z-Glu(OtBu)-Val-Cit-OH, Ac-Glu(OtBu)-Glu(OtBu)-Val-Cit-OH, and SCA(OtBu)-Glu(OtBu)-Val-Cit-OH, were all prepared by similar methods. Note that SCA (OtBu) is an abbreviation for mono-tert-butyl succinate, and SCA is an abbreviation for succinic acid.

By solid-phase peptide synthesis using Cl-TCP (Cl) ProTide Resin (CEM Corporation) by the Fmoc method, peptides capped with an acetyl group at the N-terminus and those capped with succinic acid at the N-terminus were prepared, and then stirred overnight in a 20% HFIP/dichloromethane solution to cleave the peptides from the resin while maintaining the protection of the amino acid side chains. The resin was removed by filtration, and the solution was concentrated, which was then purified by preparative HPLC to obtain peptides as products.

### Ac-Glu (OtBu)-Val-Cit-OH

¹H NMR (400 MHz, DMSO-d₆) δ12.50 (brs, 1H), 8.22 (d, J = 7.2 Hz, 1H), 8.05 (d, J = 8.0 Hz, 1H), 7.69 (d, J = 8.8 Hz, 1H), 5.95-5.93 (m, 1H), 5.38 (brs, 2H), 4.33-4.27 (m, 1H), 4.22-4.18 (m, 1H), 4.15-4.10 (m, 1H), 2.96-2.95 (m, 2H), 2.25-2.19 (m, 2H), 2.00-1.93 (m, 1H), 1.89-1.80 (m, 4H), 1.73-1.66 (m, 2H), 1.61-1.51 (m, 1H), 1.46-1.34 (m, 11H), 0.86 (d, J = 6.8 Hz, 3H), 0.83 (d, J = 6.8 Hz, 3H).
MS (ESI) m/z: 502.30 [M + H]⁺
Ac-Glu (OtBu)-Glu (OtBu)-Val-Cit-OH

¹H NMR (400 MHz, DMSO-d₆) δ12.50 (brs, 1H), 8.21 (d, J = 7.2 Hz, 1H), 8.08 (d, J = 8.0 Hz, 1H), 8.04 (d, J = 8.0 Hz, 1H), 7.68 (d, J = 8.0 Hz, 1H), 5.95 (brs, 1H), 5.37 (brs, 2H), 4.32-4.19 (m, 3H), 4.16-4.11 (m, 1H), 2.98-2.94 (m, 2H), 2.27-2.13 (m, 4H), 2.00-1.79 (m, 5H), 1.76-1.52 (m, 5H), 1.42-1.36 (m, 20H), 0.86 (d, J = 6.8 Hz, 3H), 0.82 (d, J = 6.8 Hz, 3H).
MS (ESI) m/z: 687.35[M + H]⁺

### (1-1) Synthesis of Payload-Linker (P1)

Payload-Linker (P1) was synthesized as follows.

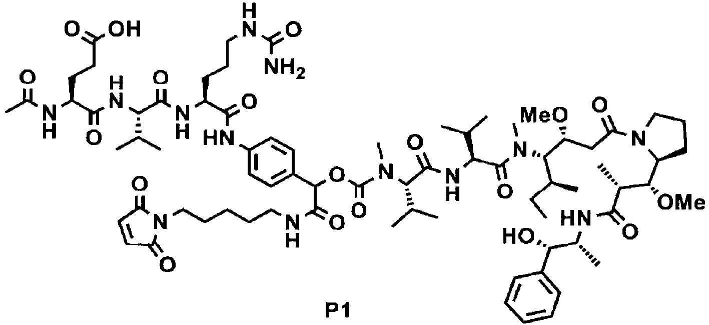

### (1-1-1) Synthesis of Compound (2)

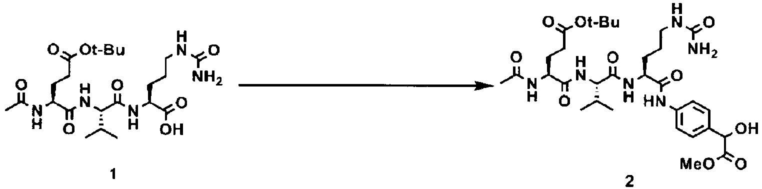

Ac-Glu(OtBu)-Val-Cit-OH (19.9 mg, 39.7 µmol) was dissolved in N,N-dimethylformamide (400 µL), and 1-[bis(dimethylamino)methylene]-1H-1,2,3,triazolo[4,5-b]pyridinium 3-oxide hexafluorophosphate (18.1 mg, 47.6 µmol) and 2,4,6-trimethylpyridine (6.27 µL, 47.6 µmol) were added, followed by stirring at room temperature for 10 minutes. Subsequently, methyl 4-aminomandelate (8.63 mg, 47.6 µmol) was added, and the mixture was stirred at room temperature for 21.5 hours, followed by purification by preparative reversed-phase chromatography. Fractions containing the product were collected, and the acetonitrile was removed by concentration under reduced pressure, followed by lyophilization to obtain Compound (2) (28.5 mg, quant).

¹H NMR (400 MHz, DMSO-d₆) δ9.95 (s, 1H), 8.07 (d, J = 7.4 Hz, 1H), 7.99 (d, J = 8.0 Hz, 1H), 7.66 (d, J = 8.4 Hz, 1H), 7.50 (d, J = 8.4 Hz, 2H), 7.25 (d, J = 8.4 Hz, 2H), 5.92 (brs, 1H), 5.36 (brs, 2H), 5.01 (s, 1H), 4.34-4.29 (m, 1H), 4.26-4.20 (m, 1H), 4.14-4.10 (m, 1H), 3.53 (s, 3H), 3.00-2.83 (m, 2H), 2.18-2.13 (m, 2H), 1.94-1.89 (m, 2H), 1.84-1.23 (m, 17H), 0.79 (d, J = 6.8 Hz, 3H), 0.75 (d, J = 6.8 Hz, 3H).
MS (ESI) m/z: 665.30 [M + H]⁺

### (1-1-2) Synthesis of Compound (3)

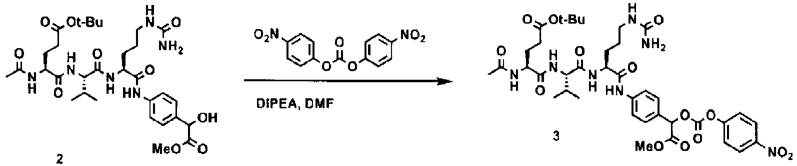

Compound (2) (105 mg, 0.158 mmol) obtained in (1-1-1) was dissolved in N,N-dimethylformamide (2 mL) and stirred under ice cooling for 5 minutes, then bis(4-nitrophenyl) carbonate (100 mg, 0.329 mmol) and N,N-diisopropylethylamine (83 µL, 0.48 mmol) were added, and the mixture was stirred at room temperature for 19.5 hours under a nitrogen atmosphere. After N,N-dimethylformamide was removed with an evaporator, the resulting crude product was dissolved by adding ethyl acetate (3 mL), followed by addition of diethyl ether (3 mL). After removing the residue from the resulting solution by filtration, the organic solvent was removed by a vacuum pump to obtain Compound (3) (102 mg, 0.123 mmol).
MS (ESI) m/z: 830.1 [M + H]⁺, 852.1 [M+Na]⁺

### (1-1-3) Synthesis of Compound (4)

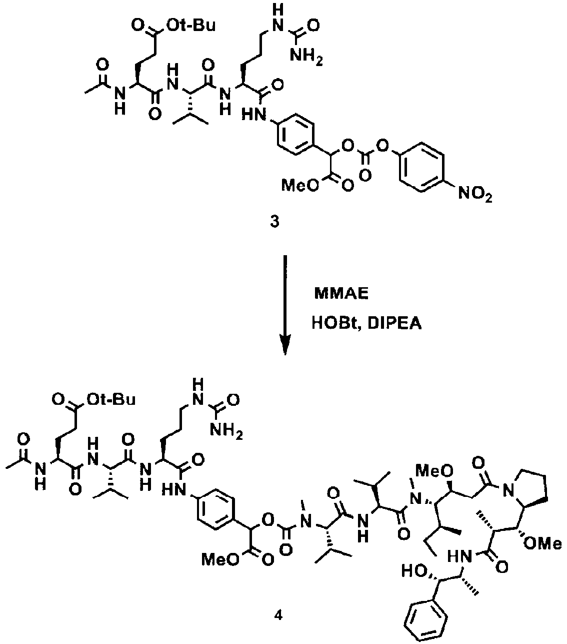

Compound (3) (69 mg, 0.083 mmol) obtained in (1-1-2) was dissolved in N,N-dimethylformamide (3.5 mL), and 1-hydroxybenzotriazole (16 mg, 0.10 mmol) and commercially available monomethylauristatin E (MMAE, 61 mg, 0.085 mmol) were added at room temperature. Subsequently, diisopropylethylamine (29 µL, 0.17 mmol) was added, and the mixture was stirred at room temperature for 22.5 hours under a nitrogen atmosphere. After the organic solvent was removed with an evaporator, a solution of acetonitrile: water = 1:1 was added, and the mixture was purified by preparative reversed-phase chromatography. Fractions containing the product were collected, and the acetonitrile was removed by concentration under reduced pressure, followed by lyophilization to obtain Compound (4) (79 mg, 0.056 mmol).
MS (ESI) m/z: 1408.9 [M + H]⁺

### (1-1-4) Synthesis of Compound (5)

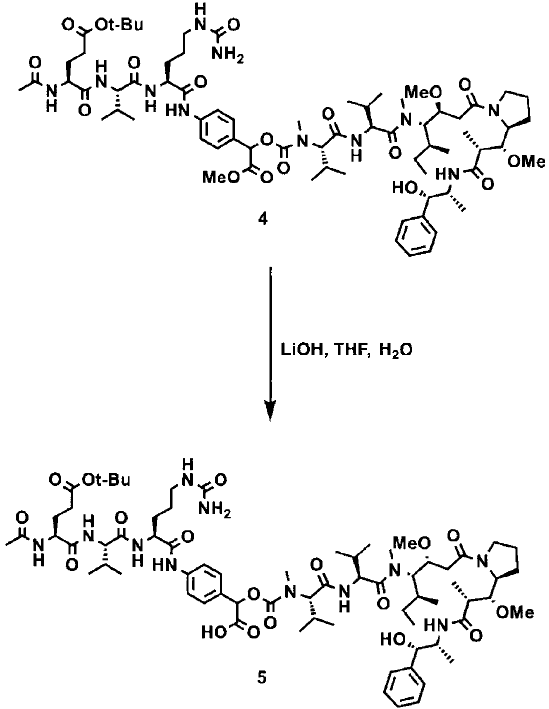

Compound (4) obtained in Example (1-1-3) (97 mg, 0.069 mmol) was dissolved in tetrahydrofuran (7 mL) and water (2 mL), and lithium hydroxide (1.0 M, 1.4 mL, 1.4 mmol) was added under ice cooling, and the mixture was stirred for one hour. After adjusting the pH to 6 by adding hydrochloric acid to the reaction solution, acetonitrile:water = 1:1 was added, and the mixture was purified by preparative reversed-phase chromatography. Fractions containing the product were collected, and the acetonitrile was removed by concentration under reduced pressure, followed by lyophilization to obtain Compound (5) (70 mg, 0.050 mmol).
MS (ESI) m/z: 1394.7 [M + H]⁺

### (1-1-5) Synthesis of Compound (6)

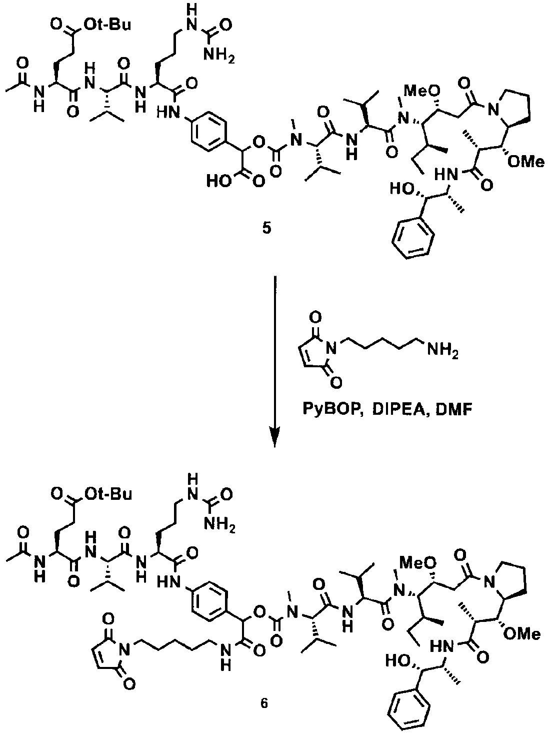

Compound (5) obtained in Example (1-1-4) (34 mg, 0.024 mmol) was dissolved in N,N-dimethylformamide (3 mL) and cooled on ice, then N,N-diisopropylethylamine (25 µL, 0.14 mmol) and 1H-benzotriazole-1-yloxytripyrrolidinophosphonium hexafluorophosphate (20 mg, 0.038 mmol) were added. Next, N-(5-aminopentyl)maleimide hydrochloride (8.7 mg, 0.040 mmol) was added, and the mixture was allowed to warm to room temperature and stirred for 20 hours. After the reaction was completed, the reaction mixture was purified by preparative reversed-phase chromatography. Fractions containing the product were collected, and the acetonitrile was removed by concentration under reduced pressure, followed by lyophilization to obtain Compound (6) (29 mg, 0.019 mmol).
MS (ESI) m/z: 1558.9 [M + H]⁺

### (1-1-6) Synthesis of Payload-Linker (P1)

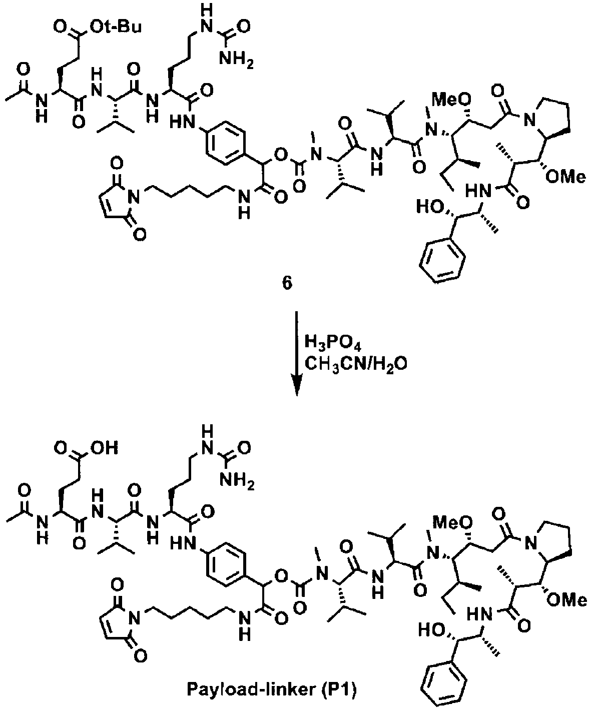

To Compound (6) (29 mg, 0.019 mmol) obtained in Example (1-1-5), acetonitrile (500 µL) and 85 wt% aqueous phosphoric acid solution (0.50 mL, 7.3 mmol) were sequentially added, and the mixture was stirred at room temperature for 3 hours. After the reaction was completed, water (2 mL) was added, and the reaction solution was purified by preparative reversed-phase chromatography, and fractions containing the product were collected, and the acetonitrile was removed by concentration under reduced pressure, followed by lyophilization to obtain Payload-Linker (P1) (18 mg, 0.012 mmol).
MS (ESI) m/z: 1502.9 [M + H]⁺

### (1-2) Synthesis of Payload-Linker (P2)

Payload-Linker (P2) was synthesized as follows.

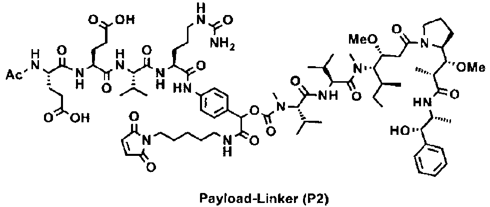

### (1-2-1) Synthesis of Compound (8)

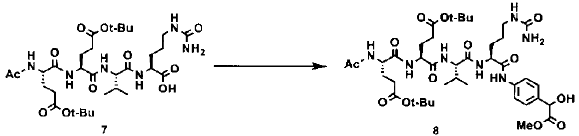

Ac-Glu(t-Bu)-Glu(t-Bu)-Val-Cit-OH (50.0 mg, 72.8 µmol) was dissolved in N,N-dimethylformamide (800 µL), and 1-[bis(dimethylamino)methylene]-1H-1,2,3,triazolo[4,5-b]pyridinium 3-oxide hexafluorophosphate (33.2 mg, 87.4 µmol) and 2,4,6-trimethylpyridine (11.5 µL, 87.4 µmol) were added, followed by stirring at room temperature for 10 minutes. Subsequently, methyl 4-aminomandelate (15.8 mg, 87.4 µmol) was added, and the mixture was stirred at room temperature for 16 hours, followed by purification by preparative reversed-phase chromatography. Fractions containing the product were collected, and the acetonitrile was removed by concentration under reduced pressure, followed by lyophilization to obtain the above-described Compound (8) (54.0 mg, 63.5 µmol).

¹H NMR (400 MHz, DMSO-d₆) δ10.00 (s, 1H), 8.26-7.88 (m, 3H), 7.68-7.60 (m, 1H), 7.57 (d, J = 8.4 Hz, 2H), 7.32 (d, J = 8.4 Hz, 2H), 6.00-5.97 (m, 1H), 5.43 (brs, 2H), 5.08 (s, 1H), 4.40-4.37 (m, 1H), 4.32-4.19 (m, 3H), 3.60 (s, 3H), 3.09-2.90 (m, 2H), 2.25-2.18 (m, 4H), 2.03-1.53 (m, 10H), 1.46-1.36 (m, 20H), 0.86 (d, J = 6.8 Hz, 3H), 0.82 (d, J = 6.8 Hz, 3H).
MS (ESI) m/z: 850.40 [M + H]⁺

### (1-2-2) Synthesis of Compound (9)

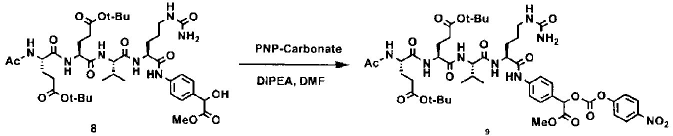

Compound (8) (140 mg, 0.165 mmol) obtained in (1-2-1) was dissolved in N,N-dimethylformamide (4 mL) and stirred under ice cooling for 5 minutes, then bis(4-nitrophenyl) carbonate (108 mg, 0.355 mmol) and N,N-diisopropylethylamine (100 µL, 0.574 mmol) were added, and the mixture was stirred at room temperature for 18 hours under a nitrogen atmosphere. After the organic solvent was removed with an evaporator, a solution of acetonitrile: water = 1:1 was added, and the mixture was purified by preparative reversed-phase chromatography. Fractions containing the product were collected, and the acetonitrile was removed by concentration under reduced pressure, followed by lyophilization to obtain Compound (9) (130 mg, 0.128 mmol).

MS (ESI) m/z: 1015.6 [M + H]⁺

### (1-2-3) Synthesis of Compound (10)

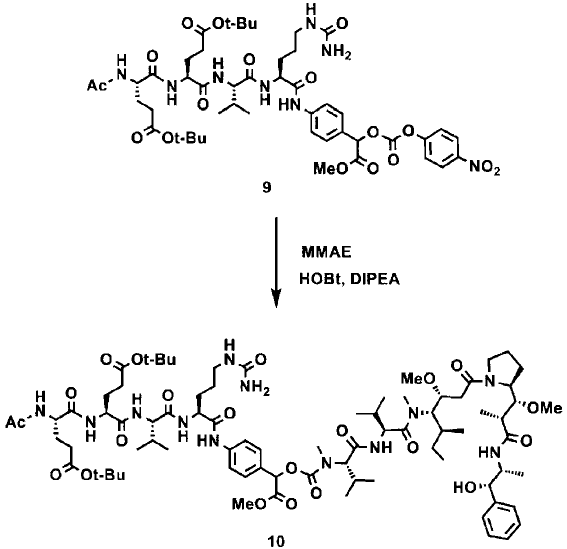

Compound (9) (85 mg, 0.084 mmol) obtained in Example (1-2-2) was dissolved in N,N-dimethylformamide (2 mL), and 1-hydroxybenzotriazole (20 mg, 0.13 mmol) and commercially available monomethylauristatin E (MMAE, 63 mg, 0.088 mmol) were added at room temperature. Subsequently, diisopropylethylamine (75 µL, 0.43 mmol) was added, and the mixture was stirred at room temperature for 23 hours under a nitrogen atmosphere. After the organic solvent was removed with an evaporator, a solution of acetonitrile: water = 1:1 was added, and the mixture was purified by preparative reversed-phase chromatography. Fractions containing the product were collected, and the acetonitrile was removed by concentration under reduced pressure, followed by lyophilization to obtain Compound (10) (94 mg, 0.059 mmol).

MS (ESI) m/z: 1593.6 [M + H]⁺

### (1-2-4) Synthesis of Compound (11)

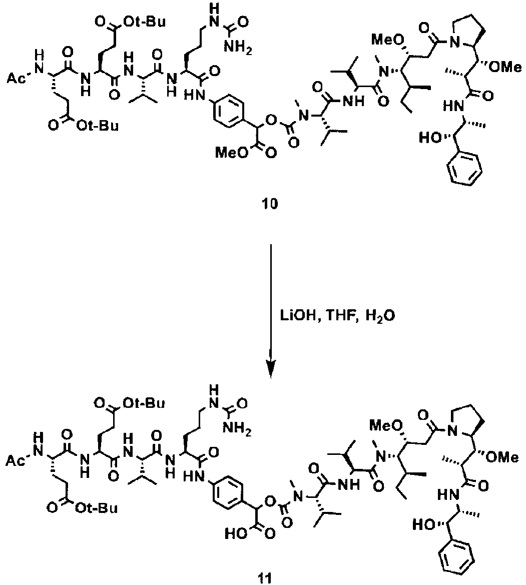

Compound (10) obtained in Example (1-2-3) (94 mg, 0.059 mmol) was dissolved in tetrahydrofuran (5 mL) and water (2 mL), and lithium hydroxide (1.0 M, 0.6 mL, 0.6 mmol) was added under ice cooling, and the mixture was stirred for one hour. After adjusting the pH to 5 by adding hydrochloric acid to the reaction solution, acetonitrile: water = 1:1 was added, and the mixture was purified by preparative reversed-phase chromatography. Fractions containing the product were collected, and the acetonitrile was removed by concentration under reduced pressure, followed by lyophilization to obtain Compound (11) (77 mg, 0.049 mmol).

MS (ESI) m/z: 1579.7 [M + H]⁺

### (1-2-5) Synthesis of Compound (12)

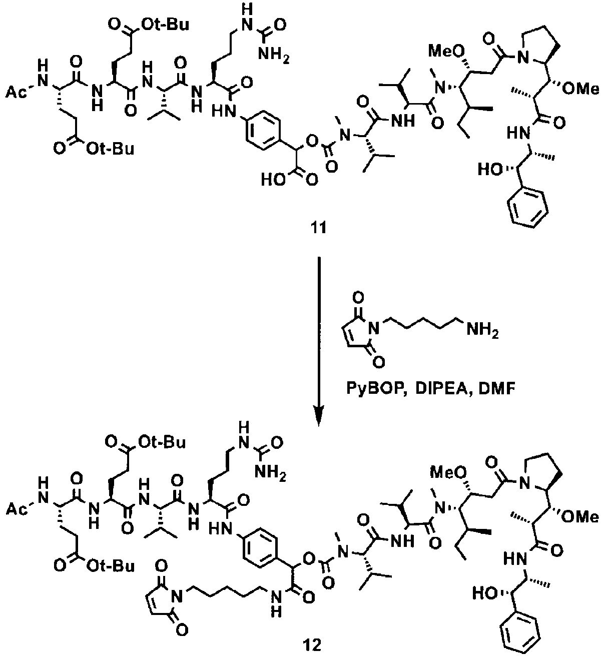

Compound (11) (77 mg, 0.049 mmol) obtained in Example (1-2-4) was dissolved in N,N-dimethylformamide (3 mL) and cooled on ice, then N,N-diisopropylethylamine (50 µL, 0.29 mmol) and 1H-benzotriazole-1-yloxytripyrrolidinophosphonium hexafluorophosphate (87 mg, 0.17 mmol) were added. Next, N-(5-aminopentyl)maleimide hydrochloride (35 mg, 0.16 mmol) was added, and the mixture was allowed to warm to room temperature and stirred for 20 hours. After the reaction was completed, the reaction mixture was purified by preparative reversed-phase chromatography. Fractions containing the product were collected, and the acetonitrile was removed by concentration under reduced pressure, followed by lyophilization to obtain Compound (12) (65 mg,0.037 mmol).

MS (ESI) m/z: 1744.7 [M + H]⁺

### (1-2-6) Synthesis of Payload-Linker (P2)

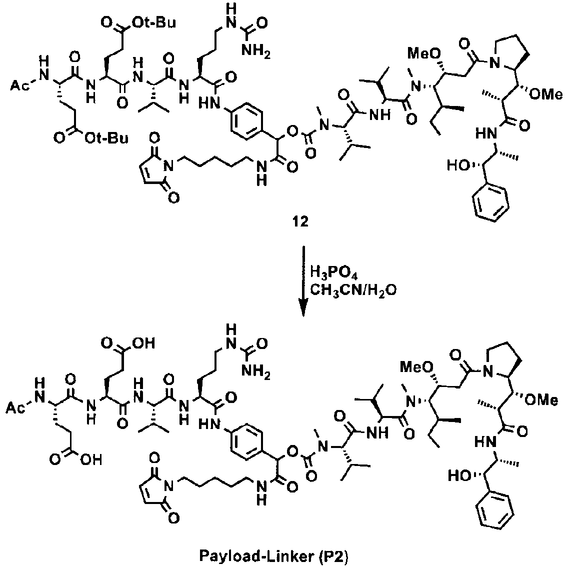

To Compound (12) (65 mg, 0.037 mmol) obtained in Example (1-2-5), acetonitrile (2 mL) and 85 wt% aqueous phosphoric acid solution (1.00 mL, 14.6 mmol) were sequentially added, and the mixture was stirred at room temperature for 6 hours. After the reaction was completed, water (2 mL) was added, and the reaction solution was purified by preparative reversed-phase chromatography, and fractions containing the product were collected, and the acetonitrile was removed by concentration under reduced pressure, followed by lyophilization to obtain Payload-Linker (P2) (49 mg, 0.030 mmol).

MS (ESI) m/z: 1631.6 [M + H]⁺

### (1-3) Synthesis of Payload-Linker (P3)

Payload-Linker (P3) was synthesized as follows.

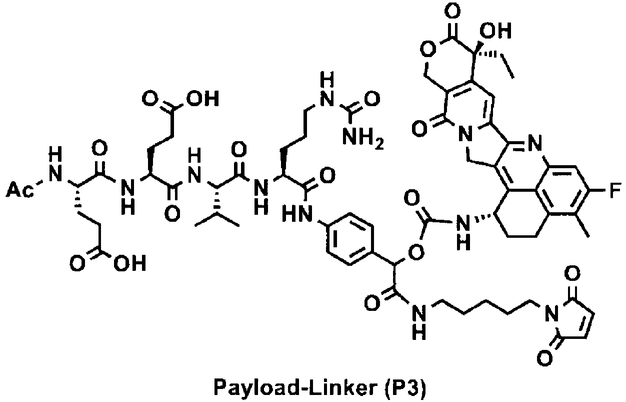

### (1-3-1) Synthesis of Compound (13)

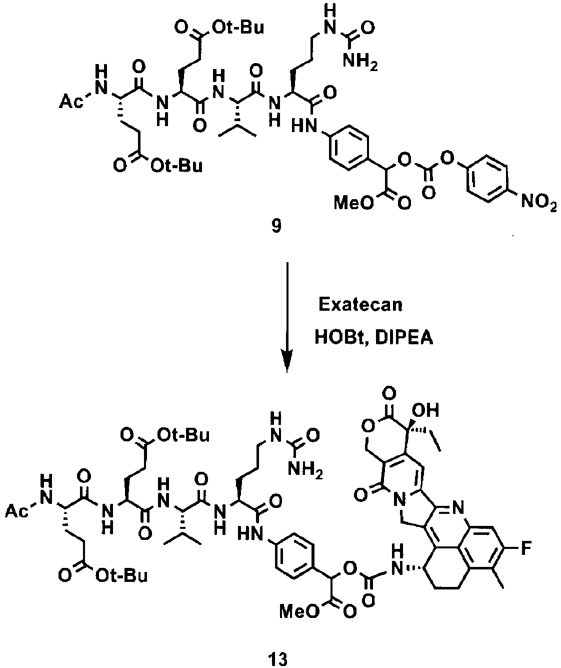

Compound (9) (67 mg, 0.066 mmol) obtained in Example (1-2-3) was dissolved in N,N-dimethylformamide (2 mL), and 1-hydroxybenzotriazole (17 mg, 0.11 mmol) and commercially available exatecan mesylate (CAS: 169869-90-3, 35 mg, 0.066 mmol) were added at room temperature. Subsequently, diisopropylethylamine (50 µL, 0.29 mmol) was added, and the mixture was stirred at room temperature for 4 hours under a nitrogen atmosphere. After the organic solvent was removed with an evaporator, a solution of acetonitrile: water = 1:1 was added, and the mixture was purified by preparative reversed-phase chromatography. Fractions containing the product were collected, and the acetonitrile was removed by concentration under reduced pressure, followed by lyophilization to obtain Compound (13) (57 mg, 0.043 mmol).
MS (ESI) m/z: 1311.7 [M + H]⁺

### (1-3-2) Synthesis of Compound (14)

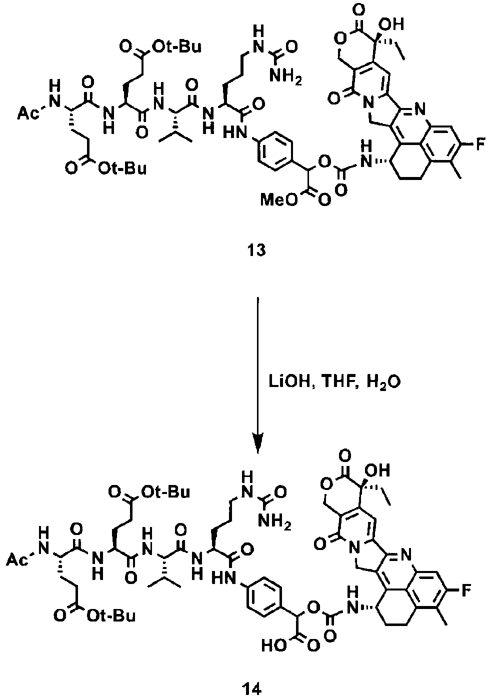

Compound (13) (57 mg, 0.043 mmol) obtained in Example (1-3-1) was dissolved in tetrahydrofuran (3 mL) and water (1.5 mL), and lithium hydroxide (1.0 M, 0.5 mL, 0.5 mmol) was added under ice cooling, and the mixture was stirred for one hour. After adjusting the pH to 5 by adding hydrochloric acid to the reaction solution, acetonitrile: water = 1:1 was added, and the mixture was purified by preparative reversed-phase chromatography. Fractions containing the product were collected, and the acetonitrile was removed by concentration under reduced pressure, followed by lyophilization to obtain Compound (14) (45 mg, 0.035 mmol).
MS (ESI) m/z: 1297.6 [M + H]⁺

### (1-3-3) Synthesis of Compound (15)

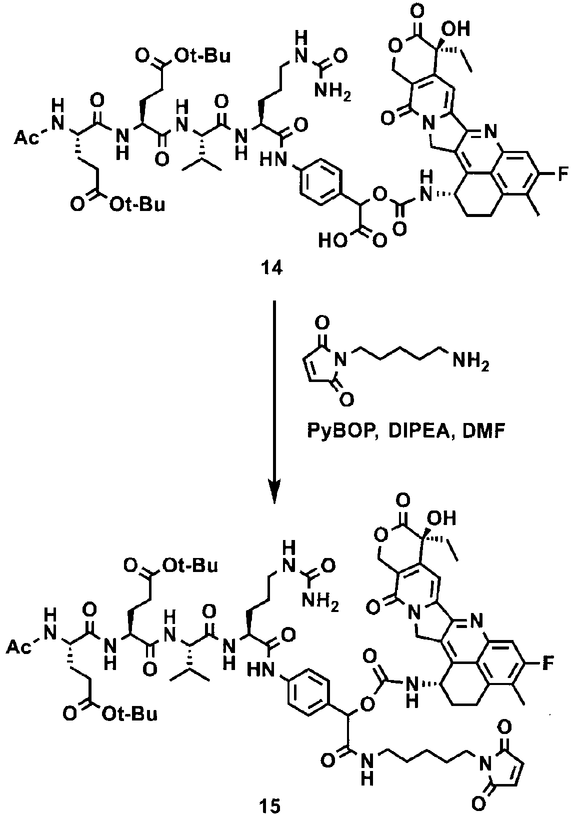

Compound (14) (45 mg, 0.035 mmol) obtained in Example (1-3-2) was dissolved in N,N-dimethylformamide (3 mL) and cooled on ice, then N,N-diisopropylethylamine (30 µL, 0.17 mmol) and 1H-benzotriazole-1-yloxytripyrrolidinophosphonium hexafluorophosphate (55 mg, 0.11 mmol) were added. Next, N-(5-aminopentyl)maleimide hydrochloride (22 mg, 0.10 mmol) was added, and the mixture was allowed to warm to room temperature and stirred for 18 hours. After the reaction was completed, the reaction mixture was purified by preparative reversed-phase chromatography. Fractions containing the product were collected, and the acetonitrile was removed by concentration under reduced pressure, followed by lyophilization to obtain Compound (15) (22 mg, 0.015 mmol).
MS (ESI) m/z: 1462.7 [M + H]⁺

### (1-3-4) Synthesis of Payload-Linker (P3)

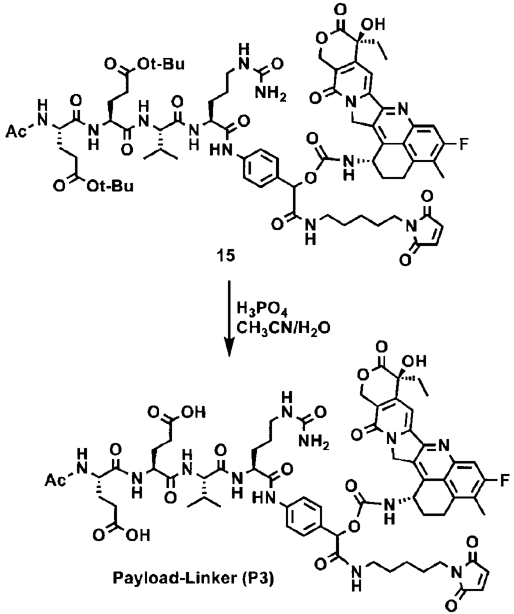

To Compound (15) (22 mg, 0.015 mmol) obtained in Example (1-3-3), acetonitrile (1 mL) and 85 wt% aqueous phosphoric acid solution (1.0 mL, 14.6 mmol) were sequentially added, and the mixture was stirred at room temperature for 1.5 hours. After the reaction was completed, water (1 mL) was added, and the reaction solution was purified by preparative reversed-phase chromatography, and fractions containing the product were collected, and the acetonitrile was removed by concentration under reduced pressure, followed by lyophilization to obtain Payload-Linker (P3) (18.8 mg, 0.0139 mmol).

¹H NMR (300 MHz; DMSO-d6) δ10.04 (s, 1H), 8.10-8.05 (m, 4H), 7.80-7.77 (m, 2H), 7.59-7.55 (m, 2H), 7.35-7.30 (m, 3H), 6.99 (d, J = 8.8 Hz, 2H), 6.54-6.53 (m, 1H), 6.01 (brs, 1H), 5.74 (d, J = 9.0 Hz, 1H), 5.43 (brs, 4H), 5.33-5.28 (m, 2H), 4.29-4.15 (m, 4H), 3.19-2.98 (m, 5H), 2.43-2.38 (m, 5H), 2.27-2.20 (m, 7H), 1.95-1.83 (m, 8H), 1.73-1.62 (m, 4H), 1.42-1.30 (m, 7H), 1.23-1.13 (m, 3H), 0.84 (m, 9H).
MS (ESI) m/z: 1349.2 [M + H]⁺

### (1-4) Synthesis of Payload-Linker (P4)

Payload-Linker (P4) shown below was synthesized in the same manner as for the synthesis of Payload-Linker (P1), except that exatecan mesylate was used instead of MMAE.

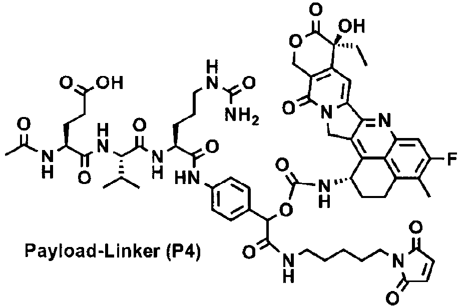

MS (ESI) m/z: 1220.50 [M + H]⁺

### (1-5) Synthesis of Payload mimic-Linker (P5)

Payload mimic-Linker (P5) was synthesized as follows.

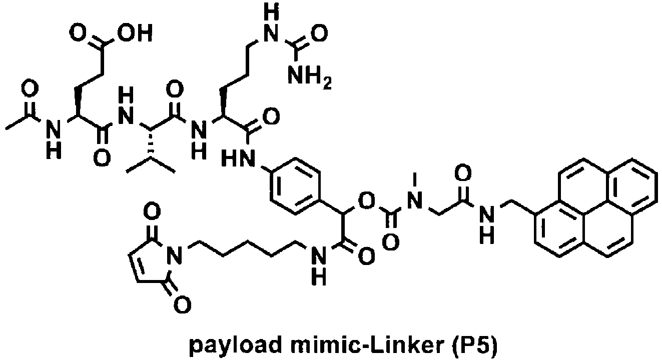

### (1-5-1) Synthesis of Compound (16)

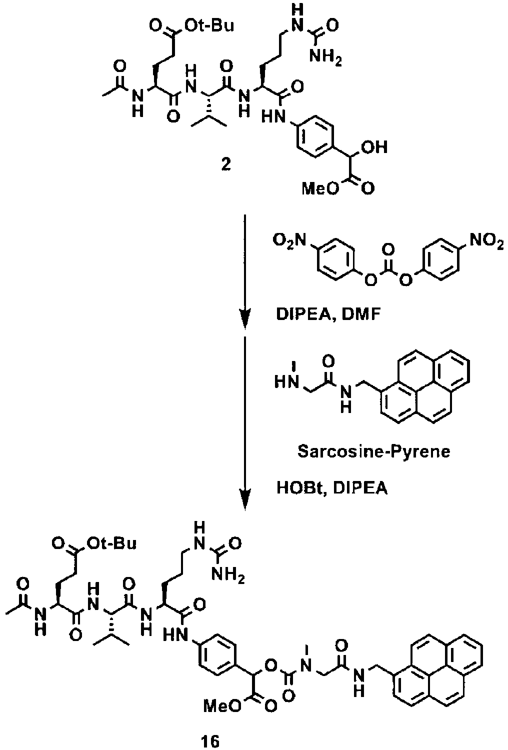

Alcohol (2) (28.5 mg) obtained in Example (1-1-1) was dissolved in N,N-dimethylformamide (430 µL) and stirred under ice cooling for 5 minutes, then bis(4-nitrophenyl) carbonate (26.6 mg, 85.7 µmol) and N,N-diisopropylethylamine (11.1 µL, 64.4 µmol) were added, and the mixture was stirred at room temperature for 1.5 hours. LC-MS tracking of the reaction revealed the presence of unreacted starting material, and therefore bis(4-nitrophenyl) carbonate (13.3 mg, 42.9 µmol) and N,N-diisopropylethylamine (5.54 µL, 32.2 µmol) were added, and the mixture was stirred at room temperature for 5 hours. Subsequently, the mixture was cooled on ice, then Sarcosine-Pyrene (64.9 mg, 215 µmol), 1-hydroxybenzotriazole (8.7 mg, 64 µmol), and N,N-diisopropylethylamine (57.2 µL, 333 µmol) were added, and the mixture was stirred at room temperature for 16 hours. After the reaction, the reaction mixture was purified by preparative reversed-phase chromatography. Fractions containing the product were collected, and the acetonitrile was removed by concentration under reduced pressure, followed by lyophilization to obtain the above-described Compound (16) (26.1 mg, 26.3 µmol).

¹H NMR (400 MHz, DMSO-d₆) δ10.09-10.07 (m, 1H), 8.63-8.60 (m, 1H), 8.33-7.65 (m, 13H), 7.60-7.57 (m, 2H), 7.37-7.32 (m, 2H), 5.94-5.91 (m, 1H), 5.72-5.70 (m, 1H), 5.37 (brs, 2H), 4.98-4.96 (m, 1H), 4.93-4.00 (m, 4H), 3.85-3.75 (m, 1H), 3.56-3.55 (m, 3H), 2.97-2.87 (m, 5H), 2.17-2.13 (m, 2H), 1.93-1.17 (m, 19H), 0.80-0.73 (m, 6H).
MS (ESI) m/z: 993.40 [M + H]⁺

### (1-5-2) Synthesis of Compound (17)

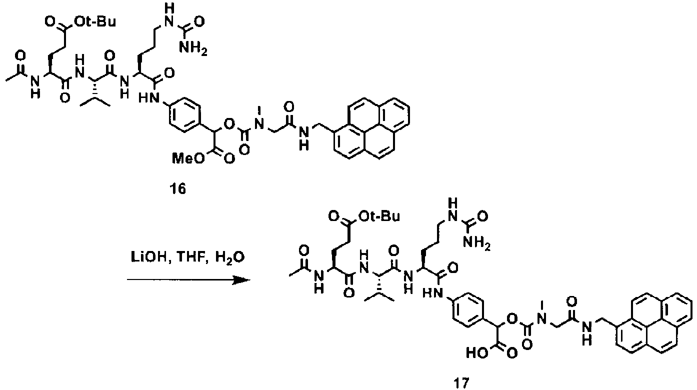

Compound (16) (10.8 mg, 10.9 µmol) obtained in Example (1-5-1) was dissolved in tetrahydrofuran (700 µL) and water (400 µL) and stirred under ice cooling for 5 minutes, then 1 M aqueous lithium hydroxide solution (109 µL, 109 µmol) was added, and the mixture was stirred at room temperature for one hour. After the reaction was completed, the pH was adjusted to about 6 with 0.1 M hydrochloric acid, and the reaction mixture was purified by preparative reversed-phase chromatography. Fractions containing the product were collected, and the acetonitrile was removed by concentration under reduced pressure, followed by lyophilization to obtain Compound (17) (4.5 mg, 4.6 µmol).

¹H NMR (400 MHz, DMSO-d₆) δ13.05 (brs, 1H), 10.08-10.05 (m, 1H), 8.62-8.59 (m, 1H), 8.33-7.56 (m, 15H), 7.37-7.35 (m, 2H), 5.92 (brs, 1H), 5.61-5.60 (m, 1H), 5.36 (brs, 2H), 4.98-4.03 (m, 5H), 3.88-3.74 (m, 1H), 2.99-2.83 (m, 5H), 2.15-2.13 (m, 2H), 1.93-1.14 (m, 19H), 0.84-0.73 (m, 6H).
MS (ESI) m/z: 979.40 [M + H]⁺

### (1-5-3) Synthesis of Compound (18)

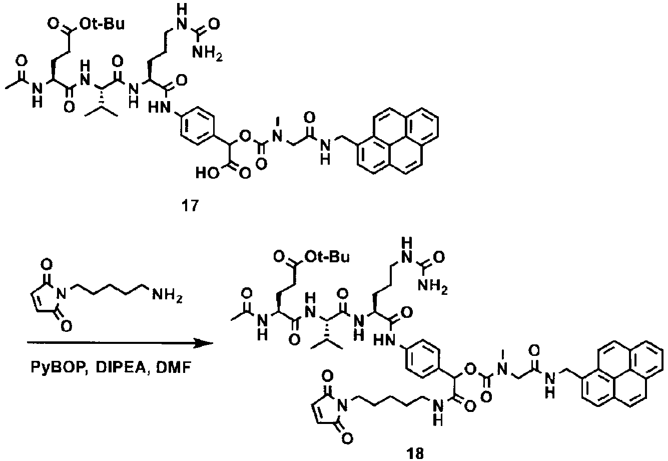

Compound (17) (3.7 mg, 3.8 µmol) obtained in Example (1-5-2) was dissolved in N,N-dimethylformamide (400 µL) and cooled on ice, then N,N-diisopropylethylamine (1.9 µL, 11 µmol) and 1H-benzotriazole-1-yloxytripyrrolidinophosphonium hexafluorophosphate (2.9 mg,5.6 µmol) were added. Next, N-(5-aminopentyl)maleimide hydrochloride (1.3 mg, 5.7 µmol) was added, and the mixture was allowed to warm to room temperature and stirred for 2 hours. After the reaction was completed, the reaction mixture was purified by preparative reversed-phase chromatography. Fractions containing the product were collected, and the acetonitrile was removed by concentration under reduced pressure, followed by lyophilization to obtain Compound (18) (1.3 mg, 1.1 µmol).

¹H NMR (400 MHz, DMSO-d₆) δ10.02-9.99 (m, 1H), 8.85-7.88 (m, 14H), 7.67-7.65 (m, 1H), 7.60-7.51 (m, 2H), 7.33-7.27 (m, 2H), 6.91-6.87 (m, 2H), 5.92-5.91 (m, 1H), 5.63-5.62 (m, 1H), 5.36 (brs, 2H), 5.07-4.92 (m, 2H), 4.35-3.76 (m, 5H), 3.18-3.14 (m, 1H), 2.99-2.83 (m, 7H), 2.17-2.13 (m, 2H), 1.95-1.89 (m, 1H), 1.85-1.72 (m, 4H), 1.66-1.45 (m, 3H), 1.40-1.17 (m, 15H), 1.05-1.01 (m, 2H), 0.83-0.73 (m, 6H).
MS (ESI) m/z: 1143.45 [M + H]⁺

### (1-5-4) Synthesis of Payload mimic-Linker (P5)

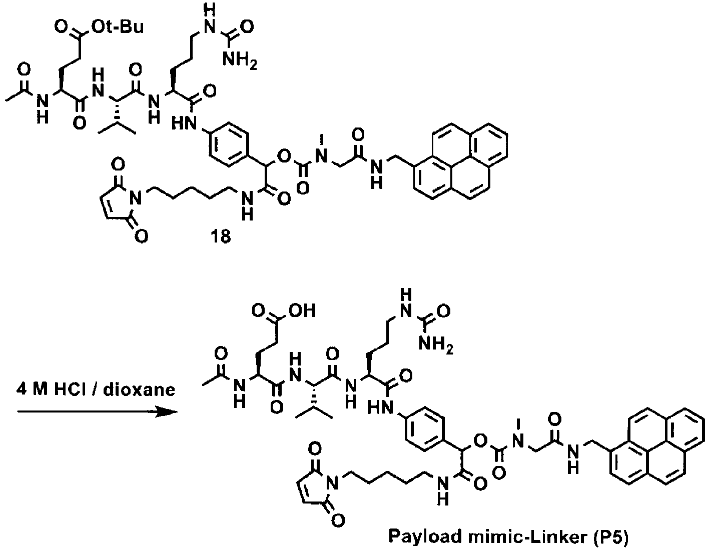

To Compound (18) (2.2 mg, 1.9 µmol) obtained in Example (1-5-3), 1,4-dioxane (380 µL) and 4 M HCl in dioxane (95 µL, 380 µmol) were sequentially added, and the mixture was stirred at room temperature for 4 hours. After cooling on ice, N,N-diisopropylethylamine (71.8 µL, 418 µmol) was added, and the mixture was stirred at room temperature for 10 minutes. The reaction solution was purified by preparative reversed-phase chromatography, and fractions containing the product were collected, and the acetonitrile was removed by concentration under reduced pressure, followed by lyophilization to obtain Payload mimic-Linker (P5) (2.1 mg, 1.9 µmol).

¹H NMR (400 MHz, DMSO-d₆) δ12.06 (brs, 1H), 10.03-10.00 (m, 1H), 8.84-7.88 (m, 14H), 7.67-7.64 (m, 1H), 7.56-7.51 (m, 2H), 7.33-7.27 (m, 2H), 6.90-6.87 (m, 2H), 5.92-5.90 (m, 1H), 5.63-5.61 (m, 1H), 5.36 (brs, 2H), 5.08-4.96 (m, 2H), 4.35-3.76 (m, 5H), 3.18-3.14 (m, 1H), 2.97-2.83 (m, 7H), 2.20-2.16 (m, 2H), 1.93-1.88 (m, 1H), 1.81-1.78 (m, 4H), 1.69-1.53 (m, 3H), 1.35-1.17 (m, 6H), 1.08-1.01 (m, 2H), 0.81-0.73 (m, 6H).
MS (ESI) m/z: 1087.45 [M + H]⁺

### (1-6) Synthesis of Payload mimic-Linker (P6)

Payload mimic-Linker (P6) was synthesized as follows.

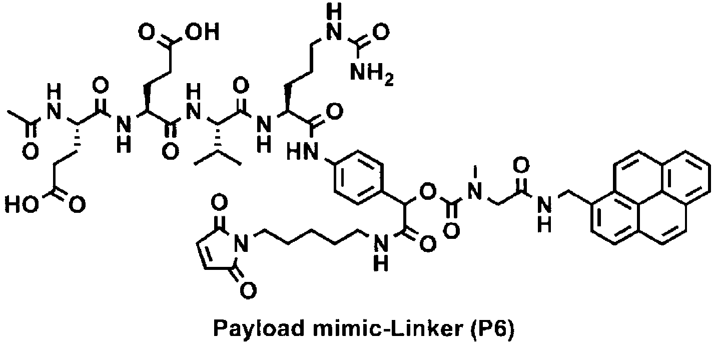

### (1-6-1) Synthesis of Compound (19)

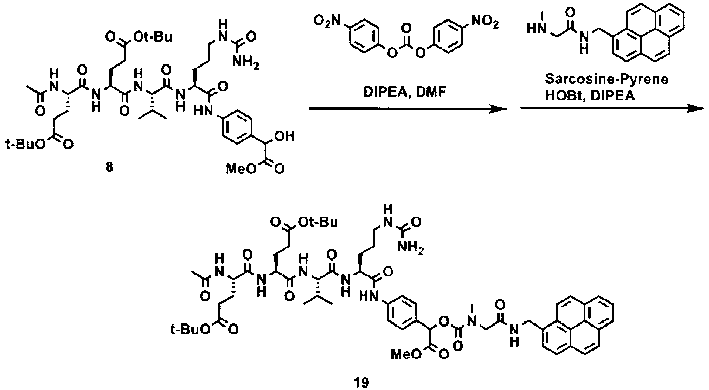

Compound (8) (50.3 mg,59.2 µmol) obtained in Example (1-2-1) was dissolved in N,N-dimethylformamide (650 µL) and stirred under ice cooling for 5 minutes, then bis(4-nitrophenyl) carbonate (54.0 mg,178 µmol) and N,N-diisopropylethylamine (22.7 µL,133 µmol) were added, and the mixture was stirred at room temperature for 5 hours under a nitrogen atmosphere. Subsequently, the mixture was cooled on ice, then Sarcosine-Pyrene (89.5 mg, 296 µmol), 1-hydroxybenzotriazole (12.0 mg, 88.8 µmol), and N,N-diisopropylethylamine (78.1 µL, 459 µmol) were added, and the mixture was stirred at room temperature for 18 hours. After the reaction, the reaction mixture was purified by preparative reversed-phase chromatography. Fractions containing the product were collected, and the acetonitrile was removed by concentration under reduced pressure, followed by lyophilization to obtain the above-described Compound (19) (34.0 mg, 28.9 µmol).

¹H NMR (400 MHz, DMSO-d₆) δ10.14-10.12 (m, 1H), 8.69-8.67 (m, 1H), 8.40-7.82 (m, 13H), 7.76-7.72 (m, 1H), 7.68-7.65 (m, 2H), 7.44-7.39 (m, 2H), 5.99 (brs, 1H), 5.79 (d, J = 6.4 Hz, 1H), 5.44 (brs, 2H), 5.05-4.97 (m, 2H), 4.44-4.08 (m, 4H), 3.93-3.80 (m, 1H), 3.63-3.62 (m, 3H), 3.05-2.92 (m, 5H), 2.25-2.14 (m, 4H), 2.00-1.28 (m, 30H), 0.87-0.80 (m, 6H).
MS (ESI) m/z: 1178.50 [M + H]⁺

### (1-6-2) Synthesis of Compound (20)

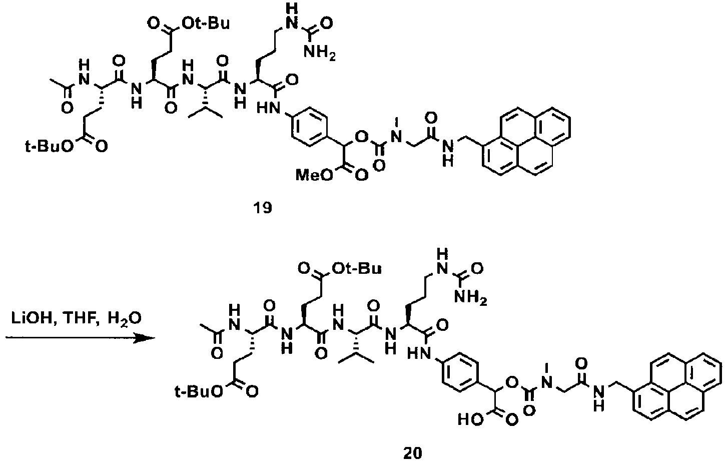

Compound (19) (30.7 mg, 26.1 µmol) obtained in Example (1-6-1) was dissolved in tetrahydrofuran (2.25 mL) and water (0.75 mL) and stirred under ice cooling for 5 minutes, then lithium hydroxide monohydrate (5.5 mg, 0.13 mmol) was added, and the mixture was stirred at room temperature for 4 hours. After the reaction was completed, the pH was adjusted to about 6 with 0.1 M hydrochloric acid, and the reaction mixture was purified by preparative reversed-phase chromatography. Fractions containing the product were collected, and the acetonitrile was removed by concentration under reduced pressure, followed by lyophilization to obtain the above-described Pyrene (20) (17.2 mg, 14.8 µmol).

¹H NMR (400 MHz, DMSO-d₆) δ13.06 (brs, 1H), 10.13-10.10 (m, 1H), 8.69-8.66 (m, 1H), 8.40-7.85 (m, 13H), 7.77-7.73 (m, 1H), 7.67-7.64 (m, 2H), 7.44-7.42 (m, 2H), 5.99 (brs, 1H), 5.68-5.67 (m, 1H), 5.44 (brs, 2H), 5.05-4.96 (m, 2H), 4.46-4.10 (m, 4H), 3.92-3.81 (m, 1H), 3.05-2.90 (m, 5H), 2.25-2.14 (m, 4H), 2.03-1.29 (m, 30H), 0.88-0.80 (m, 6H).
MS (ESI) m/z: 1164.55 [M + H]⁺

### (1-6-3) Synthesis of Compound (21)

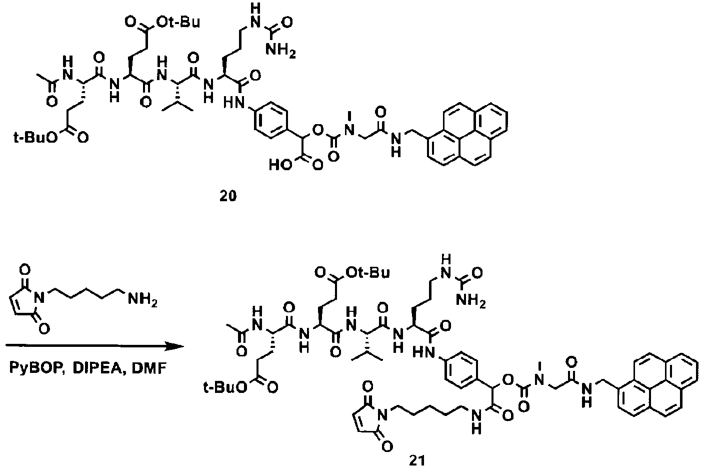

Compound (20) (14.7 mg, 12.6 µmol) obtained in Example (1-6-2) was dissolved in N,N-dimethylformamide (1.0 mL) and cooled on ice, then N,N-diisopropylethylamine (4.29 µL,25.2 µmol) and 1H-benzotriazole-1-yloxytripyrrolidinophosphonium hexafluorophosphate (9.8 mg,19 µmol) were added. Next, N-(5-aminopentyl)maleimide hydrochloride (4.1 mg, 19 µmol) was added, and the mixture was allowed to warm to room temperature and stirred for 3.5 hours. After the reaction was completed, the reaction mixture was purified by preparative reversed-phase chromatography. Fractions containing the product were collected, and the acetonitrile was removed by concentration under reduced pressure, followed by lyophilization to obtain Compound (21) (7.0 mg,9.2 µmol).

¹H NMR (400 MHz, DMSO-d₆) δ10.08-10.05 (m, 1H), 8.92-7.95 (m, 15H), 7.75-7.73 (m, 1H), 7.63-7.59 (m, 2H), 7.40-7.34 (m, 2H), 6.97-6.94 (m, 2H), 6.00-5.98 (m, 1H), 5.70-5.69 (m, 1H), 5.43 (brs, 2H), 5.14-5.01 (m, 2H), 4.45-4.38 (m, 1H), 4.32-3.83 (m, 5H), 3.25-3.20 (m, 1H), 3.10-2.90 (m, 7H), 2.25-2.18 (m, 4H), 2.08-1.24 (m, 34H), 1.12-1.04 (m, 2H), 0.88-0.81 (m, 6H).
MS (ESI) m/z: 1328.60 [M + H]⁺

### (1-6-4) Synthesis of Payload mimic-Linker (P6)

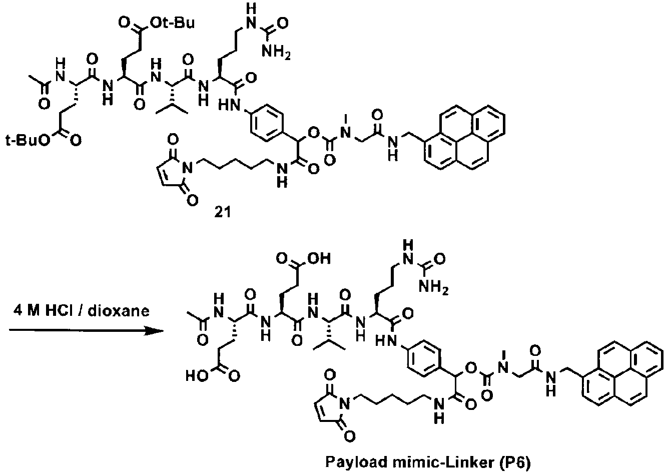

To Compound (21) (6.1 mg, 4.6 µmol) obtained in Example (1-6-3), 1,4-dioxane (920 µL) and 4 M HCl in dioxane (230 µL, 918 µmol) were sequentially added, and the mixture was stirred at room temperature for 4 hours. After cooling on ice, N,N-diisopropylethylamine (172 µL, 1.10 mmol) was added, and the mixture was stirred at room temperature for 10 minutes. The reaction solution was purified by preparative reversed-phase chromatography, and fractions containing the product were collected, and the acetonitrile was removed by concentration under reduced pressure, followed by lyophilization to obtain Payload mimic-Linker (P6) (3.7 mg, 3.0 µmol).

¹H NMR (400 MHz, DMSO-d₆) δ12.04 (brs, 2H), 10.01-9.98 (m, 1H), 8.83-7.89 (m, 15H), 7.71-7.69 (m, 1H), 7.56-7.51 (m, 2H), 7.33-7.26 (m, 2H), 6.90-6.87 (m, 2H), 5.91-5.90 (m, 1H), 5.64-5.62 (m, 1H), 5.36 (brs, 2H), 5.08-4.92 (m, 2H), 4.35-4.32 (m, 1H), 4.25-3.76 (m, 5H), 3.18-3.14 (m, 1H), 2.99-2.82 (m, 7H), 2.21-2.15 (m, 4H), 1.93-1.17 (m, 16H), 1.05-1.01 (m, 2H), 0.82-0.74 (m, 6H).
MS (ESI) m/z: 1216.45 [M + H]⁺

### Example 2: Synthesis of ADC and ADC mimic of DAR = 10

### (2-1) Synthesis of antibody with two peptides bound (T-1)

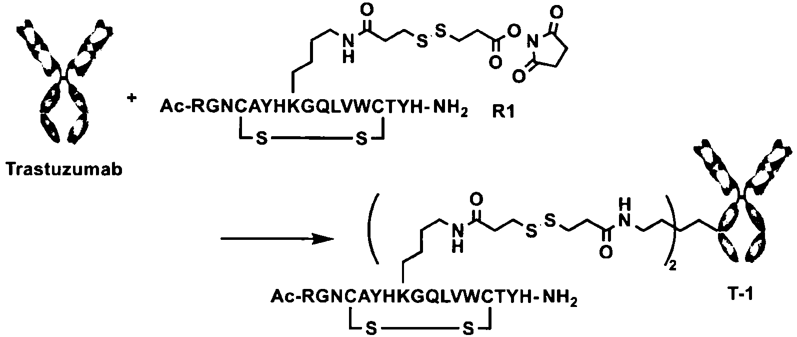

(The SEQ ID NO of the above-described amino acid sequence is SEQ ID NO: 1)

To a 10 mg/mL solution of an anti-human HER2 monoclonal antibody Trastuzumab (Chugai Pharmaceutical Co., Ltd.) in acetate buffer (50 mM Sodium acetate, pH5.5), a solution of a peptide reagent (R1) described in the previous report (WO2018/199337A1) in dimethylformamide (8 equivalents relative to the antibody, 8% v/v dimethylformamide (DMF) solution) was added, and the mixture was stirred at room temperature for one hour. The reaction solution was purified using a NAP-25 desalting column (Cytiva) to obtain an antibody with two peptide reagents introduced (T-1). HIC-HPLC analysis was performed according to the previous report (Anal. Chem., 2019, 91, 20, 12724-12732) to confirm that two peptide reagents were introduced.

(2-2) Synthesis of antibody having 10 molecules of thiol group (T-2) 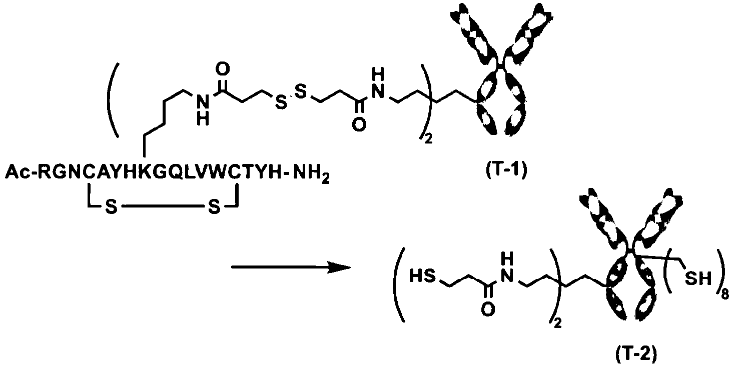 (The SEQ ID NO of the above-described amino acid sequence is SEQ ID NO: 1)

To a 5 mg/mL solution of the antibody with two peptides bound (T-2) synthesized in Example (2-1) in PBSE buffer (10 mM Phosphate Buffered Saline (PBS), 10 mM EDTA, pH7.4), 15 equivalents of a 20 mM solution of a reducing agent (TCEP) in PBSE relative to the antibody were added, and the mixture was agitated at 37°C for 1.5 hours. The reaction solution was purified using a NAP-25 desalting column (Cytiva) to obtain an antibody having 10 molecules of thiol group (T-2).

### (2-3) Synthesis of ADC with DAR of 10 (T-3)

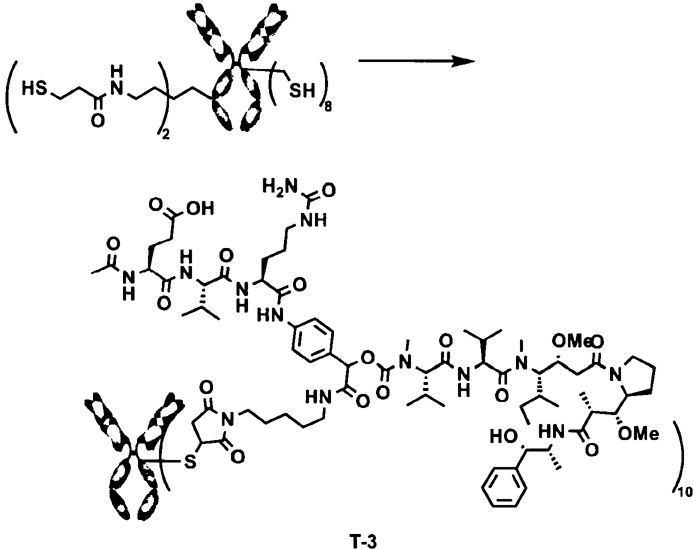

To a 5 mg/mL solution of the antibody having 10 molecules of thiol group (T-2) synthesized in Example (2-2) in PBSE buffer (10 mM Phosphate Buffered Saline (PBS), 10 mM EDTA, pH7.4), 15 equivalents of a 20 mM solution of a reducing agent (TCEP) in PBSE relative to the antibody were added, and the mixture was agitated at 37°C for 30 minutes. To the reaction solution, a solution of Payload-Linker (P1) synthesized in Example (1-1) in dimethylacetamide (20 equivalents relative to the antibody, 8% v/v dimethylacetamide (DMA) solution) was added, and the mixture was agitated at room temperature for 30 minutes. The reaction solution was purified using a NAP-25 desalting column (Cytiva) to obtain an antibody with 10 molecules of Payload-Linker (P1) introduced (T-3). ESI-TOFMS analysis was performed according to the previous report (WO2019/240287A1) to observe heavy chain peaks at 56691 and 56853 and a light chain peak at 24941, confirming that 10 molecules of Payload-Linker (P1) were introduced.

### (2-4) Synthesis of ADC with DAR of 10 (T-4)

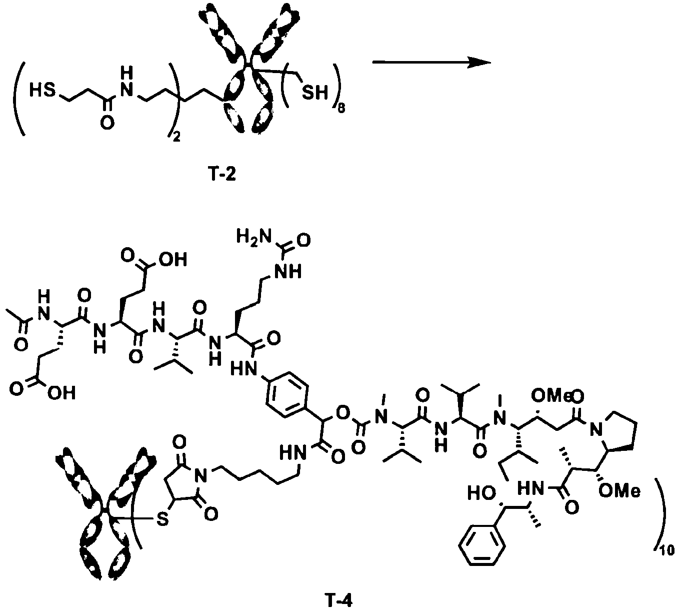

According to Example (2-3), the antibody having 10 molecules of thiol group (T-2) obtained in Example (2-2) and Payload-Linker (P2) were used to obtain an antibody with 10 molecules of Payload-Linker (P2) introduced (T-4). ESI-TOFMS analysis was performed according to the previous report (WO2019/240287A1) to observe heavy chain peaks at 57208 and 57370 and a light chain peak at 25071, confirming that 10 molecules of Payload-Linker (P2) were introduced.

### (2-5) Synthesis of ADC with DAR of 10 (T-5)

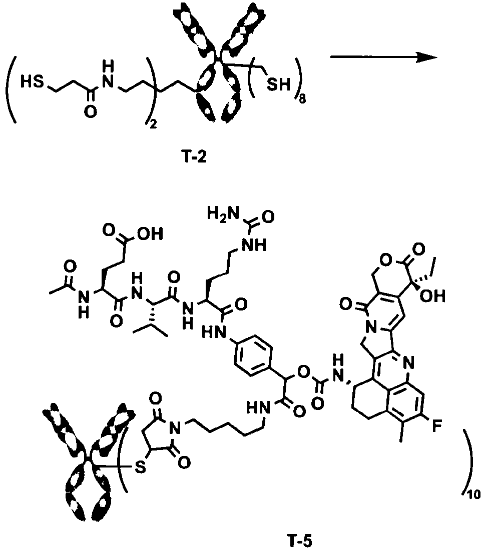

The antibody having 10 molecules of thiol group (T-2) obtained in Example (2-2) and Payload-Linker (P4) were used according to Example (2-3) to obtain an antibody with 10 molecules of Payload-Linker (P4) introduced (T-5). ESI-TOFMS analysis was performed according to the previous report (WO2019/240287A1) to observe heavy chain peaks at 55564 and 55725 and a light chain peak at 24660, confirming that 10 molecules of Payload-Linker (P4) were introduced.

### (2-6) Synthesis of ADC with DAR of 10 (T-6)

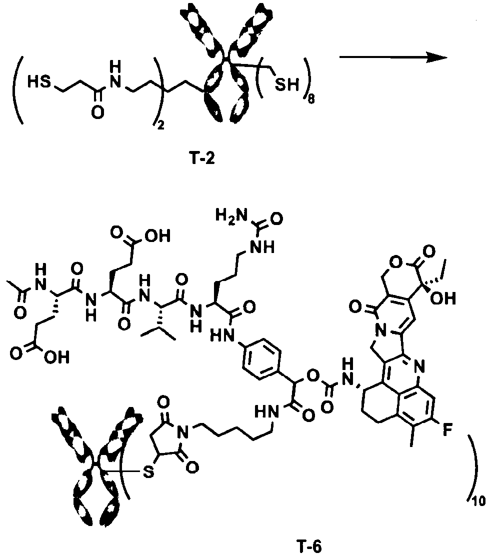

According to Example (2-3), the antibody having 10 molecules of thiol group (T-2) obtained in Example (2-2) and Payload-Linker (P3) were used to obtain an antibody with 10 molecules of Payload-Linker (P3) introduced (T-6). ESI-TOFMS analysis was performed according to the previous report (WO2019/240287A1) to observe heavy chain peaks at 56081 and 56243 and a light chain peak at 24788, confirming that 10 molecules of Payload-Linker (P3) were introduced.

### (2-7) Synthesis of ADC mimic with DAR of 10 (T-7)

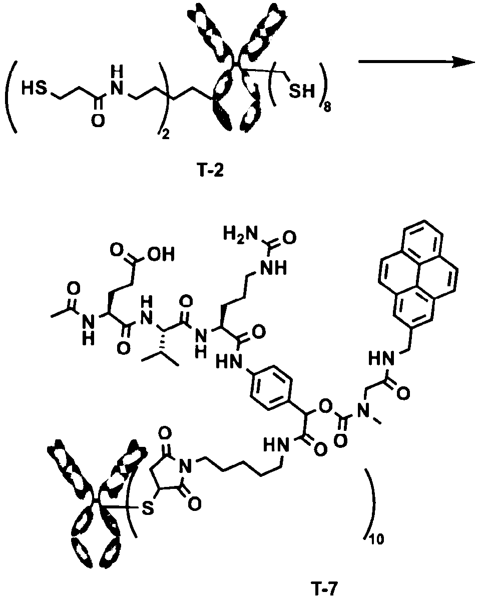

According to Example (2-3), the antibody having 10 molecules of thiol group (T-2) obtained in Example (2-2) and Payload mimic-Linker (P5) were used to obtain an antibody with 10 molecules of Payload-Linker (P5) introduced (T-7). ESI-TOFMS analysis was performed according to the previous report (WO2019/240287A1) to observe heavy chain peaks at 55029 and 55191 and a light chain peak at 24526, confirming that 10 molecules of Payload mimic-Linker (P5) were introduced.

### (2-8) Synthesis of ADC mimic with DAR of 10 (T-8)

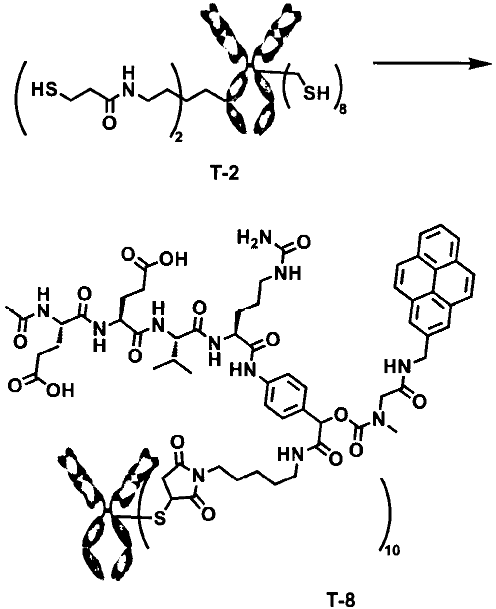

According to Example (2-3), the antibody having 10 molecules of thiol group (T-2) obtained in Example (2-2) and Payload mimic-Linker (P6) were used to obtain an antibody with 10 molecules of Payload-Linker (P6) introduced (T-8). ESI-TOFMS analysis was performed according to the previous report (WO2019/240287A1) to observe heavy chain peaks at 55549 and 55711 and a light chain peak at 24656, confirming that 10 molecules of Payload mimic-Linker (P6) were introduced.

### Comparative Example 1: Synthesis of Payload-Linker and Payload mimic-Linker

### (1-1) Synthesis of Payload-Linker (P7)

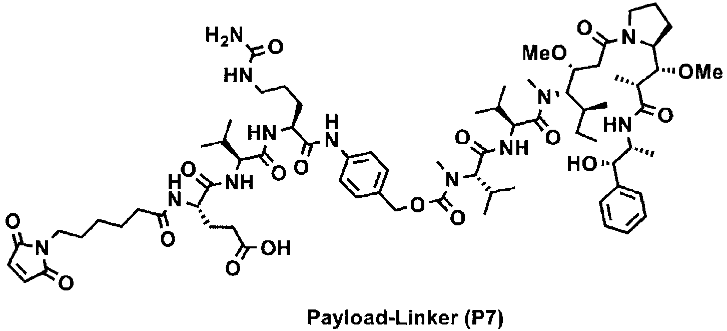

According to Example (1-1), Payload-Linker (P7) was synthesized using the known compound Glu-Val-Cit-PAB-MMAE (Organic & Biomolecular Chemistry, 2016,14,9501-9518).
MS (ESI) m/z: 1445.8 [M + H]⁺

### (1-2) Synthesis of Payload mimic-Linker (P8)

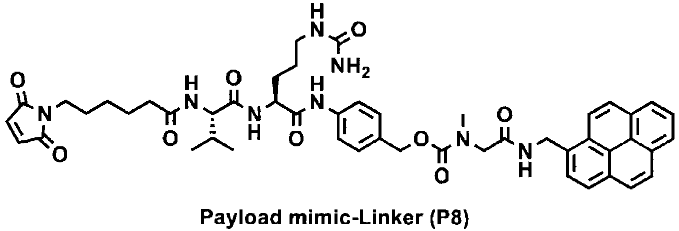

Payload mimic-Linker (P8) was synthesized as follows. Payload mimic-Linker (P8) was synthesized in one step from commercially available MC-VC-PABA-PNP (CAS No: 159857-81-5) and known Sarcosine-pyrene (WO2018218004A1).

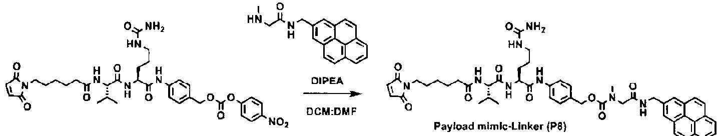

Commercially available MC-VC-PAB-PNP (CAS No: 159857-81-5) (15.5 mg, 0.021 mmol) was dissolved in dichloromethane (1 mL), and a solution of N,N-diisopropylethylamine (0.025 mL, 0.142 mmol) and known Sarcosine-Pyrene (WO2018218004A1) (7.6 mg, 0.025 mmol) in dimethylformamide (0.5 mL) was added, followed by stirring for 17 hours. After purification by preparative reversed-phase chromatography, fractions containing the product were collected, and the acetonitrile was removed by concentration under reduced pressure, followed by lyophilization to obtain Payload mimic-Linker (P8) (7.3 mg, 0.008 mmol).

¹H NMR (400 MHz, DMSO-d6) δ9.98 (s, 1H), 8.34 (d, J = 9.2 Hz, 2H), 8.32-8.23 (m, 4H), 8.16 (s, 2H), 8.10-8.00 (m, 4H), 7.80 (d, J = 8.8 Hz, 1H), 7.59 (d, J = 8.4 Hz, 2H), 7.31 (d, J = 8.0 Hz, 2H), 6.99 (s, 2H), 5.96 (m, 1H), 5.40 (s, 2H), 5.01 (s, 2H), 4.95 (d, J = 6.0 Hz, 2H), 4.38 (m, 1H), 4.19 (m, 1H), 3.03-2.92 (m, 3H), 2.67 (m, 1H), 2.33 (m, 1H), 2.20-2.07 (m, 2H), 1.97 (m, 1H), 1.67 (m, 1H), 1.59 (m, 1H), 1.51-1.45 (m, 6H), 1.26-1.15 (m, 3H), 0.83 (dd, J = 12.8, 6.8 Hz, 6H).
MS (ESI) m/z: 901.45 [M + H]⁺

### (1-3) Synthesis of Payload mimic-Linker (P9)

Payload mimic-Linker (P9) was synthesized as follows.

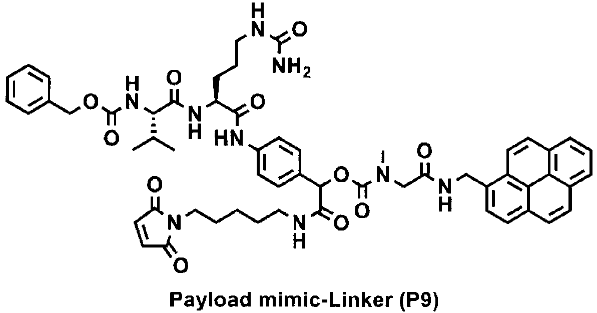

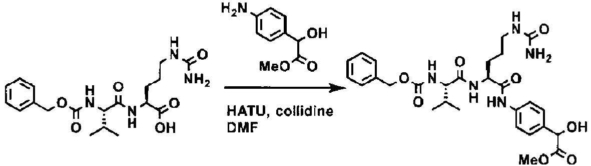

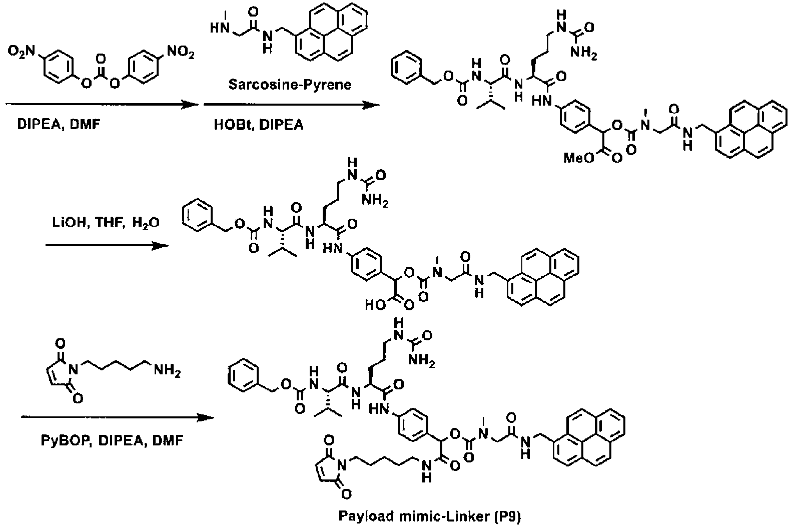

### MS (ESI) m/z: 1050.55 [M + H]⁺

Comparative Example 2: Synthesis of ADC and ADC mimic of DAR = 10 (2-1) Synthesis of ADC of DAR = 10 (T-9)

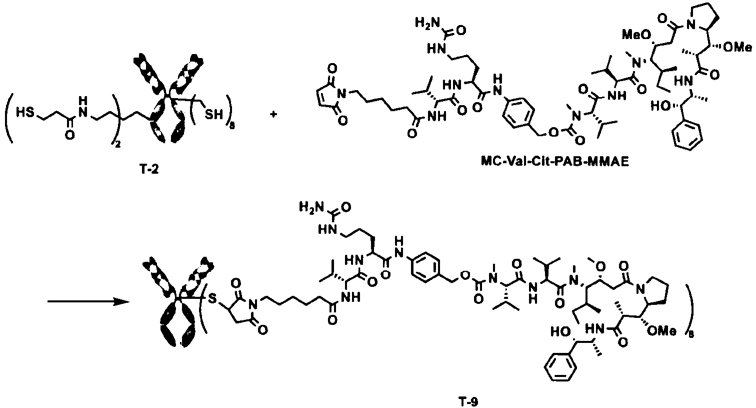

According to Example (2-3), the antibody having 10 molecules of thiol group (T-2) obtained in Example (2-2) and the known compound MC-Val-Cit-PABA-MMAE (Organic & Biomolecular Chemistry, 2016, 14, 9501-9518) were used to obtain an antibody with 10 molecules of Val-Cit-PABA-MMAE introduced (T-9). ESI-TOFMS analysis was performed according to the previous report (WO2019/240287A1) to observe heavy chain peaks at 55946 and 56109 and a light chain peak at 24755, confirming that 10 molecules of Val-Cit-PABA-MMAE were introduced.

### (2-2) Synthesis of ADC of DAR = 10 (T-10)

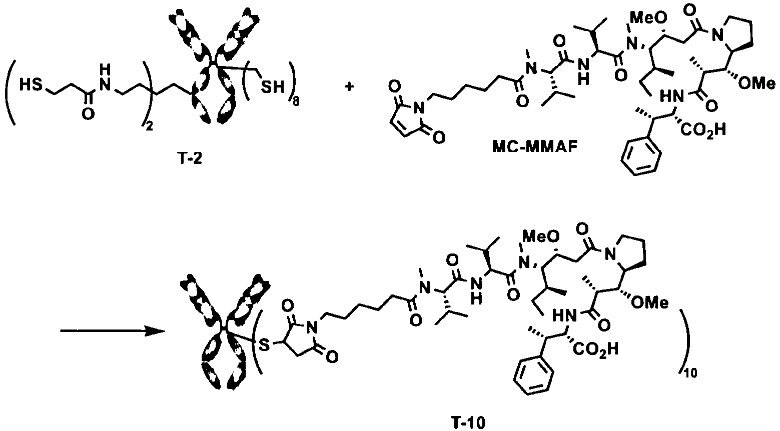

According to Example (2-3), the antibody having 10 molecules of thiol group (T-2) obtained in Example (2-2) and the known compound MC-MMAF were used to obtain an antibody with 10 molecules of Val-Cit-PABA-MMAE introduced (T-10). ESI-TOFMS analysis was performed according to the previous report (WO2019/240287A1) to observe heavy chain peaks at 54381 and 54543 and a light chain peak at 24364, confirming that 10 molecules of MMAF were introduced.

### (2-3) Synthesis of ADC of DAR = 10 (T-11)

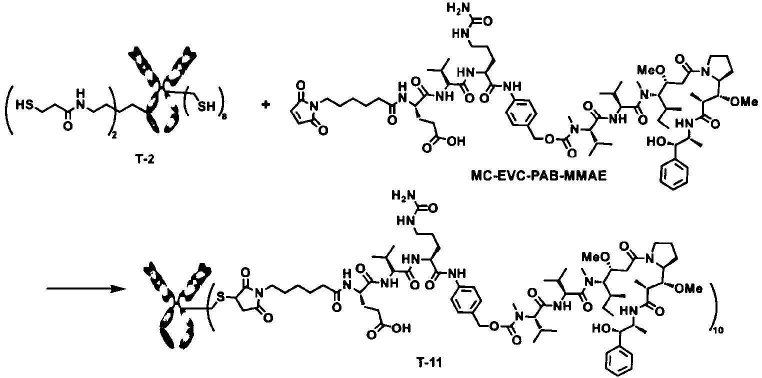

According to Example (2-3), the antibody having 10 molecules of thiol group (T-2) obtained in Example (2-2) and MC-Glu-Val-Cit-PAB-MMAE (P7) were used to obtain an antibody with 10 molecules of P7 introduced (T-11). ESI-TOFMS analysis was performed according to the previous report (WO2019/240287A1) to observe heavy chain peaks at 56463 and 56625 and a light chain peak at 24884, confirming that 10 molecules of P7 were introduced.

### (2-4) Synthesis of ADC of DAR = 10 (T-12)

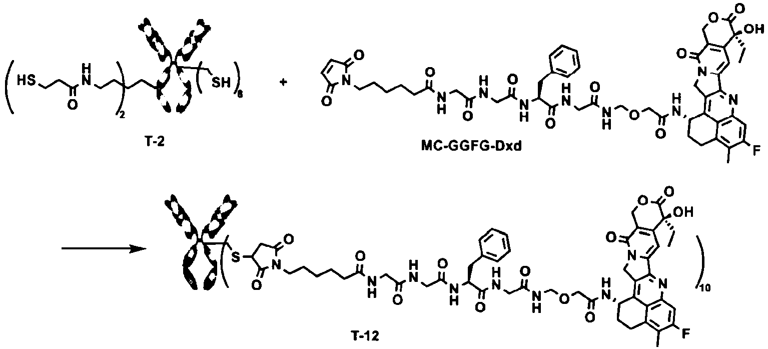

According to Example (2-3), the antibody having 10 molecules of thiol group (T-2) obtained in Example (2-2) and known MC-Gly-Gly-Phe-Gly-Dxd (Gly-Gly-Phe-Gly, SEQ ID NO: 4) were used to obtain an antibody with 10 molecules of Gly-Gly-Phe-Gly-Dxd (Gly-Gly-Phe-Gly, SEQ ID NO: 4) introduced (T-12). ESI-TOFMS analysis was performed according to the previous report (WO2019/240287A1) to observe heavy chain peaks at 54816 and 54978 and a light chain peak at 24473, confirming that 10 molecules of Gly-Gly-Phe-Gly-Dxd (Gly-Gly-Phe-Gly, SEQ ID NO: 4) were introduced.

### (2-5) Synthesis of ADC mimic of DAR = 10 (T-13)

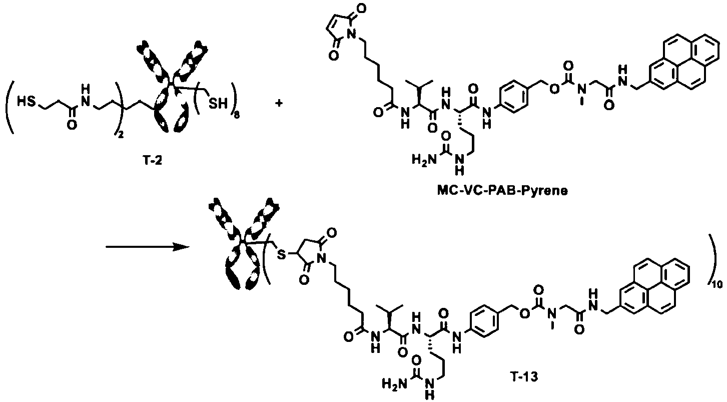

According to Example (2-3), the antibody having 10 molecules of thiol group (T-2) obtained in Example (2-2) and known MC-Val-Cit-PAB-Pyrene (P8) were used to obtain an antibody with 10 molecules of P8 introduced (T-13). Analysis was performed according to the previous report (Anal. Chem., 2019, 91, 20, 12724-12732) by HIC-HPLC, RP-HPLC, and QTOF-MS analysis to observe heavy chain peaks at 54287 and 54449 and a light chain peak at 24341, confirming that 10 molecules of P8 were introduced.

### (2-6) Synthesis of ADC mimic of DAR = 10 (T-14)

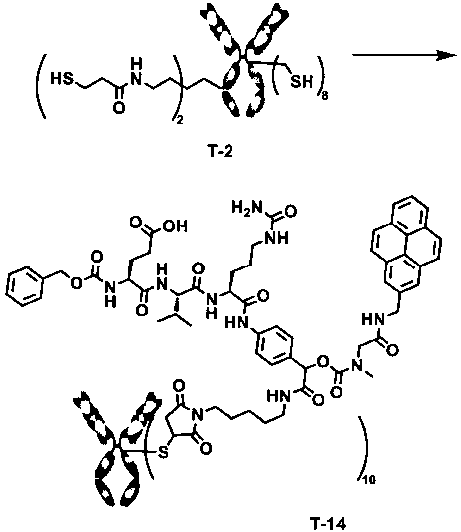

According to Example (2-3), the antibody having 10 molecules of thiol group (T-2) obtained in Example (2-2) and Payload mimic-Linker (P9) synthesized in Comparative Example (1-3) were used to obtain an antibody with 8 to 10 molecules of Payload-Linker (P9) introduced (T-14). ESI-TOFMS analysis was performed according to the previous report (WO2019/240287A1) to observe heavy chain peaks at 54884 and 555046 and a light chain peak at 24490, confirming that 10 molecules of Payload mimic-Linker (P9) were introduced.

Example 3: Evaluation of degree of hydrophobicity of ADC and ADC mimic by hydrophobic interaction chromatography (HIC-HPLC)
HIC-HPLC analysis was performed according to the previous report (Anal. Chem., 2019, 91, 20, 12724-12732). The measurement was performed using the following conditions. The degree of hydrophobicity of the ADC can be evaluated based on the retention time of the ADC in the HIC chromatogram.
Measurement system: 1260 HPLC system (Agilent Technologies, Inc.)
Column: Tosoh Biobuthyl NPR 2.5 µm 4.6 × 35 mm column manufactured by Tosoh Bioscience LLC
Gradient: Linear gradient of eluent A/B
Flow rate: 0.8 mL/min
Eluent A: 1.1 M (NH₄)₂SO₄, 25 mM Na₂HPO₄/NaH₂PO₄ (pH6.0)
Eluent B: 25 mM Na₂HPO₄/NaH₂PO₄ (pH6.0, 25% v/v isopropanol added)
Detector: UV (280 nm)

### (3-1) Evaluation of degree of hydrophobicity of T-3, T-4, T-9, and T-11 by hydrophobic interaction chromatography (HIC-HPLC)

The evaluation results of the degree of hydrophobicity of T-3, T-4, T-9, and T-11 by hydrophobic interaction chromatography (HIC-HPLC) are shown in Table 1-1.

**Table 1-1: Evaluation of degree of hydrophobicity of T-3, T-4, T-9, and T-11 using HIC-HPLC**

| | Linker-payload mimic / Linker-payload | Example / Comparable Example | Retention time (min) |
|---|---|---|---|
| T-3 | P1 | Example (2-3) | 15.4 |
| T-4 | P2 | Example (2-4) | 14.3 |
| T-9 | MC-Val-Cit-PAB-MMAE | Comparative Example (2-1) | 18.4 |
| T-11 | P7 | Comparative Example (2-3) | 18.0 |

The ADCs (T-3 and T-4) to which Payload-Linker having a hydrophilic group at the exo-position is conjugated, synthesized in Examples (2-3) and (2-4), were confirmed to have a tendency for earlier retention times compared to the ADC to which endo-type Payload-Linker is conjugated (T-9) synthesized in Comparative Example (2-1) and the ADC to which Payload-Linker having a hydrophilic group at the endo-position is conjugated (T-11) synthesized in Comparative Example (2-3), indicating high degree of hydrophilicity. Therefore, it was confirmed that the Payload-Linker ADCs having a hydrophilic group at the exo-position (T-3 and T-4) synthesized in Example (2-3) and (2-4) are preferable ADCs because they are considered to have slow plasma clearance and a long residence time in the body.

### (3-2) Evaluation of degree of hydrophobicity of T-5, T-6, and T-12 by hydrophobic interaction chromatography (HIC-HPLC)

The evaluation results of the degree of hydrophobicity of T-5, T-6, and T-12 by hydrophobic interaction chromatography (HIC-HPLC) are shown in Table 1-2.

**Table 1-2: Evaluation of degree of hydrophobicity of T-5, T-6, and T-12 using HIC-HPLC**

| | Linker-payload mimic / Linker-payload | Example / Comparable Example | Retention time (min) |
|---|---|---|---|
| T-5 | P4 | Example 2-5 | 8.9 |
| T-6 | P3 | Example 2-6 | 8.5 |
| T-12 | MC-Gly-Gly-Phe-Gly-Dxd (Gly-Gly-Phe-Gly, SEQ ID NO: 4) | Comparative Example 2-4 | 9.8 |

The ADCs (T-5 and T-6) to which Payload-Linker having a hydrophilic group at the exo-position is conjugated, synthesized in Examples (2-5) and (2-6), were confirmed to have a tendency for earlier retention times compared to the ADC to which endo-type Payload-Linker is conjugated (T-12) synthesized in Comparative Example (2-4), indicating high degree of hydrophilicity. Therefore, it was confirmed that the ADCs to which Payload-Linker having a hydrophilic group at the exo-position is conjugated (T-5 and T-6) synthesized in Example (2-5) and (2-6) are preferable ADCs because they are considered to have slow plasma clearance and a long residence time in the body.

### (3-3) Evaluation of degree of hydrophobicity of T-7, T-8, T-13, and T-14 by hydrophobic interaction chromatography (HIC-HPLC)

The evaluation results of the degree of hydrophobicity of T-7, T-8, T-13, and T-14 by hydrophobic interaction chromatography (HIC-HPLC) are shown in Table 1-3.

**Table 1-3: Evaluation of degree of hydrophobicity of T-7 T-8, T-13, and T-14 using HIC-HPLC**

| | Linker-payload mimic / Linker-payload | Example / Comparable Example | Retention time (min) |
|---|---|---|---|
| T-7 | P5 | Example (2-7) | 13.3 |
| T-8 | P6 | Example (2-8) | 12.4 |
| T-13 | P8 | Comparative Example (2-5) | 14.7 |
| T-14 | P9 | Comparative Example (2-6) | 16.5 |

The ADC mimics (T-7 and T-8) to which Payload mimic-Linker having a hydrophilic group at the exo-position is conjugated, synthesized in Examples (2-7) and (2-8), were confirmed to have a tendency for earlier retention times compared to the ADC mimic to which endo-type Payload mimic-Linker is conjugated (T-13) synthesized in Comparative Example (2-5) and the ADC mimic to which Payload mimic-Linker having no hydrophilicity at the endo-position is conjugated (T-14) synthesized in Comparative Example (2-6), indicating high degree of hydrophilicity. Therefore, it was confirmed that the ADC mimics to which Payload mimic-Linker having a hydrophilic group at the Exo-position is conjugated (T-7 and T-8) synthesized in Example (2-7) and (2-8) are preferable ADCs because they are considered to have slow plasma clearance and a long residence time in the body.

### Example 4: Evaluation of aggregation rate of ADC and ADC mimic by size exclusion chromatography (SEC-HPLC)

SEC-HPLC analysis was performed according to the previous report (ChemistrySelect, 2020, 5, 8435-8439). The measurement was performed using the following conditions.
Measurement system: 1260 HPLC system (Agilent Technologies, Inc.)
Column: AdvanceBio SEC 300Å 2.7 µm, 4.6 mm × 300 mm manufactured by Agilent Technologies, Inc.
Flow rate: 0.25 mL/min
Eluent: 100 mM sodium dihydrogen phosphate/sodium hydrogen phosphate, 250 mM aqueous sodium chloride solution (pH 6.8), 10 %v/v isopropanol
Detector: UV (280 nm)

(4-1) Evaluation of aggregation rate of T-3, T-4, and T-9 by size exclusion chromatography (SEC-HPLC)
The evaluation results of the aggregation rate of T-3, T-4, and T-9 by size exclusion chromatography (SEC-HPLC) are shown in Table 2-1.

**Table 2-1: Evaluation of degree of hydrophobicity of T-3, T-4, and T-9 using SEC-HPLC**

| | Linker-payload mimic / Linker-payload | Example / Comparable Example | Aggregation rate (%) |
|---|---|---|---|
| T-3 | P1 | Example (2-3) | 0.6 |
| T-4 | P2 | Example (2-4) | 0.5 |
| T-9 | MC-Val-Cit-PAB-MMAE | Comparative Example (2-1) | 3.64 |
| T-11 | P7 | Comparative Example (2-3) | 1.42 |

The ADCs to which Payload-Linker having a hydrophilic group at the exo-position is conjugated (T-3 and T-4) synthesized in Examples (2-3) and (2-4) were confirmed to have a tendency for lower aggregation rates compared to the ADC to which endo-type Payload-Linker is conjugated (T-9) synthesized in Comparative Example (2-1) and the ADC to which Payload-Linker having a hydrophilic group at the endo-position is conjugated (T-11) synthesized in Comparative Example (2-3).

(4-2) Evaluation of aggregation rate of T-7, T-8, T-13, and T-14 by size exclusion chromatography (SEC-HPLC)
The evaluation results of the aggregation rate of T-7, T-8, T-13, and T-14 by size exclusion chromatography (SEC-HPLC) are shown in Table 2-2.

**Table 2-2: Evaluation of degree of hydrophobicity of T-7, T-8, T-13, and T-14 using SEC-HPLC**

| | Linker-payload mimic / Linker-payload | Example / Comparable Example | Aggregation rate (%) |
|---|---|---|---|
| T-7 | P5 | Example (2-7) | 0.9 |
| T-8 | P6 | Example (2-8) | 1.1 |
| T-13 | P8 | Comparative Example (2-5) | 30 |
| T-14 | P9 | Comparative Example (2-6) | 21 |

The ADC mimics (T-7 and T-8) to which Payload mimic-Linker having a hydrophilic group at the exo-position is conjugated, synthesized in Examples (2-7) and (2-8), were confirmed to have a tendency for lower aggregation rate compared to the ADC mimic to which endo-type Payload mimic-Linker is conjugated (T-13) and the ADC mimic to which Payload mimic-Linker having no hydrophilicity at the exo-position is conjugated (T-14) synthesized in Comparative Example (2-6).

### Example 5: Synthesis of antibody with two molecules of thiol group bound to Lys288/290 of the antibody (T16)

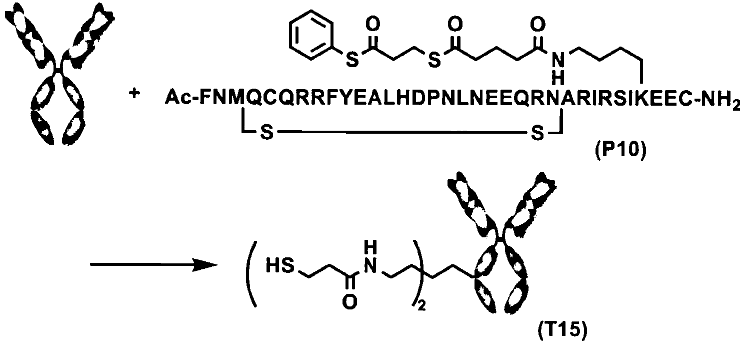

(The SEQ ID NO of the above-described amino acid sequence is SEQ ID NO: 2)

An anti-human HER2 monoclonal antibody Trastuzumab (Chugai Pharmaceutical Co., Ltd.) and a peptide reagent (P10) described in the previous report (WO2022/164116A1) were used following the method described in the previous report (WO2022/164116A1) to obtain an antibody with two molecules of thiol group introduced. HIC-HPLC analysis was performed according to the previous report (Anal. Chem., 2019, 91, 20, 12724-12732) to confirm that two molecules of thiol group were introduced into Lys288/290 of the antibody.

### Example 6: Synthesis of ADC with DAR of 2 + 8

### (6-1) Synthesis of antibody with two molecules of thiol group bound to Lys246/248 of the antibody (T2)

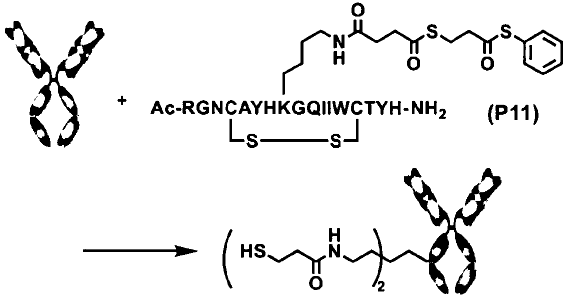

(The SEQ ID NO of the above-described amino acid sequence is SEQ ID NO: 3)

An anti-human HER2 monoclonal antibody Trastuzumab (Chugai Pharmaceutical Co., Ltd.) and a peptide reagent (P11) described in the previous report (WO2019/240287A1) were used following the method described in the previous report (WO2019/240287A1) to obtain an antibody with two molecules of thiol group introduced. HIC-HPLC analysis was performed according to the previous report (Anal. Chem., 2019, 91, 20, 12724-12732) to confirm that two molecules of thiol group were introduced into Lys246/248 of the antibody.

### (6-2) Synthesis of ADC with DAR of 2

### (6-2-1) Synthesis of ADC with DAR of 2 (N-1)

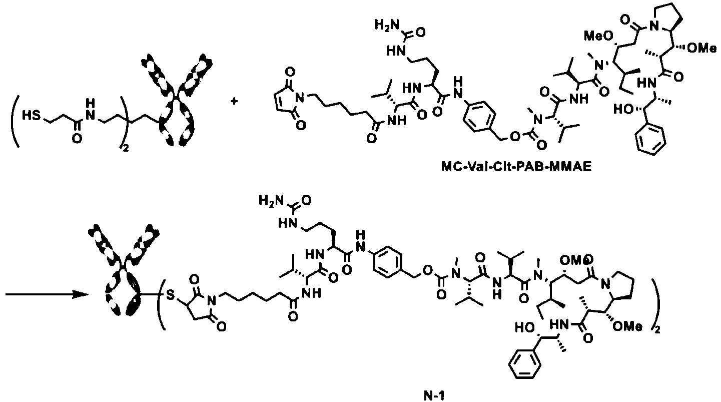

To a 4 mg/mL solution of the antibody with two molecules of thiol group introduced synthesized in Example (6-1) in PBSE buffer (10 mM Phosphate Buffered Saline (PBS), 10 mM EDTA, pH7.4), a solution of a known compound MC-Val-Cit-PABA-MMAE (Organic & Biomolecular Chemistry, 2016, 14, 9501-9518) in DMA (5 equivalents relative to the antibody, 8% v/v dimethylacetamide (DMA) solution) was added, and the mixture was agitated at room temperature for one hour. The final mixture was purified using a NAP-10 desalting column (Cytiva) to obtain an ADC with DAR of 2 (N-1). ESI-TOFMS analysis was performed according to the previous report (WO2019/240287A1) to observe a peak at 151028, confirming that two molecules of Val-Cit-PABA-MMAE were introduced.

### (6-2-2) Synthesis of ADC with DAR of 2 (N-2)

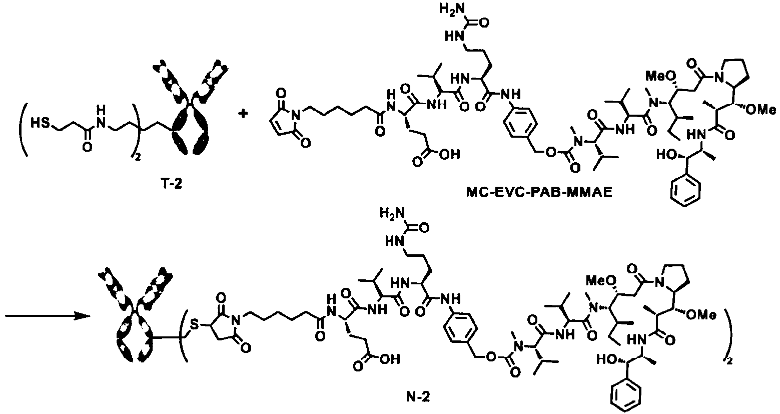

According to Example (6-2-1), the antibody having two molecules of thiol group obtained in Example (6-1) and MC-Glu-Val-Cit-PAB-MMAE (P7) synthesized in Comparative Example (1-1) were used to obtain an ADC with two molecules of P7 introduced (N-2). ESI-TOFMS analysis was performed according to the previous report (WO2019/240287A1) to observe a peak at 151290, confirming that two molecules of P7 were introduced.

### (6-2-3) Synthesis of ADC with DAR of 2 (N-3)

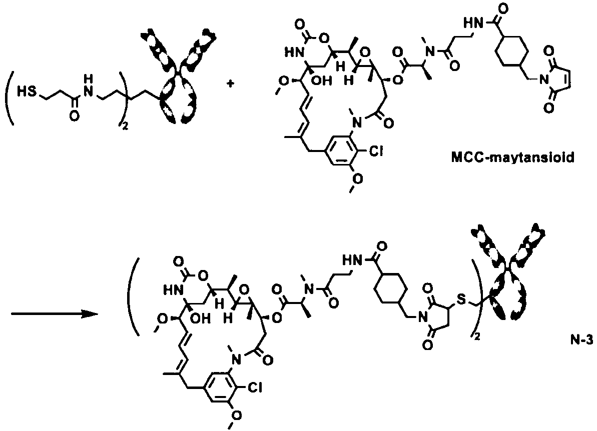

According to Example (6-2-1), the antibody having two molecules of thiol group obtained in Example (6-1) and a known compound MCC-maytansinoid were used to obtain an ADC (N-3). ESI-TOFMS analysis was performed according to the previous report (WO2019/240287A1) to observe a peak at 150278, confirming that two molecules of maytansinoid were introduced.

### (6-2-4) Synthesis of ADC with DAR of 2 (N-4)

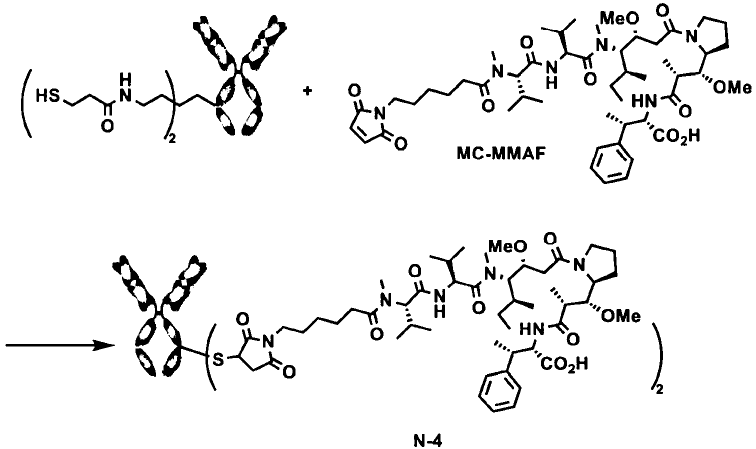

According to Example (6-2-1), the antibody having two molecules of thiol group obtained in Example (6-1) and a known compound MC-MMAF were used to obtain an ADC (N-4). ESI-TOFMS analysis was performed according to the previous report (WO2019/240287A1) to observe a peak at 150243, confirming that two molecules of MMAF were introduced.

### (6-3) Synthesis of ADC and ADC mimic with DAR of 2 + 8

### (6-3-1) Synthesis of ADC with DAR of 2 + 8 (N-5)

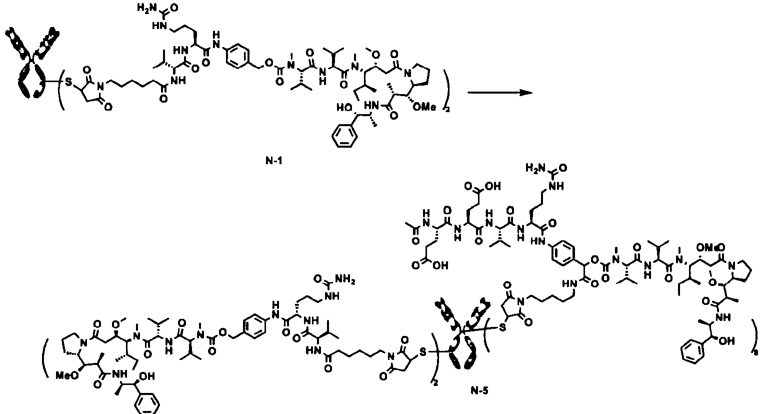

To a 5 mg/mL solution of the ADC (N-1) synthesized in Example (6-2-1) in PBSE buffer (10 mM Phosphate Buffered Saline (PBS), 10 mM EDTA, pH7.4), 15 equivalents of a 20 mM solution of a reducing agent (TCEP) in PBSE relative to the antibody were added, and the mixture was agitated at 37°C for 1.5 hours. To the reaction solution, a solution of Payload-Linker (P2) synthesized in Example (1-2) in DMA (20 equivalents relative to the antibody, 8% v/v dimethylacetamide (DMA) solution) was added, and the mixture was agitated at room temperature for one hour. The final mixture was purified using a NAP-10 desalting column (Cytiva) to obtain an ADC with DAR of 8 (N-5). ESI-TOFMS analysis was performed according to the previous report (WO2019/240287A1) to observe heavy chain peaks at 56895 and 57056 and a light chain peak at 25072, confirming that 10 molecules of Payload were introduced.

### (6-3-2) Synthesis of ADC with DAR of 2 + 8 (N-6)

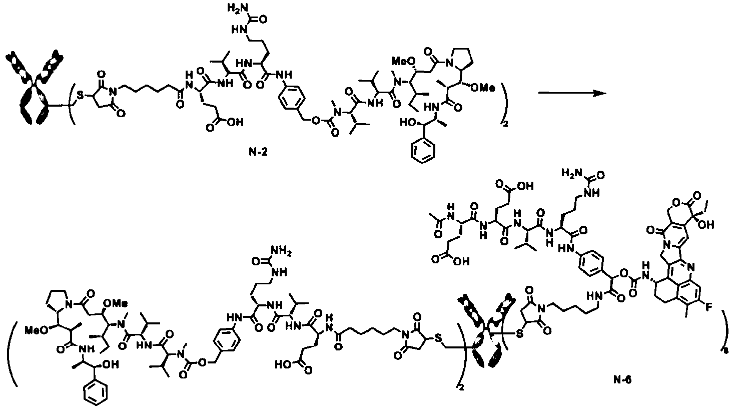

According to Example (6-3-1), ADC (N-2) synthesized in Example (6-2-2) and Payload-Linker (P3) synthesized in Example (1-3) were used to obtain an ADC with DAR of 2 + 8 (N-6). ESI-TOFMS analysis was performed according to the previous report (WO2019/240287A1) to observe heavy chain peaks at 56177 and 56338 and a light chain peak at 24789, confirming that 10 molecules of Payload were introduced.

### (6-3-3) Synthesis of ADC with DAR of 2 + 8 (N-7)

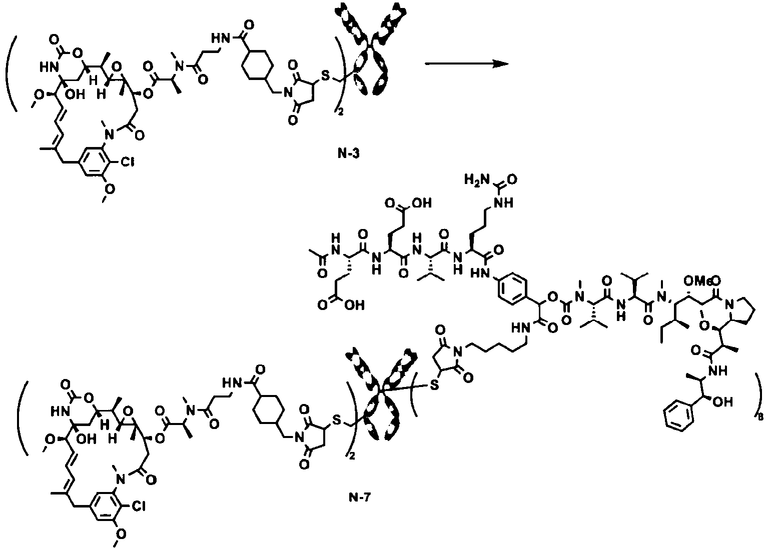

According to Example (6-3-1), ADC (N-3) synthesized in Example (6-2-3) and Payload-Linker (P2) synthesized in Example (1-2) were used to obtain an ADC with DAR of 2 + 8 (N-7). ESI-TOFMS analysis was performed according to the previous report (WO2019/240287A1) to observe heavy chain peaks at 56519 and 56680 and a light chain peak at 25072, confirming that 10 molecules of Payload were introduced.

### (6-3-4) Synthesis of ADC with DAR of 2 + 8 (N-8)

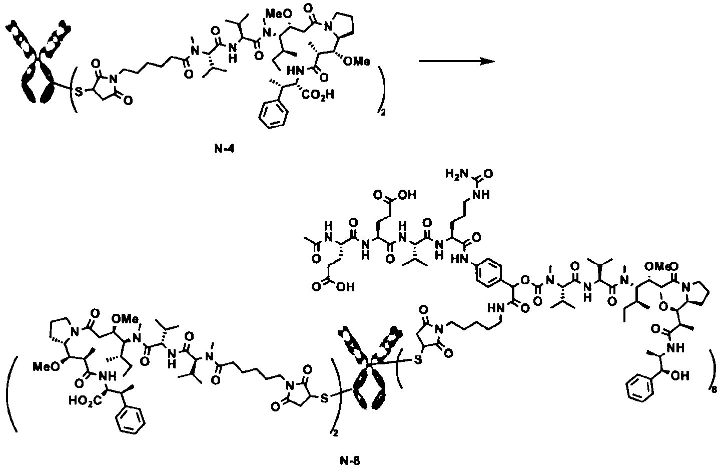

According to Example (6-3-1), ADC (N-4) synthesized in Example (6-2-4) and Payload-Linker (P2) synthesized in Example (1-2) were used to obtain an ADC with DAR of 2 + 8 (N-8). ESI-TOFMS analysis was performed according to the previous report (WO2019/240287A1) to observe heavy chain peaks at 56504 and 56665 and a light chain peak at 25072, confirming that 10 molecules of Payload were introduced.

### (6-3-5) Synthesis of ADC with DAR of 2 + 8 mimic (N-9)

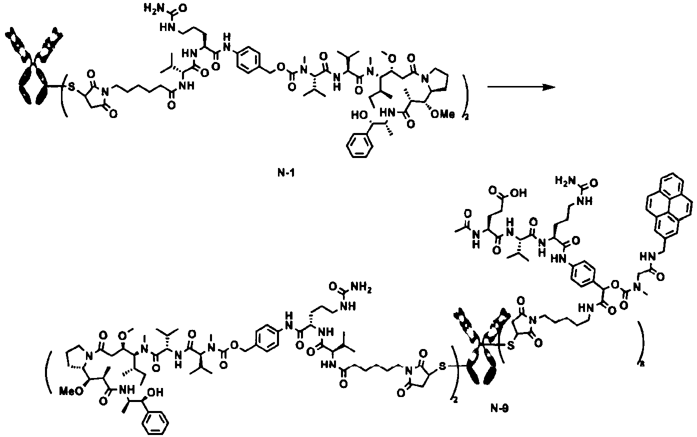

According to Example (6-3-1), ADC (N-1) synthesized in Example (6-2-1) and Payload-Linker (P5) synthesized in Example (1-5) were used to obtain an ADC mimic with DAR of 2 + 8 (N-9). ESI-TOFMS analysis was performed according to the previous report (WO2019/240287A1) to observe heavy chain peaks at 55260 and 55421 and a light chain peak at 24527, confirming that 10 molecules of Payload/Payload mimic were introduced.

### (6-3-6) Synthesis of ADC with DAR of 2 + 8 mimic (N-10)

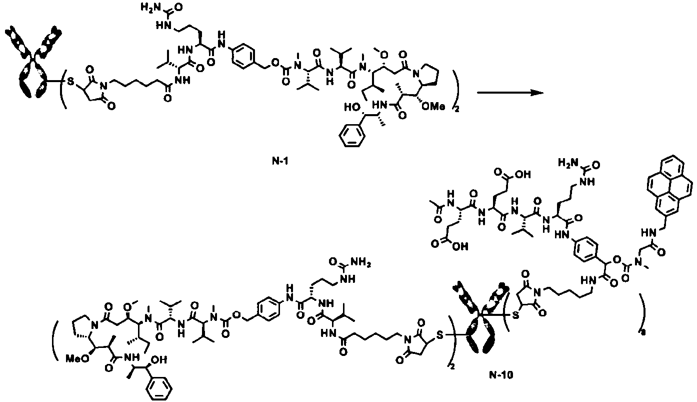

According to Example (6-3-1), ADC (N-1) synthesized in Example (6-2-1) and Payload-Linker (P6) synthesized in Example (1-6) were used to obtain an ADC mimic with DAR of 2 + 8 (N-10). ESI-TOFMS analysis was performed according to the previous report (WO2019/240287A1) to observe heavy chain peaks at 55649 and 55810 and a light chain peak at 24656, confirming that 10 molecules of Payload/Payload mimic were introduced.

### Comparative Example 3: Synthesis of ADC and ADC mimic with DAR of 2 + 8

### (3-1) Synthesis of ADC with DAR of 2 + 8 (N-11)

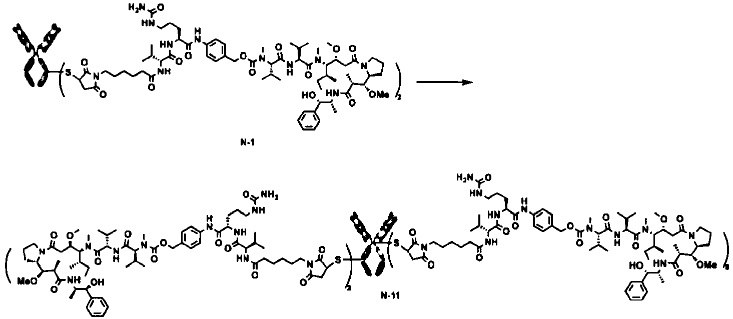

According to Example (6-3-1), ADC (N-1) synthesized in Example (6-2-1) and a known compound MC-Val-Cit-PABA-MMAE were used to obtain an ADC with DAR of 2 + 8 (N-11). ESI-TOFMS analysis was performed according to the previous report (WO2019/240287A1) to observe heavy chain peaks at 55949 and 56111 and a light chain peak at 24756, confirming that 10 molecules of Payload were introduced.

### (3-2) Synthesis of ADC with DAR of 2 + 8 (N-12)

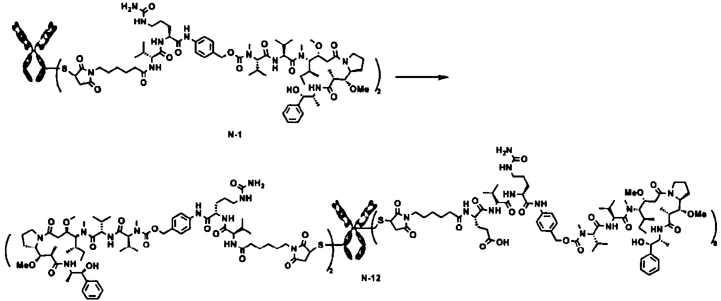

According to Example (6-3-1), ADC (N-1) synthesized in Example (6-2-1) and a known compound MC-Glu-Val-Cit-PABA-MMAE were used to obtain an ADC with DAR of 2 + 8 (N-12). ESI-TOFMS analysis was performed according to the previous report (WO2019/240287A1) to observe heavy chain peaks at 56337 and 56498 and a light chain peak at 24886, confirming that 10 molecules of Payload were introduced.

### (3-3) Synthesis of ADC with DAR of 2 + 8 (N-13)

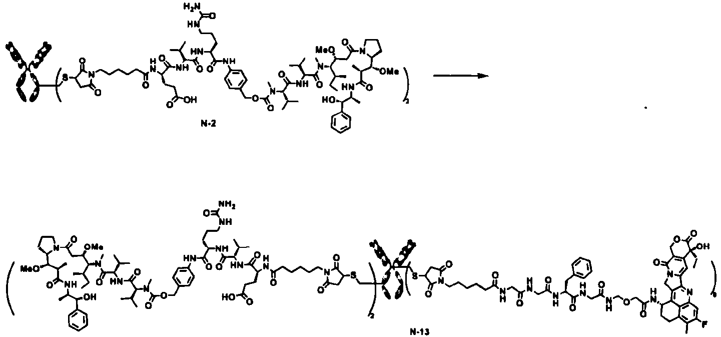

According to Example (6-3-1), ADC (N-2) synthesized in Example (6-2-2) and known MC-Gly-Gly-Phe-Gly-Dxd (Gly-Gly-Phe-Gly, SEQ ID NO: 4) were used to obtain an ADC with DAR of 2 + 8 (N-13). ESI-TOFMS analysis was performed according to the previous report (WO2019/240287A1) to observe heavy chain peaks at 55102 and 55263 and a light chain peak at 24474, confirming that 10 molecules of Payload were introduced.

### (3-4) Synthesis of ADC with DAR of 2 + 8 (N-14)

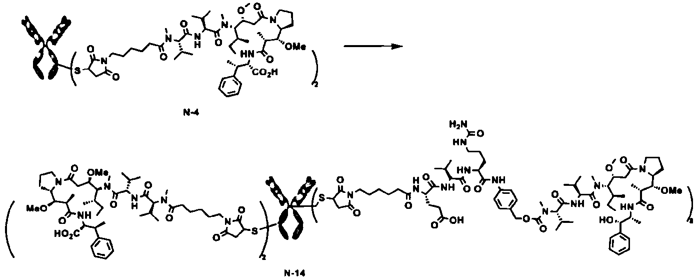

According to Example (6-3-1), ADC (N-4) synthesized in Example (6-2-4) and known MC-Glu-Val-Cit-PABA-MMAE were used to obtain an ADC with DAR of 2 + 8 (N-14). ESI-TOFMS analysis was performed according to the previous report (WO2019/240287A1) to observe heavy chain peaks at 55946 and 56107 and a light chain peak at 24885, confirming that 10 molecules of Payload were introduced.

### (3-5) Synthesis of ADC with DAR of 2 + 8 mimic (N-15)

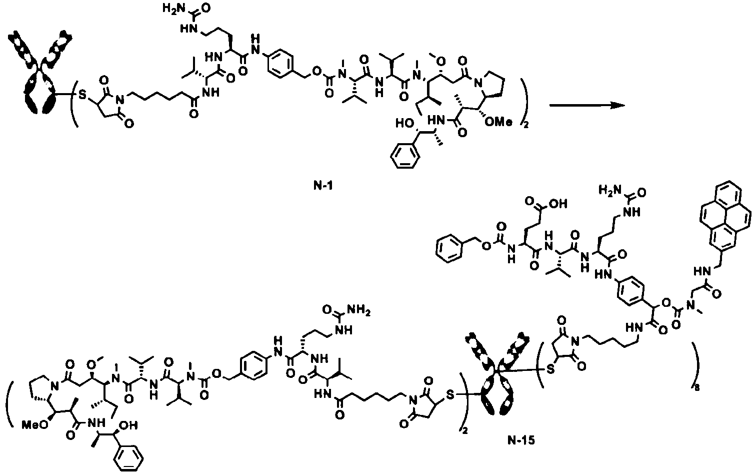

According to Example (6-3-1), ADC (N-1) synthesized in Example (6-2-1) and Payload mimic-Linker (P9) synthesized in Comparative Example (1-3) were used to obtain an ADC with DAR of 2 + 8 (N-15). ESI-TOFMS analysis was performed according to the previous report (WO2019/240287A1) to observe heavy chain peaks at 55152 and 55313 and a light chain peak at 24490, confirming that 10 molecules of Payload/Payload mimic were introduced.

### Example 7: Evaluation of degree of hydrophobicity of ADC and ADC mimic by hydrophobic interaction chromatography (HIC-HPLC)

HIC-HPLC analysis was performed according to the previous report (Anal. Chem., 2019, 91, 20, 12724-12732). The measurement was performed using the following conditions. The degree of hydrophobicity of the ADC can be evaluated based on the retention time of the ADC in the HIC chromatogram.
Measurement system: 1260 HPLC system (manufactured by Agilent Technologies, Inc.)
Column: Tosoh Biobuthyl NPR 2.5 µm 4.6 × 35 mm column manufactured by Tosoh Bioscience LLC
Gradient: Linear gradient of eluent A/B
Flow rate: 0.8 mL/min
Eluent A: 1.1M (NH₄)₂SO₄, 25 mM Na₂HPO₄/NaH₂PO₄ (pH6.0)
Eluent B: 25 mM Na₂HPO₄/NaH₂PO₄ (pH6.0, 25% v/v isopropanol added)
Detector: UV (280 nm)

### (7-1) Evaluation of degree of hydrophobicity of N-8 and N-14 by hydrophobic interaction chromatography (HIC-HPLC)

The evaluation results of the degree of hydrophobicity of N-8 and N-14 by hydrophobic interaction chromatography (HIC-HPLC) are shown in Table 3-1.

**Table 3-1: Evaluation of degree of hydrophobicity of N-8 and N-14 using HIC-HPLC**

| | Compound with two molecules conjugated / Compound with eight molecules conjugated | Example / Comparable Example | Retention time (min) |
|---|---|---|---|
| N-8 | MC-MMAF/P2 | Example (6-3-4) | 14.0 |
| N-14 | MC-MMAF/P7 | Comparative Example (3-4) | 17.9 |

The ADC to which eight molecules of Payload-Linker having a hydrophilic group at the Exo-position are conjugated (N-8) synthesized in Example (6-3-4) was confirmed to have a tendency for earlier retention times compared to the ADC to which eight molecules of Payload-Linker having a hydrophilic group at the Endo position are conjugated (N-14) synthesized in Comparative Example (3-4), indicating high degree of hydrophilicity. Therefore, it was confirmed that the ADC to which eight molecules of Payload-Linker having a hydrophilic group at the Exo-position are conjugated (N-8) synthesized in Example (6-3-4) is a preferable ADC because it is considered to have slow plasma clearance and a long residence time in the body.

### (7-2) Evaluation of degree of hydrophobicity of N-5, N-11, and N-12 by hydrophobic interaction chromatography (HIC-HPLC)

The evaluation results of the degree of hydrophobicity of N-5, N-11, and N-12 by hydrophobic interaction chromatography (HIC-HPLC) are shown in Table 3-2.

**Table 3-2: Evaluation of degree of hydrophobicity of N-5, N-11, and N-12 using HIC-HPLC**

| | Compound with two molecules conjugated / Compound with eight molecules conjugated | Example / Comparable Example | Retention time (min) |
|---|---|---|---|
| N-5 | MC-Val-Cit-PAB-MMAE/P2 | Example (6-3-1) | 15.1 |
| N-11 | MC-Val-Cit-PAB-MMAE/MC-Val-Cit-PAB-MMAE | Comparative Example (3-1) | 17.7 |
| N-12 | MC-Val-Cit-PAB-MMAE/P7 | Comparative Example (3-2) | 18.2 |

The ADC to which eight molecules of Payload-Linker having a hydrophilic group at the exo-position are conjugated (N-5) synthesized in Example (6-3-1) was confirmed to have a tendency for earlier retention times compared to the ADC to which eight molecules of Endo-type Payload-Linker are conjugated (N-11) synthesized in Comparative Example (3-1) and the ADC to which eight molecules of Payload-Linker having a hydrophilic group at the Endo position are conjugated (N-12) synthesized in Comparative Example (3-2), indicating high degree of hydrophilicity. Therefore, it was confirmed that the ADC to which eight molecules of Payload-Linker having a hydrophilic group at the Exo-position are conjugated (N-5) synthesized in Example (6-3-1) is a preferable ADC because it is considered to have slow plasma clearance and a long residence time in the body.

### (7-3) Evaluation of degree of hydrophobicity of N-9, N-10, and N-15 by hydrophobic interaction chromatography (HIC-HPLC)

The evaluation results of the degree of hydrophobicity of N-9, N-10, and N-15 by hydrophobic interaction chromatography (HIC-HPLC) are shown in Table 3-3.

**Table 3-3: Evaluation of degree of hydrophobicity of N-9, N-10, and N-15 using HIC-HPLC**

| | Compound with two molecules conjugated / Compound with eight molecules conjugated | Example /Comparable Example | Retention time (min) |
|---|---|---|---|
| N-9 | MC-Val-Cit-PAB-MMAE/P5 | Example (6-3-5) | 13.9 |
| N-10 | MC-Val-Cit-PAB-MMAE/P6 | Example (6-3-6) | 13.6 |
| N-15 | MC-Val-Cit-PAB-MMAE/P9 | Comparative Example (3-5) | 16.5 |

The ADCs to which eight molecules of Payload mimic-Linker having a hydrophilic group at the exo-position are conjugated (N-9 and N-10) synthesized in Examples (6-3-5) and (6-3-6) were confirmed to have a tendency for earlier retention times compared to the ADC mimic to which eight molecules of Payload mimic-Linker having no hydrophilic group at the exo-position are conjugated (N-15) synthesized in Comparative Example (3-5), indicating high degree of hydrophilicity. Therefore, it was confirmed that the ADC mimics to which eight molecules of Payload mimic-Linker having a hydrophilic group at the exo-position are conjugated (N-9 and N-10) synthesized in Examples (6-3-5) and (6-3-6) are preferable ADC mimics because they are considered to have slow plasma clearance and a long residence time in the body.

### Example 8: Evaluation of aggregation rate of ADC and ADC mimic by size exclusion chromatography (SEC-HPLC)

SEC-HPLC analysis was performed according to the previous report (ChemistrySelect, 2020, 5, 8435-8439). The measurement was performed using the following conditions.
Measurement system: 1260 HPLC system (Agilent Technologies, Inc.)
Column: AdvanceBio SEC 300 Å 2.7 µm, 4.6 mm × 300 mm manufactured by Agilent Technologies, Inc.
Flow rate: 0.25 mL/min
Eluent: 100 mM sodium dihydrogen phosphate/sodium hydrogen phosphate, 250 mM aqueous sodium chloride solution (pH 6.8), 10% v/v isopropanol
Detector: UV (280 nm)

### (8-1) Evaluation of aggregation rate of N-5, N-11, and N-12 by size exclusion chromatography (SEC-HPLC)

The Evaluation results of aggregation rate of N-5, N-11, and N-12 by size exclusion chromatography (SEC-HPLC) are shown in Table 4-1.

**Table 4-1: Evaluation of degree of hydrophobicity of N-5, N-11, and N-12 using SEC-HPLC**

| | Compound with two molecules conjugated / Compound with eight molecules conjugated | Example / Comparable Example | Aggregation rate (%) |
|---|---|---|---|
| N-5 | MC-Val-Cit-PAB-MMAE/P2 | Example (6-3-1) | 0.38 |
| N-11 | MC-Val-Cit-PAB-MMAE/MC-Val-Cit-PAB-MMAE | Comparative Example (3-1) | 2.93 |
| N-12 | MC-Val-Cit-PAB-MMAE/P7 | Comparative Example (3-2) | 1.22 |

The ADC to which eight molecules of Payload-Linker having a hydrophilic group at the exo-position are conjugated (N-5) synthesized in Example (6-3-1) was confirmed to have a tendency for lower aggregation rate compared to the ADC to which eight molecules of Endo-type Payload-Linker are conjugated (N-11) synthesized in Comparative Example (3-1) and the ADC to which eight molecules of Payload-Linker having a hydrophilic group at the Endo position are conjugated (N-12) synthesized in Comparative Example (3-2).

### (8-2) Evaluation of aggregation rate of N-9, N-10, and N-15 by size exclusion chromatography (SEC-HPLC)

The evaluation results of the aggregation rate of N-9, N-10, and N-15 by size exclusion chromatography (SEC-HPLC) are shown in Table 4-2.

**Table 4-2: Evaluation of degree of hydrophobicity of N-9, N-10, and N-15 using SEC-HPLC**

| | Compound with two molecules conjugated / Compound with eight molecules conjugated | Example / Comparable Example | Aggregation rate (%) |
|---|---|---|---|
| N-9 | MC-Val-Cit-PAB-MMAE/P5 | Example (6-3-5) | 0.66 |
| N-10 | MC-Val-Cit-PAB-MMAE/P6 | Example (6-3-6) | 0.68 |
| N-15 | MC-Val-Cit-PAB-MMAE/P9 | Comparative Example (3-5) | 9.4 |

The ADCs to which eight molecules of Payload mimic-Linker having a hydrophilic group at the Exo-position are conjugated (N-9 and N-10) synthesized in Examples (6-3-5) and (6-3-6) was confirmed to have a tendency for lower aggregation rate compared to the ADC mimic to which eight molecules of Payload mimic-Linker having no hydrophilic group at the Exo-position are conjugated (N-15) synthesized in Comparative Example (3-5).

### Example 9: Synthesis of ADC with DAR of 10

### (9-1) Synthesis of ADC with DAR of 10 (Q3)

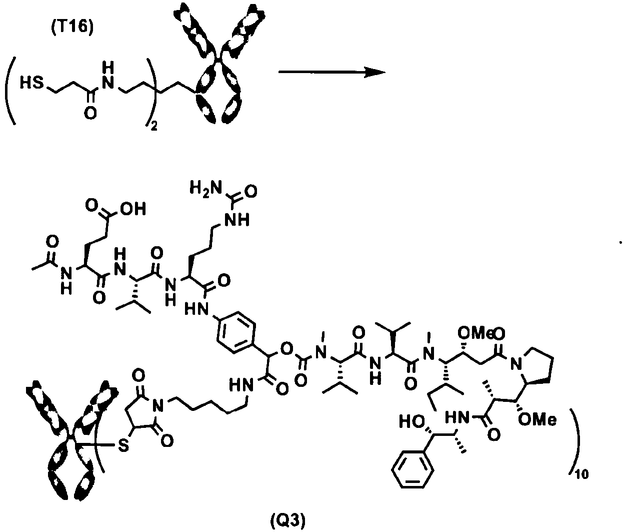

To a 5 mg/mL solution of the antibody having two molecules of thiol group (T-16) synthesized in Example (5) in PBSE buffer (10 mM Phosphate Buffered Saline (PBS), 10 mM EDTA, pH7.4), 15 equivalents of a 20 mM solution of a reducing agent (TCEP) in PBSE relative to the antibody were added, and the mixture was agitated at 37°C for 90 minutes. To the reaction solution, a solution of Payload-Linker (P1) synthesized in Example (1-1) in dimethylacetamide (20 equivalents relative to the antibody, 8% v/v dimethylacetamide (DMA) solution) was added, and the mixture was agitated at room temperature for 30 minutes. The reaction solution was purified using a NAP-25 desalting column (Cytiva) to obtain an antibody with 10 molecules of Payload-Linker (P1) introduced (Q3). ESI-TOFMS analysis was performed according to the previous report (WO2019/240287A1) to observe heavy chain peaks at 56695 and 56856 and a light chain peak at 24943, confirming that 10 molecules of Payload-Linker (P1) were introduced.

### Example 10: Synthesis of ADC with DAR of 12

### (10-1) Synthesis of antibody with four molecules of thiol group bound to Lys246/248 and Lys288/290 of the antibody (Q2)

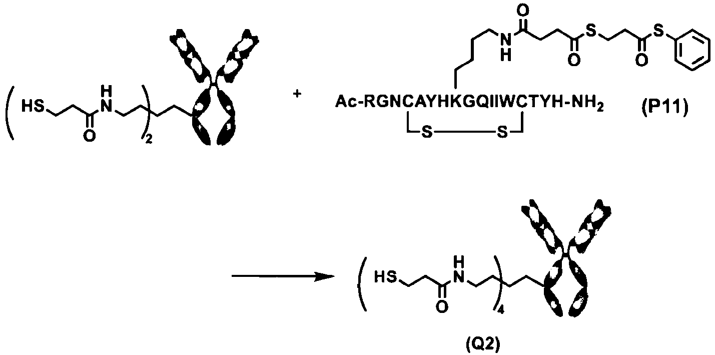

The antibody with two molecules of thiol group bound synthesized in Example (5) (T-16) and a peptide reagent (P11) described in the previous report (WO2019/240287A1) were used following the method described in the previous report (WO2019/240287A1) to obtain an antibody with four molecules of thiol group introduced. HIC-HPLC analysis was performed according to the previous report (Anal. Chem., 2019, 91, 20, 12724-12732) to confirm that four molecules of thiol group were introduced.

### (10-2) Synthesis of ADC with DAR of 12 (Q4)

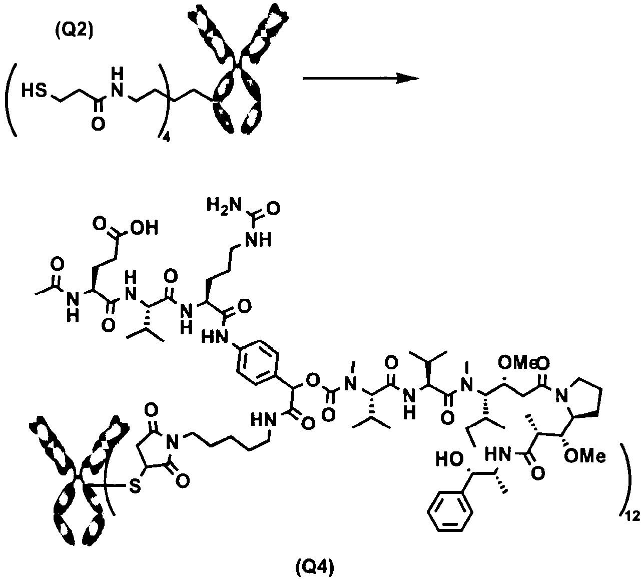

To a 5 mg/mL solution of the antibody having four molecules of thiol group (Q2) synthesized in Example (10-1) in PBSE buffer (10 mM Phosphate Buffered Saline (PBS), 10 mM EDTA, pH7.4), 15 equivalents of a 20 mM solution of a reducing agent (TCEP) in PBSE relative to the antibody were added, and the mixture was agitated at 37°C for 90 minutes. To the reaction solution, a solution of Payload-Linker (P1) synthesized in Example (1-1) in dimethylacetamide (20 equivalents relative to the antibody, 8% v/v dimethylacetamide (DMA) solution) was added, and the mixture was agitated at room temperature for 30 minutes. The reaction solution was purified using a NAP-25 desalting column (Cytiva) to obtain an antibody with 12 molecules of Payload-Linker (P1) introduced (Q4). ESI-TOFMS analysis was performed according to the previous report (WO2019/240287A1) to observe heavy chain peaks at 58286 and 58447 and a light chain peak at 24943, confirming that 12 molecules of Payload-Linker (P1) were introduced.

### (10-3) Synthesis of ADC with DAR of 12 (Q5)

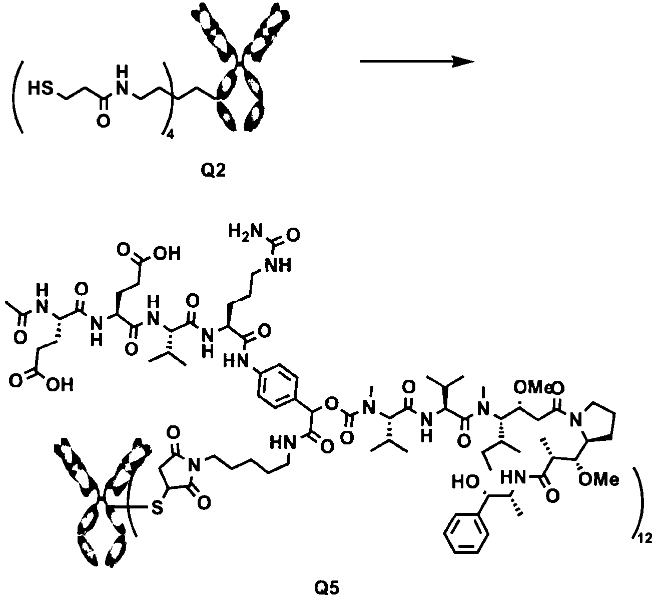

According to Example (10-2), the antibody having four molecules of thiol group (Q2) and Payload-Linker (P2) were used to obtain an antibody with 12 molecules of Payload-Linker (P2) introduced (Q5). ESI-TOFMS analysis was performed according to the previous report (WO2019/240287A1) to observe heavy chain peaks at 58932 and 59094 and a light chain peak at 25072, confirming that 12 molecules of Payload-Linker (P2) were introduced.

### Comparative Example 4: Synthesis of ADC of DAR = 10

### (4-1) Synthesis of ADC of DAR = 10 (S1)

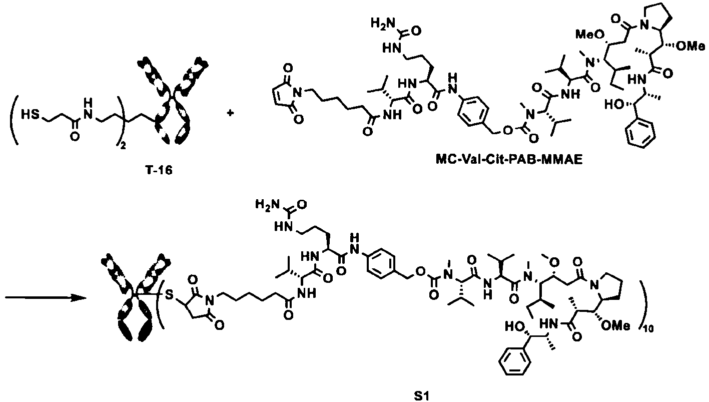

According to Example (9-1), the antibody having two molecules of thiol group (T-16) and the known compound MC-Val-Cit-PABA-MMAE (Organic & Biomolecular Chemistry, 2016, 14, 9501-9518) were used to obtain an antibody with 10 molecules of Val-Cit-PABA-MMAE introduced (S1). ESI-TOFMS analysis was performed according to the previous report (WO2019/240287A1) to observe heavy chain peaks at 55944 and 56111 and a light chain peak at 24756, confirming that 10 molecules of Val-Cit-PABA-MMAE were introduced.

### (4-2) Synthesis of ADC of DAR = 10 (S2)

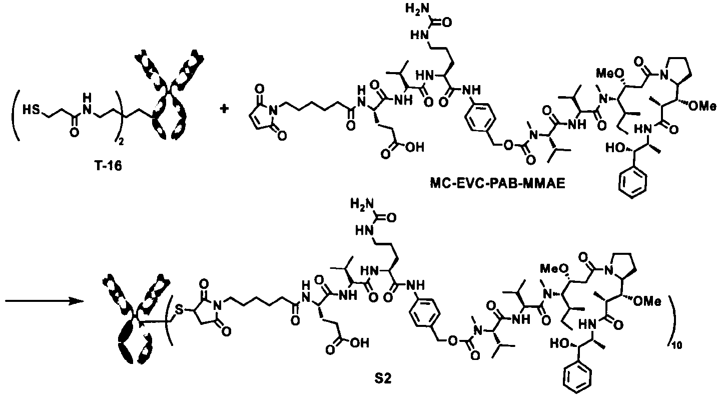

According to Example (9-1), the antibody having two molecules of thiol group (T-16) and MC-Glu-Val-Cit-PAB-MMAE (P7) were used to obtain an antibody with 10 molecules of P7 introduced (S2). ESI-TOFMS analysis was performed according to the previous report (WO2019/240287A1) to observe heavy chain peaks at 56465 and 56627 and a light chain peak at 24886, confirming that 10 molecules of P7 were introduced.

### Comparative Example 5: Synthesis of ADC with DAR of 12

### (5-1) Synthesis of ADC with DAR of 12 (S3)

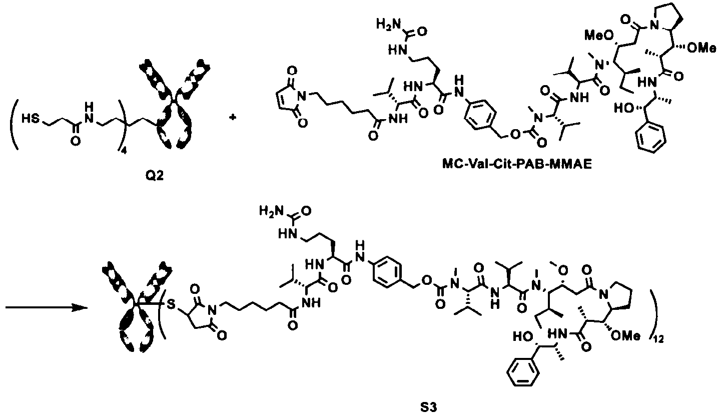

According to Example (10-2), the antibody having four molecules of thiol group (Q2) obtained in Example (10-1) and the known compound MC-Val-Cit-PABA-MMAE (Organic & Biomolecular Chemistry, 2016, 14, 9501-9518) were used to obtain an antibody with 12 molecules of MC-Val-Cit-PABA-MMAE introduced (S3). ESI-TOFMS analysis was performed according to the previous report (WO2019/240287A1) to observe heavy chain peaks at 57356 and 57518 and a light chain peak at 24756, confirming that 12 molecules of MC-Val-Cit-PABA-MMAE were introduced.

### (5-2) Synthesis of ADC with DAR of 12 (S4)

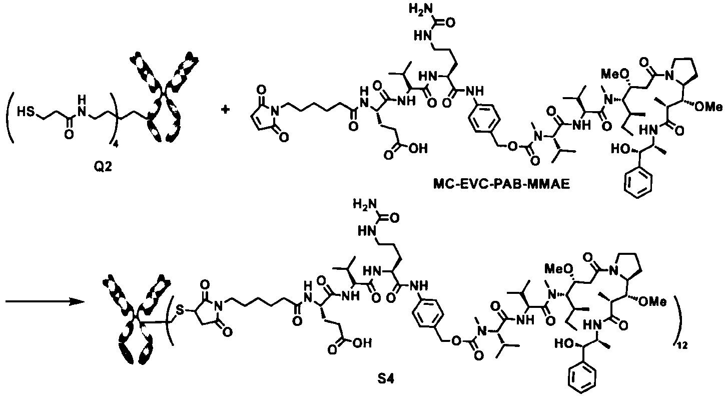

According to Example (10-2), the antibody having four molecules of thiol group (Q2) and MC-Glu-Val-Cit-PAB-MMAE (P7) were used to obtain an antibody with 12 molecules of MC-Glu-Val-Cit-PAB-MMAE (P7) introduced (S4). ESI-TOFMS analysis was performed according to the previous report (WO2019/240287A1) to observe heavy chain peaks at 58011 and 58173 and a light chain peak at 24885, confirming that 12 molecules of MC-Glu-Val-Cit-PAB-MMAE were introduced (P7).

### Example 11: Synthesis of ADC with DAR of 2 + 8

### (11-1) Synthesis of ADC with DAR of 2 (Q6)

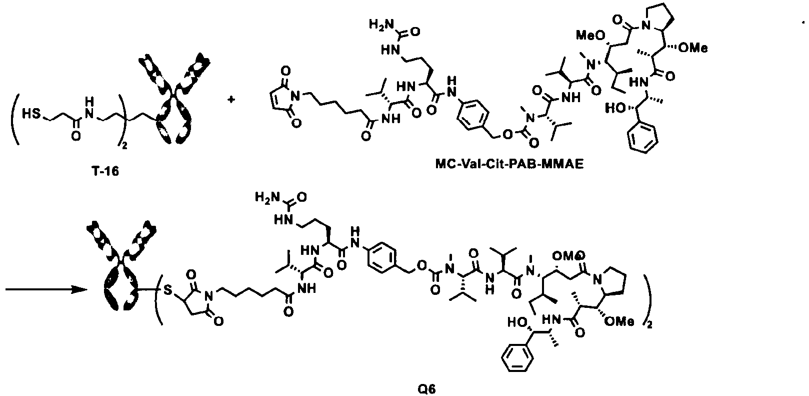

To a 4 mg/mL solution of the antibody with two molecules of thiol group introduced (T-16) synthesized in Example (5) in PBSE buffer (10 mM Phosphate Buffered Saline (PBS), 10 mM EDTA, pH7.4), a solution of a known compound MC-Val-Cit-PABA-MMAE (Organic & Biomolecular Chemistry, 2016, 14, 9501-9518) in DMA (5 equivalents relative to the antibody, 8% v/v dimethylacetamide (DMA) solution) was added, and the mixture was agitated at room temperature for one hour. The final mixture was purified using a NAP-10 desalting column (Cytiva) to obtain an ADC with DAR of 2 (Q6). ESI-TOFMS analysis was performed according to the previous report (WO2019/240287A1) to observe a peak at 151028, confirming that two molecules of Val-Cit-PABA-MMAE were introduced.

### (11-2) Synthesis of ADC with DAR of 2 + 8 (Q7)

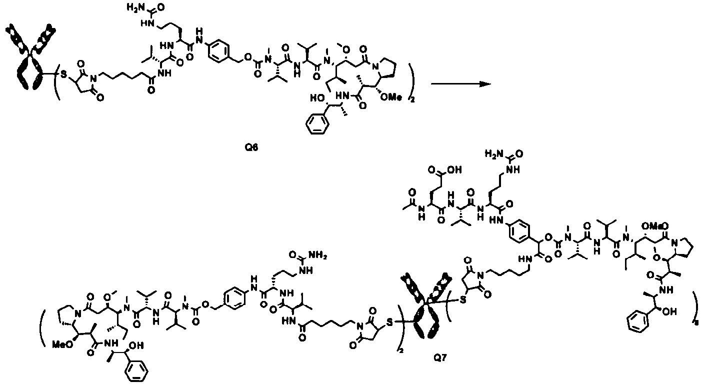

To a 5 mg/mL solution of the ADC (Q6) synthesized in Example (11-1) in PBSE buffer (10 mM Phosphate Buffered Saline (PBS), 10 mM EDTA, pH7.4), 15 equivalents of a 20 mM solution of a reducing agent (TCEP) in PBSE relative to the antibody were added, and the mixture was agitated at 37°C for 1.5 hours. To the reaction solution, a solution of Payload-Linker (P1) synthesized in Example (1-1) in DMA (20 equivalents relative to the antibody, 8% v/v dimethylacetamide (DMA) solution) was added, and the mixture was agitated at room temperature for one hour. The final mixture was purified using a NAP-10 desalting column (Cytiva) to obtain an ADC with DAR of 2 + 8 (Q7). ESI-TOFMS analysis was performed according to the previous report (WO2019/240287A1) to observe heavy chain peaks at 56511 and 56673 and a light chain peak at 24943, confirming that 10 molecules of Payload were introduced.

### Comparative Example 6: Synthesis of ADC with DAR of 2 + 8

### (6-1) Synthesis of ADC with DAR of 2 + 8 (S5)

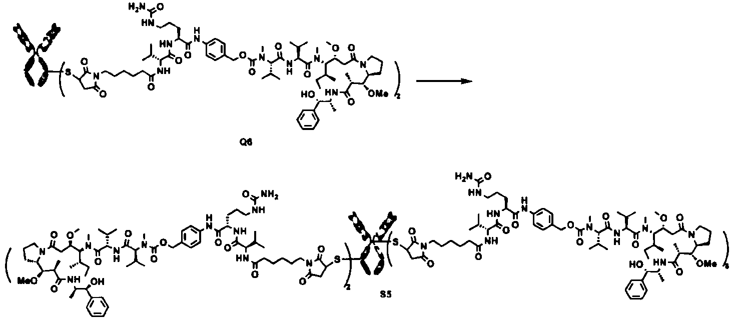

According to Example (11-2), ADC (Q6) synthesized in Example (11-1) and a known compound MC-Val-Cit-PABA-MMAE were used to obtain an ADC with DAR of 2 + 8 (S5). ESI-TOFMS analysis was performed according to the previous report (WO2019/240287A1) to observe heavy chain peaks at 55943 and 56112 and a light chain peak at 24756, confirming that 10 molecules of Payload were introduced.

### (6-2) Synthesis of ADC with DAR of 2 + 8 (S6)

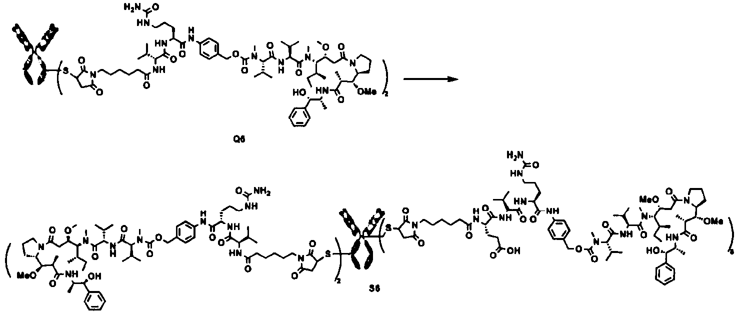

According to Example (11-2), ADC (Q6) synthesized in Example (11-1) and a known compound MC-Glu-Val-Cit-PABA-MMAE (P7) were used to obtain an ADC with DAR of 2 + 8 (S6). ESI-TOFMS analysis was performed according to the previous report (WO2019/240287A1) to observe heavy chain peaks at 56337 and 56499 and a light chain peak at 24886, confirming that 10 molecules of Payload (P7) were introduced.

### Example 12: Evaluation of degree of hydrophobicity of ADC and ADC mimic by hydrophobic interaction chromatography (HIC-HPLC)

### (12-1) Evaluation of degree of hydrophobicity of Q3, S1, and S2 by hydrophobic interaction chromatography (HIC-HPLC)

Following Example (3), the degrees of hydrophobicity of Q3, S1, and S2 were evaluated by hydrophobic interaction chromatography (HIC-HPLC). The results are shown in Table 5-1.

**Table 5-1: Evaluation of degree of hydrophobicity of Q3, S1, and S2 using HIC-HPLC**

| | Linker-payload mimic / Linker-payload | Example / Comparable Example | Retention time (min) |
|---|---|---|---|
| Q3 | P1 | Example (9-1) | 15.3 |
| S1 | MC-Val-Cit-PAB-MMAE | Comparative Example (4-1) | 19.2 |
| S2 | P7 | Comparative Example (4-2) | 18.8 |

The ADC to which Payload-Linker having a hydrophilic group at the exo-position is conjugated (Q3) synthesized in Example (9-1) was confirmed to have a tendency for earlier retention times compared to the ADC to which endo-type Payload-Linker are conjugated (S1) synthesized in Comparative Example (4-1) and the ADC to which Payload-Linker having a hydrophilic group at the endo-position is conjugated (S2) synthesized in Comparative Example (4-2), indicating high degree of hydrophilicity. Therefore, it was confirmed that the Payload-Linker ADC having a hydrophilic group at the exo-position (Q3) synthesized in Example (9-1) is a preferable ADC because it is considered to have slow plasma clearance and a long residence time in the body.

### (12-2) Evaluation of degree of hydrophobicity of Q4, Q5, S3, and S4 by hydrophobic interaction chromatography (HIC-HPLC)

Following Example (3), the degrees of hydrophobicity of Q4, Q5, S3, and S4 were evaluated by hydrophobic interaction chromatography (HIC-HPLC). The results are shown in Table 5-2.

**Table 5-2: Evaluation of degree of hydrophobicity of Q4, Q5, S3, and S4 using HIC-HPLC**

| | Linker-payload mimic / Linker-payload | Example / Comparable Example | Retention time (min) |
|---|---|---|---|
| Q4 | P1 | Example (10-2) | 17.6 |
| Q5 | P2 | Example (10-3) | 16.4 |
| S3 | MC-Val-Cit-PAB-MMAE | Comparative Example (5-1) | 19.7 |
| S4 | P7 | Comparative Example (5-2) | 19.5 |

The ADCs to which Payload-Linker having a hydrophilic group at the exo-position is conjugated (Q4 and Q5) synthesized in Examples (10-2 and 10-3) were confirmed to have a tendency for earlier retention times compared to the ADC to which endo-type Payload-Linker are conjugated (S3) and the ADC to which Payload-Linker having a hydrophilic group at the endo-position is conjugated (S4), synthesized in Comparative Examples (5-1 and 5-2), indicating high degree of hydrophilicity. Therefore, it was confirmed that the Payload-Linker ADCs having a hydrophilic group at the exo-position (Q4 and Q5) synthesized in Examples (10-2 and 10-3) are preferable ADCs because they are considered to have slow plasma clearance and a long residence time in the body.

### (12-3) Evaluation of degree of hydrophobicity of Q7, S5, and S6 by hydrophobic interaction chromatography (HIC-HPLC)

Following Example (3), the degrees of hydrophobicity of Q7, S5, and S6 were evaluated by hydrophobic interaction chromatography (HIC-HPLC). The results are shown in Table 5-3.

**Table 5-3: Evaluation of degree of hydrophobicity of Q7, S5, and S6 using HIC-HPLC**

| | Linker-payload mimic / Linker-payload | Example / Comparable Example | Retention time (min) |
|---|---|---|---|
| Q7 | MC-Val-Cit-PAB-MMAE/P1 | Example (11-2) | 16.0 |
| S5 | MC-Val-Cit-PAB-MMAE/MC-Val-Cit-PAB-MMAE | Comparative Example (6-1) | 17.7 |
| S6 | MC-Val-Cit-PAB-MMAE/P7 | Comparative Example (6-2) | 19.5 |

The ADC to which Payload-Linker having a hydrophilic group at the exo-position is conjugated (Q7) synthesized in Example (11-2) were confirmed to have a tendency for earlier retention times compared to the ADC to which endo-type Payload-Linker are conjugated (S5) and the ADC to which Payload-Linker having a hydrophilic group at the endo-position is conjugated (S6), synthesized in Comparative Examples (6-1 and 6-2), indicating high degree of hydrophilicity. Therefore, it was confirmed that the Payload-Linker ADC having a hydrophilic group at the exo-position (Q7) synthesized in Example (11-2) is a preferable ADC because it is considered to have slow plasma clearance and a long residence time in the body.

### Example 13: Evaluation of aggregation rate of ADC and ADC mimic by size exclusion chromatography (SEC-HPLC)

### (13-1) Evaluation of aggregation rate of Q3, S1, and S2 by size exclusion chromatography (SEC-HPLC)

Following Example (4), the aggregation rates of Q3, S1, and S2 were evaluated by size exclusion chromatography (SEC-HPLC). The results are shown in Table 6-1.

**Table 6-1: Evaluation of degree of hydrophobicity of Q3, S1, and S2 using SEC-HPLC**

| | Linker-payload | Example / Comparable Example | Aggregation rate (%) |
|---|---|---|---|
| Q3 | P1 | Example (9-1) | 0.46 |
| S1 | MC-Val-Cit-PAB-MMAE | Comparative Example (4-1) | >10 |
| S2 | P7 | Comparative Example (4-2) | 0.51 |

The ADC to which Payload-Linker having a hydrophilic group at the exo-position is conjugated (Q3) synthesized in Example (9-1) and ADC to which Payload-Linker having a hydrophilic group at the endo-position is conjugated (S2) synthesized in Comparative Example (4-2) are confirmed to have a tendency for lower aggregation rates compared to the ADC to which endo-type Payload-Linker is conjugated (S1) synthesized in Comparative Example (4-1).

### (13-2) Evaluation of aggregation rate of Q4, Q5, S3, and S4 by size exclusion chromatography (SEC-HPLC)

Following Example (4), the aggregation rates of Q4, Q5, S3, and S4 were evaluated by size exclusion chromatography (SEC-HPLC). The results are shown in Table 6-2.

**Table 6-2: Evaluation of degree of hydrophobicity of Q4, Q5, S3, and S4 using SEC-HPLC**

| | Linker-payload | Example / Comparable Example | Aggregation rate (%) |
|---|---|---|---|
| Q4 | P1 | Example (10-2) | 2.16 |
| Q5 | P2 | Example (10-3) | 1.74 |
| S3 | MC-Val-Cit-PAB-MMAE | Comparative Example (5-1) | 55.8 |
| S4 | P7 | Comparative Example (5-2) | 4.26 |

The ADCs to which Payload-Linker having a hydrophilic group at the exo-position is conjugated (Q4 and Q5) synthesized in Examples (10-2 and 10-3) were confirmed to have a tendency for lower aggregation rates compared to the ADC to which endo-type Payload-Linker is conjugated (S3) synthesized in Comparative Example (5-1) and the ADC to which Payload-Linker having a hydrophilic group at the endo-position is conjugated (S4) synthesized in Comparative Example (5-2).

### (13-3) Evaluation of aggregation rate of Q7, S5, and S6 by size exclusion chromatography (SEC-HPLC)

Following Example (4), the aggregation rates of Q7, S5, and S6 were evaluated by size exclusion chromatography (SEC-HPLC). The results are shown in Table 6-3.

**Table 6-3: Evaluation of degree of hydrophobicity of Q7, S5, and S6 using SEC-HPLC**

| | Compound with two molecules conjugated / Compound with eight molecules conjugated | Example / Comparable Example | Aggregation rate (%) |
|---|---|---|---|
| Q7 | MC-Val-Cit-PAB-MMAE/P1 | Example (11-2) | 0.43 |
| S5 | MC-Val-Cit-PAB-MMAE/MC-Val-Cit-PAB-MMAE | Comparative Example (6-1) | >10 |
| S6 | MC-Val-Cit-PAB-MMAE/P7 | Comparative Example (6-2) | 0.99 |

The ADC to which Payload-Linker having a hydrophilic group at the exo-position is conjugated (Q7) synthesized in Examples (11-2) and ADC to which Payload-Linker having a hydrophilic group at the endo-position is conjugated (S6) synthesized in Comparative Example (6-2) were confirmed to have a tendency for lower aggregation rates compared to the ADC to which endo-type Payload-Linker is conjugated (S5) synthesized in Comparative Example (6-1).

### Example 14: Evaluation of ADC using mouse plasma

### (14-1) Plasma stability test of ADC

To 500 µL of mouse plasma (Charles River Laboratories), the ADC mimic was added to a concentration of 0.1 mg/mL, followed by sterile filtration. This solution was dispensed in 50 µL aliquots into six Eppendorf tubes. Three of the six samples were stored in an incubator set at 37°C for 4 days. The remaining three were similarly stored in a freezer at -80°C for 4 days. To each sample, 100 µL of acetonitrile was added, vortexed, and then centrifuged to obtain a precipitate. The resulting supernatant was collected and subjected to HPLC analysis.

### (14-2) Quantitation of detached payload using HPLC analysis

The quantitation was performed by measuring the amount of the payload detached from ADC using a liquid chromatography/mass spectrometry (including tandem mass spectrometry). The samples stored similarly for 4 days in a -80°C freezer in Example (24-1) were designated as Day 0 samples, and the three samples incubated at 37°C for 4 days in Example (24-1) were designated as Day 4 samples, and the MS intensities of the payload detected from the Day 4 and Day 0 samples were calculated by extracted ion chromatograms, and the difference between the intensities was determined. Separately, using MMAE, the correlation between the area of TIC by HPLC and the concentration was calculated. Using that calculation formula, the TIC derived from the fluorescence intensity of each of the above ADCs was converted to concentration. The ratio of the aforementioned difference in the ion chromatograms, with the concentration at Day 0 as 100%, was calculated as the detachment rate.

### (14-3) Plasma stability test of ADCs with DAR of 10 (T-3, -4, -9, and -11)

Plasma stability test of ADCs with DAR of 10 (T-3, -4, -9, and -11) was performed. The results are shown in Table 7-1.

**Table 7-1: Results of plasma stability test of ADCs with DAR of 10 (T-3, -4, -9, and -11)**

| | Linker-payload | Example / Comparable Example | Detachment rate of payload at Day 4 |
|---|---|---|---|
| T-3 | P1 | Example (2-3) | 3% |
| T-4 | P2 | Example (2-4) | 0% |
| T-9 | MC-Val-Cit-PAB-MMAE | Comparative Example (2-1) | 9% |
| T-11 | P7 | Comparative Example (2-3) | 2% |

As a result, it was confirmed that the ADCs synthesized in Examples (2-3) and (2-4), and Comparative Example (2-3) have high stability.

### (14-4) Plasma stability test of ADCs with DAR of 10 (Q3, S1, and S2)

Plasma stability test of ADC with DAR of 10 (Q3, S1, and S2) was performed. The results are shown in Table 7-2.

**Table 7-2: Results of plasma stability test of ADCs with DAR of 10 (Q3, S1, and S2)**

| | Linker-payload | Example / Comparable Example | Detachment rate of payload at Day 4 |
|---|---|---|---|
| Q3 | P1 | Example (9-1) | 2% |
| S1 | MC-Val-Cit-PAB-MMAE | Comparative Example (4-1) | 10% |
| S2 | P7 | Comparative Example (4-2) | 4% |

As a result, it was confirmed that the ADC synthesized in Example (9-1) has high stability.

### (14-5) Plasma stability test of ADCs with DAR of 12 (Q4, Q5, S3, and S4)

Plasma stability test of ADC with DAR of 12 (Q4, Q5, S3, and S4) was performed. The results are shown in Table 7-3.

**Table 7-3: Results of plasma stability test of ADCs with DAR of 12 (Q4, Q5, S3, and S4)**

| | Linker-payload | Example / Comparable Example | Detachment rate of payload at Day 4 |
|---|---|---|---|
| Q4 | P1 | Example (10-2) | 2% |
| Q5 | P2 | Example (10-3) | 0% |
| S3 | MC-Val-Cit-PAB-MMAE | Comparative Example (5-1) | 15% |
| S4 | P7 | Comparative Example (5-2) | 3% |

As a result, it was confirmed that the ADCs synthesized in Examples (10-2 and 10-3) and Comparative Example (5-2) have high stability.

## Claims

1. A conjugate of an antibody and a functional substance, or a salt thereof, wherein
(1) the conjugate comprises an immunoglobulin unit comprising two heavy chains and two light chains,
(2) said immunoglobulin unit comprises:
(a) two or more cysteine residue-modifying moieties connected to the immunoglobulin unit by bonding to the sulfur atoms in the side chains of two or more cysteine residues in said two heavy chains and two light chains, and
(ii) two or more lysine residue-modifying moieties regioselectively connected to the immunoglobulin unit by bonding to the nitrogen atoms in the side chains of two or more lysine residues in said two heavy chains, and
(3) said cysteine residue-modifying moiety is represented by the following formula (I-1): 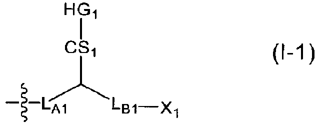 wherein
the hyphen (-) with a wavy line represents a bond to said sulfur atom,
HG₁ represents a hydrophilic group or a monovalent group containing a hydrophilic group,
CS₁ represents a divalent group containing a cleavable site,
L_{A1} and L_{B1} each independently represent a divalent group, and
X₁ represents a functional substance.

2. The conjugate or a salt thereof of claim 1, wherein the immunoglobulin unit is a human immunoglobulin unit.

3. The conjugate or a salt thereof of claim 2, wherein the human immunoglobulin unit is a human IgG antibody.

4. The conjugate or a salt thereof of claim 1, wherein
said two or more cysteine residues are 2 to 8 cysteine residues,
said two or more cysteine residue-modifying moieties are 2 to 8 cysteine residue-modifying moieties, and
the 2 to 8 cysteine residue-modifying moieties are connected to the immunoglobulin unit by bonding to the sulfur atoms in the side chains of 2 to 8 cysteine residues in said two heavy chains and two light chains.

5. The conjugate or a salt thereof of claim 1, wherein
said two or more cysteine residues are 8 cysteine residues,
said two or more cysteine residue-modifying moieties are 8 cysteine residue-modifying moieties, and
the 8 cysteine residue-modifying moieties are connected to the immunoglobulin unit by bonding to the sulfur atoms in the side chains of 8 cysteine residues in said two heavy chains and two light chains.

6. The conjugate or a salt thereof of claim 1, wherein the lysine residue-modifying moiety is represented by the following formula (I-2): 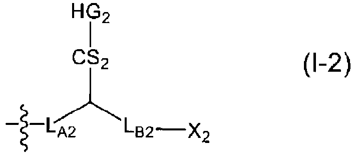 wherein
the hyphen (-) with a wavy line represents a bond to said nitrogen atom,
HG₂ represents a hydrophilic group or a monovalent group containing a hydrophilic group,
CS₂ represents a divalent group containing a cleavable site,
L_{A2} and L_{B2} each independently represent a divalent group, and
X₂ represents a functional substance,
or the following formula (II): 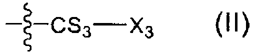 wherein
the hyphen (-) with a wavy line represents a bond to said nitrogen atom,
CS₃ represents a divalent group optionally containing a cleavable site, and
X₃ represents a functional substance.

7. The conjugate or a salt thereof of claim 1, wherein the two or more lysine residues are any of the lysine residues present at the following positions according to Eu numbering:
(A) a lysine residue present at position 246/248 in the two heavy chains;
(ii) a lysine residue present at position 288/290 in the two heavy chains;
(C) a lysine residue present at position 317 in the two heavy chains; and
(D) a combination of (A) and (ii), (A) and (C), or (ii) and (C), or (A), (B), and (C).

8. The conjugate or a salt thereof of claim 1, wherein the two or more lysine residue-modifying moieties are regioselectively connected to the immunoglobulin unit by bonding to the nitrogen atoms in the side chains of two lysine residues present at the same position in said two heavy chains.

9. The conjugate or a salt thereof of claim 1, wherein
the two or more lysine residue-modifying moieties comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i),
the first lysine residue-modifying moiety is represented by the following formula (I-2-1): 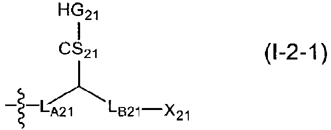 wherein
the hyphen (-) with a wavy line represents a bond to said nitrogen atom,
HG₂₁ represents a hydrophilic group or a monovalent group containing a hydrophilic group,
CS₂₁ represents a divalent group containing a cleavable site,
L_{A21} and L_{B21} each independently represent a divalent group, and
X₂₁ represents a functional substance,
or the following formula (II-1): 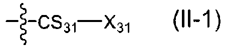 wherein
the hyphen (-) with a wavy line represents a bond to said nitrogen atom,
CS₃₁ represents a divalent group optionally containing a cleavable site, and
X₃₁ represents a functional substance,
and
the second lysine residue-modifying moiety is represented by the following formula (I-2-2): 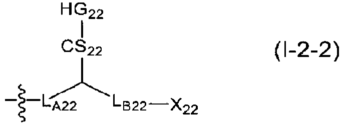 wherein
the hyphen (-) with a wavy line represents a bond to said nitrogen atom,
HG₂₂ represents a hydrophilic group or a monovalent group containing a hydrophilic group,
CS₂₂ represents a divalent group containing a cleavable site,
L_{A22} and L_{B22} each independently represent a divalent group, and
X₂₂ represents a functional substance,
or the following formula (II-2): 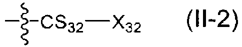 wherein
the hyphen (-) with a wavy line represents a bond to said nitrogen atom,
CS₃₂ represents a divalent group optionally containing a cleavable site, and
X₃₂ represents a functional substance.

10. The conjugate or a salt thereof of claim 1, wherein the hydrophilic group is one or more groups selected from the group consisting of a carboxylic acid group, a sulfonic acid group, a sulfamide group, a hydroxyl group, a polyethylene glycol group, a polysarcosine group, and a sugar moiety.

11. The conjugate or a salt thereof of claim 1, wherein the cleavable site is a site cleavable by an enzyme.

12. The conjugate or a salt thereof of claim 11, wherein the site cleavable by an enzyme is a peptidic site.

13. The conjugate or a salt thereof of claim 1, wherein the functional substance is a pharmaceutical agent, a labeling substance, or a stabilizing agent.

14. The conjugate or a salt thereof of claim 1, wherein the conjugation ratio of the functional substance to the antibody (functional substance/antibody) is 10 or more.

15. The conjugate or a salt thereof of claim 1, wherein said regioselective conjugate shows an aggregation rate of 2.6% or less as determined by size exclusion chromatography.

16. The conjugate or a salt thereof of claim 1, wherein
the cysteine residue-modifying moiety is represented by the following formula (I-1'): 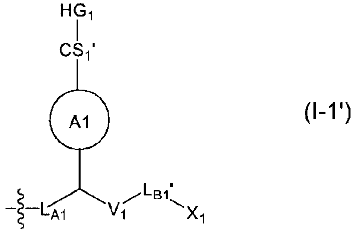 wherein
the hyphen (-) with a wavy line represents a bond to said sulfur atom,
HG₁ represents a hydrophilic group or a monovalent group containing a hydrophilic group,
CS₁' represents a divalent group containing a cleavable site,
Ring A1 represents an optionally substituted divalent aromatic ring group (wherein the divalent aromatic ring group forms a pi electron conjugated system with said cleavable site),
V₁ represents an oxygen atom, a sulfur atom, or an amino group (NH),
L_{A1} and L_{B1}' each independently represent a divalent group, and
X₁ represents a functional substance, and
the lysine residue-modifying moiety is represented by the following formula (I-2'): 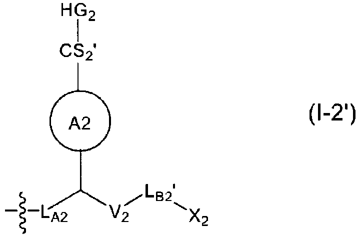 wherein
the hyphen (-) with a wavy line represents a bond to said nitrogen atom,
HG₂ represents a hydrophilic group or a monovalent group containing a hydrophilic group,
CS₂' represents a divalent group containing a cleavable site,
Ring A2 represents an optionally substituted divalent aromatic ring group (wherein the divalent aromatic ring group forms a pi electron conjugated system with said cleavable site),
V₂ represents an oxygen atom, a sulfur atom, or an amino group (NH),
L_{A2} and L_{B2}' each independently represent a divalent group, and
X₂ represents a functional substance,
or the following formula (II): 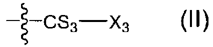 wherein
the hyphen (-) with a wavy line represents a bond to said nitrogen atom,
CS₃ represents a divalent group optionally containing a cleavable site, and
X₃ represents a functional substance.

17. The conjugate or a salt thereof of claim 16, wherein the optionally substituted divalent aromatic ring group is an optionally substituted phenylene group.

18. The conjugate or a salt thereof of claim 16, which is (A) or (ii) below:
(A) the lysine residue-modifying moieties are regioselectively connected to the immunoglobulin unit by bonding to the nitrogen atoms in the side chains of two lysine residues present at the same position in said two heavy chains; or
(ii) the lysine residue-modifying moieties comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i),
wherein
the first lysine residue-modifying moiety is represented by the following formula (I-2-1'): 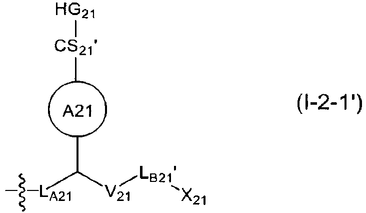
wherein
the hyphen (-) with a wavy line represents a bond to said nitrogen atom,
HG₂₁ represents a hydrophilic group or a monovalent group containing a hydrophilic group,
CS₂₁' represents a divalent group containing a cleavable site,
Ring A21 represents an optionally substituted divalent aromatic ring group (wherein the divalent aromatic ring group forms a pi electron conjugated system with said cleavable site),
V₂₁ represents an oxygen atom, a sulfur atom, or an amino group (NH),
L_{A21} and L_{B21}' each independently represent a divalent group, and
X₂₁ represents a functional substance,
or the following formula (II-1): 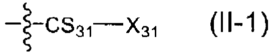 wherein
the hyphen (-) with a wavy line represents a bond to said nitrogen atom,
CS₃₁ represents a divalent group optionally containing a cleavable site, and
X₃₁ represents a functional substance, and
the second lysine residue-modifying moiety is represented by the following formula (I-2-2'): 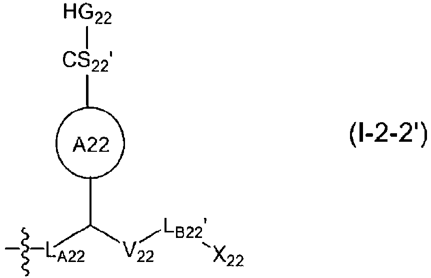 wherein
the hyphen (-) with a wavy line represents a bond to said nitrogen atom,
HG₂₂ represents a hydrophilic group or a monovalent group containing a hydrophilic group,
CS₂₂' represents a divalent group containing a cleavable site,
Ring A22 represents an optionally substituted divalent aromatic ring group (wherein the divalent aromatic ring group forms a pi electron conjugated system with said cleavable site),
V₂₂ represents an oxygen atom, a sulfur atom, or an amino group (NH),
L_{A22} and L_{B22}' each independently represent a divalent group, and
X₂₂ represents a functional substance,
or the following formula (II-2): 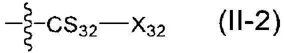 wherein
the hyphen (-) with a wavy line represents a bond to said nitrogen atom,
CS₃₂ represents a divalent group optionally containing a cleavable site, and
X₃₂ represents a functional substance.

19. The conjugate or a salt thereof of claim 1, wherein
the cysteine residue-modifying moiety is represented by the following formula (I-1"): 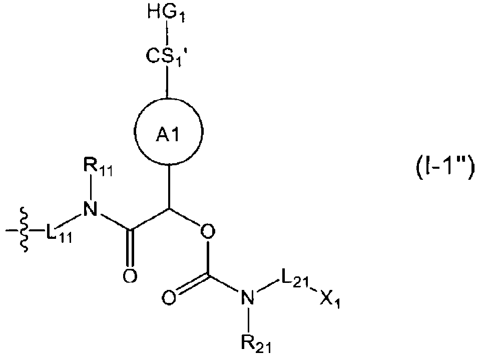 wherein
the hyphen (-) with a wavy line represents a bond to said sulfur atom,
HG₁ represents a hydrophilic group or a monovalent group containing a hydrophilic group,
CS₁' represents a divalent group containing a cleavable site,
Ring A1 represents an optionally substituted divalent aromatic ring group (wherein the divalent aromatic ring group forms a pi electron conjugated system with said cleavable site),
R₁₁ and R₂₁ each independently represent a hydrogen atom or a monovalent group,
L₁₁ and L₂₁ each independently represent a divalent group, and
X₁ represents a functional substance,
and
the lysine residue-modifying moiety is represented by the following formula (I-2''): 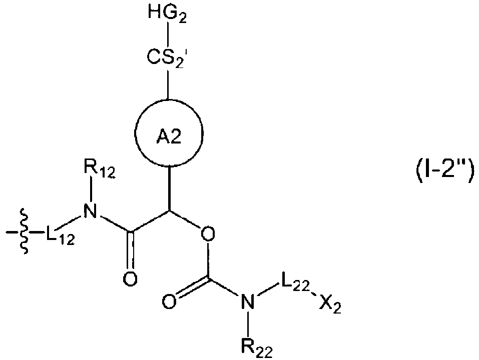 wherein
the hyphen (-) with a wavy line represents a bond to said nitrogen atom,
HG₂ represents a hydrophilic group or a monovalent group containing a hydrophilic group,
CS₂' represents a divalent group containing a cleavable site,
Ring A2 represents an optionally substituted divalent aromatic ring group (wherein the divalent aromatic ring group forms a pi electron conjugated system with said cleavable site),
R₁₂ and R₂₂ each independently represent a hydrogen atom or a monovalent group,
L₁₂ and L₂₂ each independently represent a divalent group, and
X₂ represents a functional substance,
or the following formula (II): 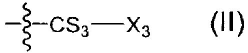 wherein
the hyphen (-) with a wavy line represents a bond to said nitrogen atom,
CS₃ represents a divalent group optionally containing a cleavable site, and
X₃ represents a functional substance.

20. The conjugate or a salt thereof of claim 19, which is (A) or (ii) below:
(A) the lysine residue-modifying moieties are regioselectively connected to the immunoglobulin unit by bonding to the nitrogen atoms in the side chains of two lysine residues present at the same position in said two heavy chains; or
(ii) the lysine residue-modifying moieties comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i),
wherein
the first lysine residue-modifying moiety is represented by the following formula (I-2-1"): 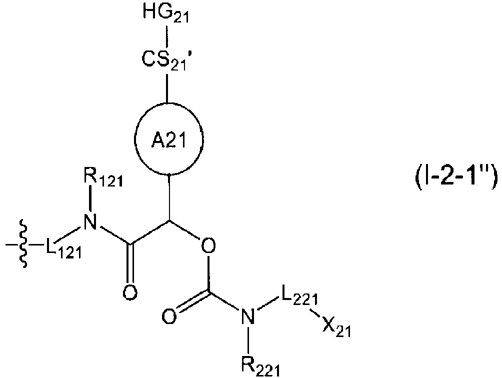 wherein
the hyphen (-) with a wavy line represents a bond to said nitrogen atom,
HG₂₁ represents a hydrophilic group or a monovalent group containing a hydrophilic group,
CS₂₁' represents a divalent group containing a cleavable site,
Ring A21 represents an optionally substituted divalent aromatic ring group (wherein the divalent aromatic ring group forms a pi electron conjugated system with said cleavable site),
R₁₂₁ and R₂₂₁ each independently represent a hydrogen atom or a monovalent group,
L₁₂₁ and L₂₂₁ each independently represent a divalent group, and
X₂₁ represents a functional substance,
or the following formula (II-1): 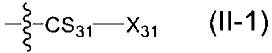 wherein
the hyphen (-) with a wavy line represents a bond to said nitrogen atom,
CS₃₁ represents a divalent group optionally containing a cleavable site, and
X₃₁ represents a functional substance,
and
the second lysine residue-modifying moiety is represented by the following formula (I-2-2''): 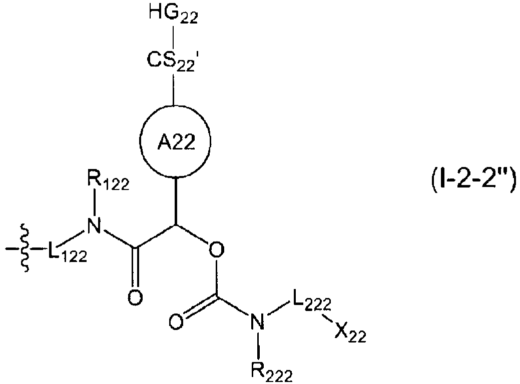 wherein
the hyphen (-) with a wavy line represents a bond to said nitrogen atom,
HG₂₂ represents a hydrophilic group or a monovalent group containing a hydrophilic group,
CS₂₂' represents a divalent group containing a cleavable site,
Ring A22 represents an optionally substituted divalent aromatic ring group (wherein the divalent aromatic ring group forms a pi electron conjugated system with said cleavable site),
R₁₂₂ and R₂₂₂ each independently represent a hydrogen atom or a monovalent group,
L₁₂₂ and L₂₂₂ each independently represent a divalent group, and
X₂₂ represents a functional substance,
or the following formula (II-2): 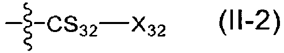 wherein
the hyphen (-) with a wavy line represents a bond to said nitrogen atom,
CS₃₂ represents a divalent group optionally containing a cleavable site, and
X₃₂ represents a functional substance.

21. An antibody intermediate having thiol groups, or a salt thereof, comprising:
(1) the antibody intermediate comprises an immunoglobulin unit comprising two heavy chains and two light chains, and
(2) said immunoglobulin unit comprises
(a') two or more thiol groups present in the side chains of two or more cysteine residues in said two heavy chains and two light chains, and
(b') two or more lysine residue-modifying moieties regioselectively connected to the immunoglobulin unit by bonding to the nitrogen atoms in the side chains of two or more lysine residues in said two heavy chains.

22. The antibody intermediate or a salt thereof of claim 21, wherein the immunoglobulin unit is a human immunoglobulin unit.

23. The antibody intermediate or a salt thereof of claim 22, wherein the human immunoglobulin unit is a human IgG antibody.

24. The antibody intermediate or a salt thereof of claim 21, wherein
said two or more cysteine residues are 2 to 8 cysteine residues, and
said two or more thiol groups are 2 to 8 thiol groups.

25. The antibody intermediate or a salt thereof of claim 21, wherein
said two or more cysteine residues are 8 cysteine residues, and
said two or more thiol groups are 8 thiol groups.

26. The antibody intermediate or a salt thereof of claim 21, wherein
the lysine residue-modifying moiety is represented by the following formula (III): 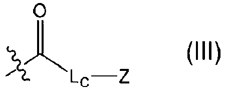 wherein
the hyphen (-) with a wavy line represents a bond to said nitrogen atom,
L_{C} represents a divalent group, and
Z represents a bioorthogonal functional group or a functional substance, or a monovalent group containing a bioorthogonal functional group or a functional substance.

27. The antibody intermediate or a salt thereof of claim 21, wherein the two or more lysine residues are any of the lysine residues present at the following positions according to Eu numbering:
(A) a lysine residue present at position 246/248 in the two heavy chains;
(ii) a lysine residue present at position 288/290 in the two heavy chains;
(C) a lysine residue present at position 317 in the two heavy chains; and
(D) a combination of (A) and (ii), (A) and (C), or (ii) and (C), or (A), (B), and (C).

28. The antibody intermediate or a salt thereof of claim 21, wherein the two or more lysine residue-modifying moieties are regioselectively connected to the immunoglobulin unit by bonding to the nitrogen atoms in the side chains of two lysine residues present at the same position in said two heavy chains.

29. The antibody intermediate or a salt thereof of claim 21, wherein
the two or more lysine residue-modifying moieties comprise (i) two first lysine residue-modifying moieties regioselectively connected to the immunoglobulin unit by bonding to the nitrogen atoms in the side chains of two lysine residues present at the same position in said two heavy chains, and (ii) two second lysine residue-modifying moieties regioselectively connected to the immunoglobulin unit by bonding to the nitrogen atoms in the side chains of two lysine residues present at the same position in said two heavy chains, wherein the two lysine residues are present at a different position from said two lysine residues in (i), and
the first lysine residue-modifying moiety is represented by the following formula (III-I): 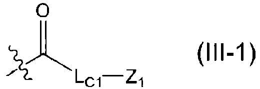 wherein
the hyphen (-) with a wavy line represents a bond to said nitrogen atom,
L_{C1} represents a divalent group, and
Z₁ represents a bioorthogonal functional group or a functional substance, or a monovalent group containing a bioorthogonal functional group or a functional substance, and
the second lysine residue-modifying moiety is represented by the following formula (III-2): 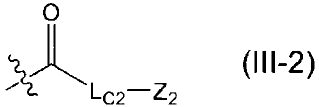 wherein
the hyphen (-) with a wavy line represents a bond to said nitrogen atom,
L_{C1} represents a divalent group, and
Z₂ represents a bioorthogonal functional group or a functional substance, or a monovalent group containing a bioorthogonal functional group or a functional substance.

30. The antibody intermediate or a salt thereof of claim 26, wherein the bioorthogonal functional group is selected from the group consisting of an azide group, an aldehyde group, a thiol group, an alkyne group, an alkene group, a halogen group, a tetrazine group, a nitrone group, a hydroxylamine group, a nitrile group, a hydrazine group, a ketone group, a boronic acid group, a cyanobenzothiazole group, an allyl group, a phosphine group, a maleimide group, a disulfide group, a thioester group, an α-halocarbonyl group, an isonitrile group, a sydnone group, and a selenium group.

31. The antibody intermediate or a salt thereof of claim 26, wherein the bioorthogonal functional group is a thiol group.

32. The antibody intermediate or a salt thereof of claim 31, wherein L_{C} is an alkylene group.
